(19) Europäisches Patentamt
European Patent Office
Office européen des brevets

(11) EP 0 565 487 B1

(12) **EUROPÄISCHE PATENTSCHRIFT**

(45) Veröffentlichungstag und Bekanntmachung des Hinweises auf die Patenterteilung:
**16.04.1997 Patentblatt 1997/16**

(21) Anmeldenummer: **93810228.2**

(22) Anmeldetag: **30.03.1993**

(51) Int. Cl.6: **C07C 69/732**, C08K 5/13, C09K 15/08, C07D 219/04, C07D 211/22, C07D 211/46

(54) **Flüssige Antioxidantien als Stabilisatoren**
Liquid antioxydants as stabilisers
Antioxydants liquides comme stabilisants

(84) Benannte Vertragsstaaten:
**BE DE DK ES FR GB IT NL**

(30) Priorität: **08.04.1992 CH 1153/92**

(43) Veröffentlichungstag der Anmeldung:
**13.10.1993 Patentblatt 1993/41**

(73) Patentinhaber: **Ciba Specialty Chemicals Holding Inc.**
**4057 Basel (CH)**

(72) Erfinder:
- **Dubs, Paul, Dr.**
  **CH-1723 Marly (CH)**
- **Martin, Roger**
  **CH-1700 Fribourg (CH)**
- **Evans, Samuel, Dr.**
  **CH-1723 Marly (CH)**

(56) Entgegenhaltungen:
**WO-A-91/13134**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 565 487 B1

Printed by Rank Xerox (UK) Business Services
2.14.2/3.4

## Beschreibung

Die vorliegende Erfindung betrifft neue flüssige und schwer flüchtige Antioxidantien, Zusammensetzungen, enthaltend ein organisches Material, bevorzugt ein Polymer oder Oel und die neuen, schwer flüchtigen, flüssigen Antioxidantien, sowie die Verwendung derselben zum Stabilisieren von organischen Materialien gegen oxidativen, thermischen oder lichtinduzierten Abbau.

Die Stabilisierung, insbesondere von Schmierstoffen oder von Kunststoffen mit Antioxidantien aus der Reihe der sterisch gehinderten Phenole, ist beispielsweise bekannt aus US-A-3 839 278, US-A-4 032 562, US-A-4 058 502, US-A-4 093 587 und US-A-4 132 702.

In WO 91/13134 ist eine Methode beschrieben, wie die Löslichkeit von Antioxidantien in einem zweiten Medium verbessert werden kann in welchem die Antioxidantien schlecht löslich sind. Die Methode ist dadurch gekennzeichnet, dass beipielsweise ein sterisch gehinderter 3-Phenylpropionsäureester mit beispielsweise Sonnenblumenöl umgesetzt wird. Bei diesen Umsetzungsprodukten sind die Antioxidantien chemisch kovalent mit dem zweiten Medium gebunden.

Die vorliegende Erfindung betrifft Produkte, erhältlich durch Umsetzung der Komponenten a), b) und c), wobei die Komponente a) eine Verbindung der Formel I oder ein Gemisch von Verbindungen der Formel I, die Komponente b) eine Verbindung der Formel II oder ein Gemisch von Verbindungen der Formel II und die Komponente c) eine Verbindung der Formel III oder ein Gemisch von Verbindungen der Formel III sind,

$X(Y)_a$ ,

(I)

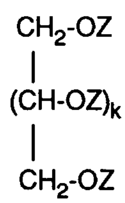

(II)

,

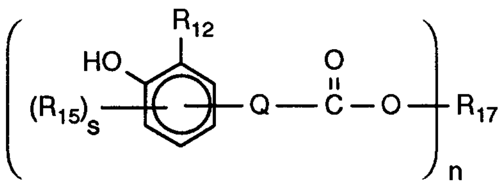

(III)

wobei in der Verbindung der Formel I die Reste

Y unabhängig voneinander OH, $(HOCH_2CH_2)_2N$- oder $-HNR_1$ darstellt und

$R_1$ Wasserstoff, $C_1$-$C_{18}$-Alkyl, $C_5$-$C_{12}$-Cycloalkyl,

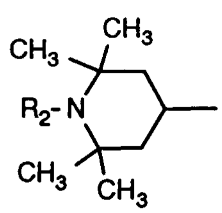

,

$C_3$-$C_6$-Alkenyl, $C_7$-$C_9$-Phenylalkyl, Phenyl oder durch 1 bis 3 Reste $A_1$ substituiertes Phenyl bedeutet, wobei die Reste $A_1$ unabhängig voneinander $C_1$-$C_{12}$-Alkyl, Halogen, Hydroxy, Methoxy oder Ethoxy bedeuten, wobei

$R_2$ Wasserstoff, $C_1$-$C_8$-Alkyl, $O^{\bullet}$, OH, NO, $-CH_2CN$, $C_1$-$C_{18}$-Alkoxy, $C_5$-$C_{12}$-Cycloalkoxy, $C_3$-$C_6$-Alkenyl, $C_7$-$C_9$-Phenylalkyl oder $C_7$-$C_9$-Phenylalkyl, welches am Phenylring mit $C_1$-$C_4$-Alkyl mono-, di- oder tri-substituiert ist, oder $R_2$ ferner $C_1$-$C_8$-Acyl oder $HOCH_2CH_2$- darstellt, und

a die Zahl 1,2,3,4 oder 6 bedeutet, wobei

wenn Y = OH und a = 1 ist,

X $C_1$-$C_{45}$-Alkyl, $C_3$-$C_{18}$-Alkenyl, $-CH_2CH_2T_1(CH_2CH_2O)_bR_4$ oder

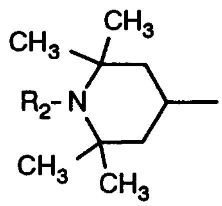

bedeutet, wobei $R_2$ die obige Bedeutung hat, und

| | |
|---|---|
| $T_1$ | Sauerstoff, Schwefel oder >N-$R_5$, |
| $R_4$ | $C_1$-$C_{20}$-Alkyl, |
| b | eine ganze Zahl aus dem Bereich von 0 bis 10 und |
| $R_5$ | Wasserstoff, $C_1$-$C_{18}$-Alkyl oder Phenyl darstellt, oder |

wenn Y = OH und a = 2 ist,

X $-CH_2CH_2T_2(CH_2CH_2O)_bCH_2CH_2-$, wobei b die obige Bedeutung hat,

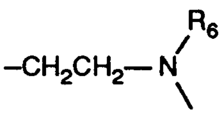 , $-C_cH_{2c}-$ , 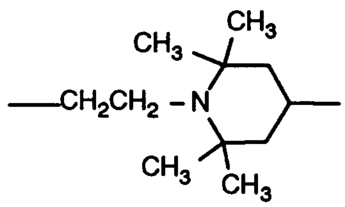 ,

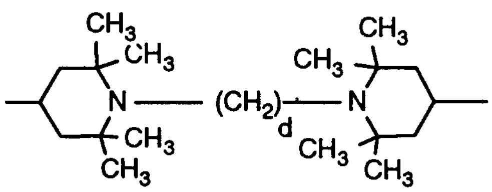 , $-CH_2$-$CH$=$CH$-$CH_2-$ ,

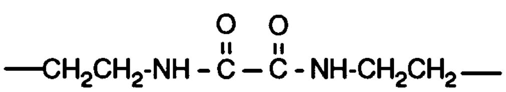 oder

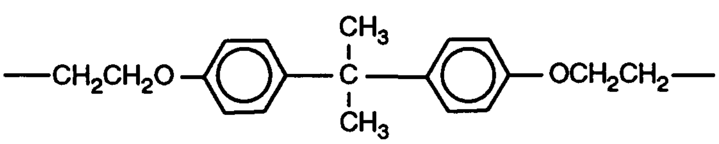

bedeutet, wobei

$T_2$ Sauerstoff, Schwefel, >N-$R_5$ oder

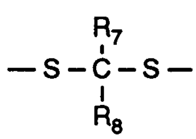

ist und $R_5$ die obige Bedeutung hat,

| | |
|---|---|
| $R_6$ | Wasserstoff, $C_1$-$C_{18}$-Alkyl oder Phenyl, |
| c | eine ganz Zahl aus dem Bereich von 2 bis 10, |
| d | eine ganz Zahl aus dem Bereich von 2 bis 6 und |

$R_7$, $R_8$ unabhängig voneinander Wasserstoff, $C_1$-$C_{18}$-Alkyl oder Phenyl sind, oder $R_7$ und $R_8$ mit dem C-Atom, an das sie gebunden sind, einen $C_5$-$C_{12}$-Cycloalkyl-Ring bilden, oder

wenn a = 3 ist,

X $C_3$-$C_{10}$-Alkantriyl oder $N(CH_2CH_2\text{-})_3$ bedeutet, oder

wenn Y = OH und a = 4 ist,

X $C_4$-$C_{10}$-Alkantetrayl,

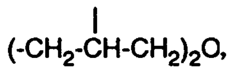

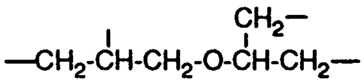 , 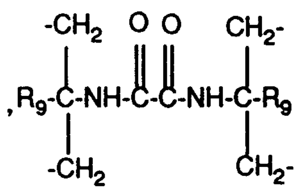 , 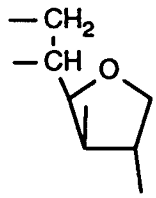 oder

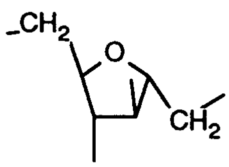

darstellt, wobei

$R_9$ $C_1$-$C_4$-Alkyl bedeutet, oder

wenn Y = OH und a = 6 ist,

X

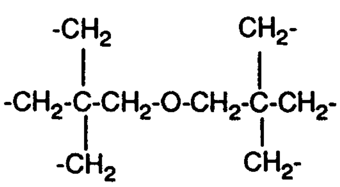 , 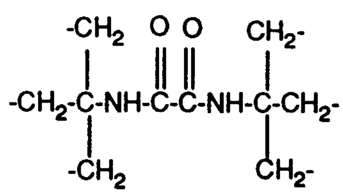

oder $C_6$-$C_{10}$-Alkanhexayl darstellt, oder

wenn Y = -$HNR_1$ und a = 1 ist,

X $C_1$-$C_{18}$-Alkyl, $C_3$-$C_{18}$-Alkenyl, $C_5$-$C_{12}$-Cycloalkyl, $C_7$-$C_9$-Phenylalkyl, Phenyl,

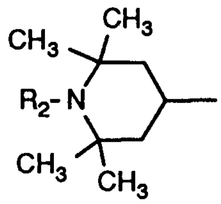 ,

wobei $R_2$ die obige Bedeutung hat, oder X ferner

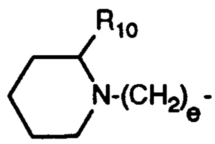

ist, oder X mit $R_1$ gemeinsam eine Gruppe der Formel $-CH_2CH_2CH_2CH_2CH_2-$ oder $-CH_2CH_2OCH_2CH_2-$ bildet, wobei

$R_{10}$ Wasserstoff oder Methyl, und
e 2 oder 3 bedeutet, oder

wenn Y = $-HNR_1$ und a = 2 ist,

X $-C_fH_{2f}-$,

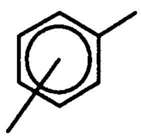 oder $-(CH_2CH_2\overset{H}{N})_g-CH_2CH_2-$

darstellt, wobei
f eine ganze Zahl aus dem Bereich von 2 bis 10 und
g eine ganze Zahl aus dem Bereich von 1 bis 6 bedeutet, und

in der Verbindung der Formel II die Reste

Z Wasserstoff oder eine Gruppe der Formel

$$-(C_hH_{2h}O)_i-\overset{O}{\overset{\|}{C}}-R_{11}$$

bedeutet, und
k eine ganze Zahl aus dem Bereich von 0 bis 6 darstellt, wobei
h 2 oder 3,
i eine ganze Zahl aus dem Bereich von 0 bis 12 und
$R_{11}$ $C_1$-$C_{30}$-Alkyl, $C_8$-$C_{30}$-Alkenyl, $C_5$-$C_{12}$-Cycloalkyl, Phenyl oder $C_7$-$C_9$-Phenylalkyl bedeutet, mit der Bedingung, dass die Verbindung der Formel II eine Gruppe

$$-(C_hH_{2h}O)_i-\overset{O}{\overset{\|}{C}}-R_{11}$$

enthält;

in der Verbindung der Formel III

$R_{12}$ $C_1$-$C_{18}$-Alkyl, $C_5$-$C_{12}$-Cycloalkyl, Phenyl oder $C_7$-$C_9$-Phenylalkyl bedeutet,
$R_{15}$ Wasserstoff, $C_1$-$C_{18}$-Alkyl, $C_5$-$C_{12}$-Cycloalkyl, Phenyl oder $C_7$-$C_9$-Phenylalkyl darstellt,
s 0, 1 oder 2 bedeutet,
Q $-C_mH_{2m}-$,

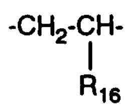 oder 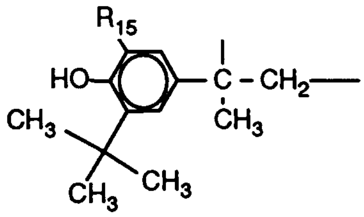

darstellt, wobei $R_{15}$ die obige Bedeutung hat,
m eine ganze Zahl aus dem Bereich von 0 bis 3 bedeutet,
$R_{16}$ $C_1$-$C_8$-Alkyl darstellt und
n eine ganze Zahl aus dem Bereich von 1 bis 6 bedeutet, wobei

wenn n = 1 ist,

$R_{17}$ Wasserstoff, $C_1$-$C_{45}$-Alkyl, $C_5$-$C_{12}$-Cycloalkyl, $C_2$-$C_{18}$-Alkenyl, einen einwertigen Rest einer Hexose, einen einwertigen Rest eines Hexitols,

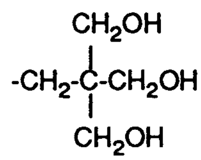 , 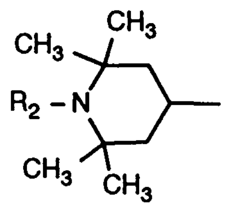 ,

wobei $R_2$ die obige Bedeutung hat, oder ferner $R_{17}$ $-CH_2CH_2-T_3-R_{19}$ oder

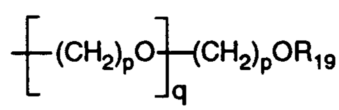

bedeutet, wobei
$T_3$ Sauerstoff, Schwefel oder >N-$R_{22}$ darstellt,
$R_{19}$

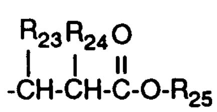 oder 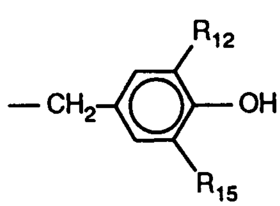

ist, wobei $R_{12}$ und $R_{15}$ die obige Bedeutung haben, oder $R_{19}$ ferner Wasserstoff, $C_1$-$C_{24}$-Alkyl, Phenyl, $C_5$-$C_{12}$-Cycloalkyl oder

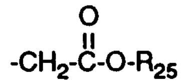

bedeutet, wobei

p eine ganze Zahl aus dem Bereich von 2 bis 4 darstellt,

q eine ganze Zahl aus dem Bereich von 2 bis 20 bedeutet,

$R_{22}$ $C_1$-$C_{18}$-Alkyl, Phenyl oder durch 1 bis 3 Reste $A_1$ substituiertes Phenyl bedeutet, wobei die Reste $A_1$ unabhängig voneinander $C_1$-$C_{12}$-Alkyl, Halogen, Hydroxy, Methoxy oder Ethoxy bedeuten, oder $R_{22}$ ferner $C_5$-$C_8$-Cycloalkyl darstellt,

$R_{23}$, $R_{24}$ unabhängig voneinander Wasserstoff oder Methyl bedeuten mit der Massgabe, dass $R_{23}$ und $R_{24}$ nicht gleichzeitig Methyl sind;

$R_{25}$ Wasserstoff oder $C_1$-$C_{24}$-Alkyl bedeutet, oder

wenn n = 2 ist,

$R_{17}$ einen zweiwertigen Rest einer Hexose, einen zweiwertigen Rest eines Hexitols,

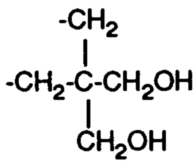 , 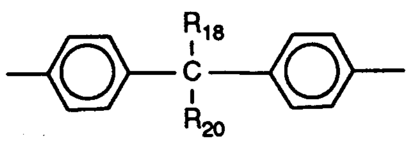 , $-C_rH_{2r}-$,

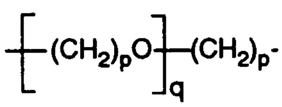 ,

wobei p und q die obige Bedeutung haben, $-CH_2CH_2-T_4-CH_2CH_2-$, $-CH_2-CH=CH-CH_2-$, $-CH_2-C{\equiv}C-CH_2-$,

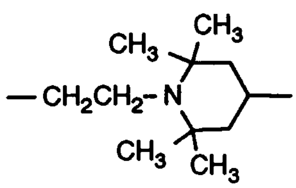 ,

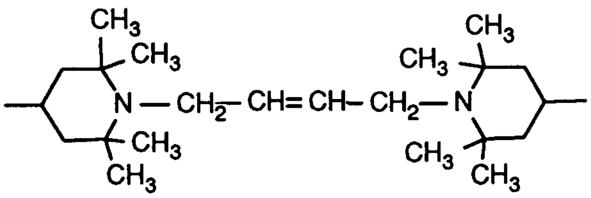

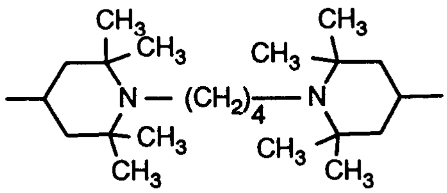 , 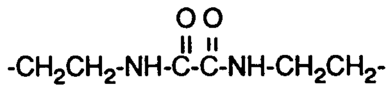 oder

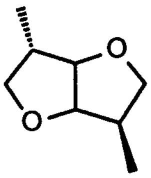

darstellt, wobei

$R_{18}$, $R_{20}$ unabhängig voneinander Wasserstoff oder $C_1$-$C_{12}$-Alkyl bedeuten oder zusammen den Rest $-CH_2CH_2CH_2CH_2CH_2-$ bilden,

r eine ganze Zahl aus dem Bereich von 2 bis 10 darstellt,

$T_4$ Schwefel, >N-$R_{26}$ oder

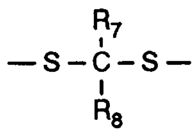

bedeutet, wobei $R_7$ und $R_8$ die obige Bedeutung haben, und

$R_{26}$ Wasserstoff, $C_1$-$C_{18}$-Alkyl, Phenyl oder durch 1 bis 3 Reste $A_1$ substituiertes Phenyl, wobei die Reste $A_1$ die obige für die Formel I beschriebene Bedeutung haben, oder $R_{26}$ ferner $C_5$-$C_8$-Cycloalkyl oder

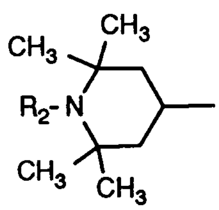

bedeutet, wobei $R_2$ die obige Bedeutung hat, oder

wenn n = 3 ist,

$R_{17}$ einen dreiwertigen Rest einer Hexose, einen dreiwertigen Rest eines Hexitols,

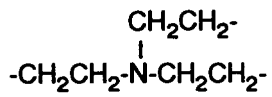,
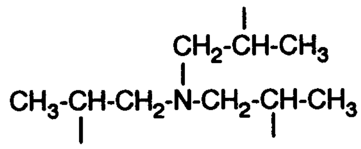
oder
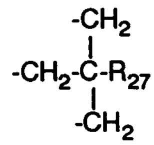

darstellt, wobei

$R_{27}$ Wasserstoff, $-CH_2OH$, $C_1$-$C_4$-Alkyl, $C_1$-$C_{18}$-Alkylamido oder

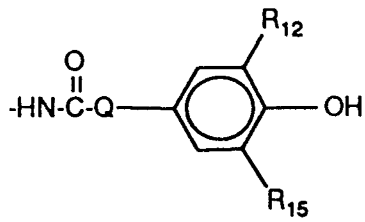

bedeutet, wobei Q, $R_{12}$ und $R_{15}$ die obige Bedeutung haben, oder

wenn n = 4 ist,

$R_{17}$ einen vierwertigen Rest einer Hexose, einen vierwertigen Rest eines Hexitols, $C_4$-$C_{10}$-Alkantetrayl,

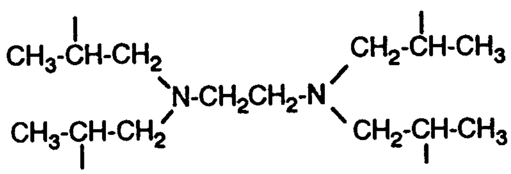,
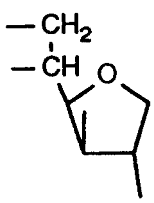
oder
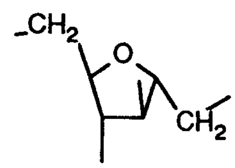

darstellt, oder

wenn n = 5 ist,

$R_{17}$ einen fünfwertigen Rest einer Hexose oder einen fünfwertigen Rest eines Hexitols darstellt, oder

wenn n = 6 ist,

$R_{17}$ einen sechswertigen Rest eines Hexitols oder

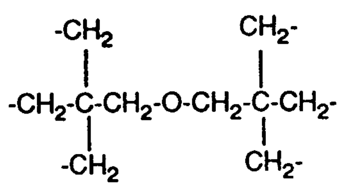

bedeutet.

Die flüssigen und schwerflüchtigen Produkte gemäss vorliegender Erfindung zeichnen sich durch eine sehr gute Stabilisierung von organischen Materialien, wie z.B. Polymeren oder Oelen sowohl gegen oxidativen, thermischen als auch lichtinduzierten Abbau aus.

Alkyl mit bis zu 45 C-Atomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl oder Pentacosyl. Eine der bevorzugten Bedeutungen von $R_1$, $R_4$ und $R_{16}$ ist beispielweise $C_1$-$C_4$-Alkyl, von $R_2$ Methyl, von $R_{11}$ $C_1$-$C_{20}$-Alkyl, von $R_{12}$ und $R_{15}$ $C_1$-$C_4$-Alkyl, insbesondere tert-Butyl, und von $R_{17}$ $C_1$-$C_{18}$-Alkyl.

Cycloalkyl mit bis zu 12 C-Atomen bedeutet beispielsweise Cyclopentyl, Cyclohexyl, Cycloheptyl, Cyclooctyl, Cyclodecyl oder Cyclododecyl. Eine der bevorzugten Bedeutungen von $R_1$, $R_{11}$, $R_{12}$ und $R_{15}$ ist $C_5$-$C_7$-Cycloalkyl. Besonders bevorzugt ist Cyclohexyl.

Alkenyl mit bis zu 30 C-Atomen bedeutet beispielsweise Vinyl, Propenyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, Oleyl, n-Octadec-2-enyl oder n-Octadec-4-enyl. Bedeuten $R_1$, $R_2$ und X $C_3$-$C_6$-Alkenyl, so ist das C-Atom, welches an den Stickstoff gebunden ist, zweckmässigerweise gesättigt.

Phenylalkyl mit 7 bis 9 C-Atomen bedeutet beispielsweise Benzyl, α-Methylbenzyl, α,α-Dimethylbenzyl oder 2-Phenylethyl. Benzyl ist bevorzugt.

Beispiele für Phenyl, welches durch 1 bis 3 Reste $A_1$ substituiert ist, sind o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 2-Methyl-4-tert-butylphenyl, 2-Ethylphenyl, 2,6-Diethylphenyl, 2,6-Diethyl-4-methylphenyl, 2,6-Diisopropylphenyl, 4-tert-Butylphenyl, p-Nonylphenyl, o-, m- oder p-Chlorphenyl, 2,3-Dichlorphenyl, 2,4-Dichlorphenyl, 2,5-Dichlorphenyl, 2,6-Dichlorphenyl, 3,4-Dichlorphenyl, 2,4,5-Trichlorphenyl, 2,4,6-Trichlorphenyl, o-, m- oder p-Hydroxyphenyl, o-, m- oder p-Methoxyphenyl, o- oder p-Ethoxyphenyl, 2,4-Dimethoxyphenyl, 2,5-Dimethoxyphenyl, 2,5-Diethoxyphenyl, o-, m- oder p-Methoxycarbonyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, 2-Hydroxy-4-methylphenyl, 3-Hydroxy-4-methylphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-dimethoxyphenyl und 4-Chlor-2,5-dimethoxyphenyl. Bevorzugt ist dabei mit 1 oder 2, insbesondere 1 Rest(en) $A_1$ substituiertes Phenyl, wobei $A_1$ insbesondere Alkyl ist.

Alkoxy mit 1 bis 18 C-Atomen bedeutet beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Eine der bevorzugten Bedeutungen von $R_2$ ist $C_6$-$C_{12}$-Alkoxy. Besonders bevorzugt sind Heptoxy und Octoxy.

Cycloalkoxy mit 5 bis 12 C-Atomen bedeutet beispielsweise Cyclopentoxy, Cyclohexoxy, Cycloheptoxy, Cyclooctoxy, Cyclodecyloxy oder Cyclododecyloxy. Eine der bevorzugten Bedeutungen von $R_2$ ist $C_5$-$C_8$-Cycloalkoxy. Besonders bevorzugt sind Cyclopentoxy und Cyclohexoxy.

Beispiele für $C_7$-$C_9$-Phenylalkyl, welches am Phenylring mit $C_1$-$C_4$-Alkyl mono-, di- oder tri-substituiert ist, sind Methylbenzyl, Dimethylbenzyl, Trimethylbenzyl oder tert-Butylbenzyl.

Acyl mit 1 bis 8 C-Atomen bedeutet beispielsweise Formyl, Acetyl, Propionyl, Butyryl, Pentanoyl, Hexanoyl, Heptanoyl, Octanoyl, Benzoyl, Acryloyl oder Crotonyl. Bevorzugt sind $C_1$-$C_8$-Alkanoyl, $C_3$-$C_8$-Alkenoyl oder Benzoyl, insbesondere Acetyl.

Alkantriyl mit 3 bis 10 C-Atomen bedeutet beispielsweise

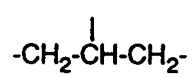 ,

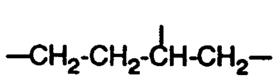 , 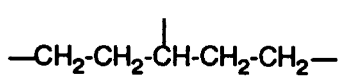 , 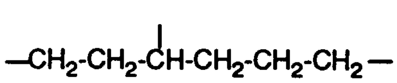

oder 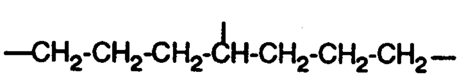 .

Bevorzugt ist Glyceryl.

Alkantetrayl mit 4 bis 10 C-Atomen bedeutet beispielsweise

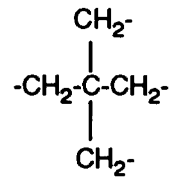 ,

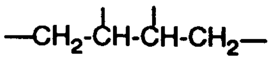 , 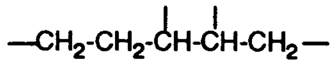 , 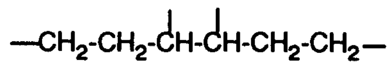 ,

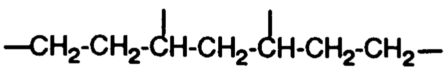 oder 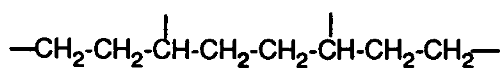 .

Bevorzugt ist Pentaerythrityl.
Alkanhexayl mit 6 bis 10 C-Atomen bedeutet beispielsweise

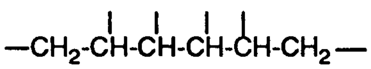 , 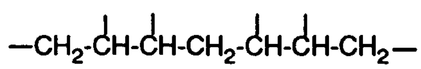 oder

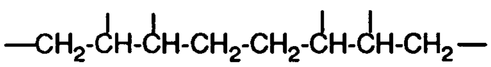 .

Bedeutet $R_{17}$ für n = 1 bis 6 einen n-wertigen Rest einer Hexose, so leitet sich dieser z.B. von Allose, Altrose, Glucose, Mannose, Gulose, Idose, Galaktose oder Talose ab, d.h. um die entsprechenden Verbindungen der Formel III zu erhalten, müssen eine, zwei, drei, vier, fünf oder sechs -OH Gruppen durch die Estergruppe E-1,

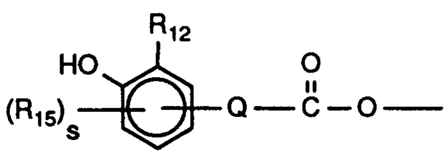 (E-1)

worin $R_{12}$, $R_{15}$, s und Q die oben angegebenen Bedeutungen haben, ersetzt werden. So kann $R_{17}$ z.B. für n = 5 eine Gruppe

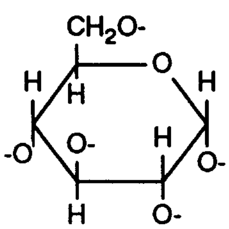

bedeuten.

Stellt $R_{17}$ den n-wertigen Rest eines Hexitols dar, so erhält man die entsprechenden Verbindungen der Formel III, indem n -OH Gruppen durch die oben angegebene Estergruppe E-1 ersetzt werden. $R_{17}$ kann als sechswertiger Rest eines Hexitols z.B.

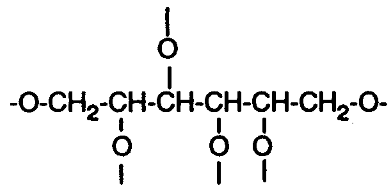

sein. Diese Gruppe leitet sich von dem D-Sorbitol ab.

Alkylamido mit 1 bis 18 C-Atomen bedeutet beispielsweise $CH_3$-CO-NH-, $CH_3CH_2$-CO-NH-, $C_6H_{13}$-CO-NH- oder $C_{18}H_{37}$-CO-NH-.

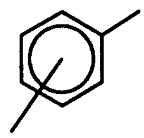

bedeutet, dass der Phenylring ortho, meta oder para substituiert sein kann.

Die Umsetzung der drei Komponenten a), b) und c) miteinander zu den Produkten der vorliegenden Erfindung kann in einer beliebigen Reihenfolge erfolgen.

Bevorzugt wird zuerst Komponente a) mit Komponente b) umgesetzt und anschliessend wird Komponente c) zugegeben.

Zweckmässigerweise wird die Reaktion in Gegenwart eines Katalysators durchgeführt. Als Katalysatoren kommen insbesondere Lewis-Säuren oder Basen in Frage.

Geeignete basische Katalysatoren sind beispielsweise Metallhydride, Metallalkylide, Metallarylide, Metallhydroxide, Metallalkoholate, Metallphenolate, Metallamide oder Metallcarboxylate.

Bevorzugte Metallhydride sind beispielsweise Lithiumhydrid, Natriumhydrid oder Kaliumhydrid.

Bevorzugte Metallalkylide sind beispielsweise Butyllithium oder Methyllithium.

Ein bevorzugtes Metallarylid ist beispielsweise Phenyllithium.

Bevorzugte Metallhydroxide sind beispielsweise Lithiumhydroxid, Natriumhydroxid, Kaliumhydroxid, Cäsiumhydroxid, Rubidiumhydroxid, Magnesiumhydroxid, Calciumhydroxid, Bariumhydroxid oder Aluminiumhydroxid.

Bevorzugte Metallalkoholate sind beispielsweise Natriummethanolat, Natriumethanolat, Kaliummethanolat, Kaliumethanolat, Natriumisopropylat oder Kalium tert-butylat.

Bevorzugte Metallphenolate sind beispielweise Natriumphenolat oder Kaliumphenolat.

Bevorzugte Metallamide sind beispielsweise Natriumamid oder Lithiumamid.

Ein bevorzugtes Metallcarboxylat ist beispielsweise Calciumacetat.

Geeignete Lewis-Säuren-Katalysatoren sind beispielsweise

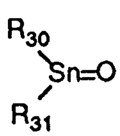 , 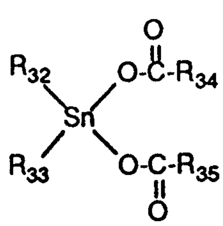 oder 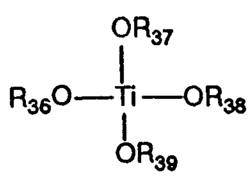 ,

wobei die Reste $R_{30}$, $R_{31}$, $R_{32}$, $R_{33}$, $R_{34}$, $R_{35}$, $R_{36}$, $R_{37}$, $R_{38}$ und $R_{39}$ z.B. unabhängig voneinander $C_1$-$C_{18}$-Alkyl oder Phenyl bedeuten. Bevorzugt ist $C_1$-$C_8$-Alkyl. Ein besonders bevorzugter Lewis-Säure-Katalysator ist Dibutylzinnoxid.

Der Katalysator wird zu den Komponenten a), b) und c) z.B. in einer Menge von 0,05 bis 10 Gewichtspromille zugegeben, bevorzugt in einer Menge von 0,1 bis 5 Gewichtspromille. Besonders bevorzugt ist die Zugabe von 1 bis 2 Gewichtspromille Dibutylzinnoxid.

Die Umsetzung der Komponenten a), b) und c) kann in einem Lösungsmittel, wie beispielsweise Xylol, oder ohne Lösungsmittel durchgeführt werden. Bevorzugt wird die Umsetzung ohne Lösungsmittel durchgeführt.

Die Reaktionstemperatur liegt z.B. zwischen 130 und 250°C. Bevorzugt wird die Umsetzung in einem Temperaturbereich von 130 bis 190°C durchgeführt.

Ein weiterer Anmeldungsgegenstand ist auch ein Verfahren zur Herstellung der erfindungsgemässen Produkte, dadurch gekennzeichnet, dass die Komponenten a), b) und c) in einem molaren Mengenverhältnis von 0,1:1:0,1 bis 15:1:30 umgesetzt werden.

Die Komponenten a), b) und c) können, sofern sie nicht im Handel erhältlich sind, nach bekannten Verfahren bzw. in Analogie zu solchen hergestellt werden. Mögliche Herstellungsverfahren für die Verbindungen der Formel III sind z.B. in folgenden Veröffentlichungen zu finden: GB-A-996 502, US-A-3 330 859, US-A-3 944 594, US-A-4 593 057, EP-A-154 518 oder US-A-3 960 928.

Ein bevorzugter Gegenstand der Erfindung sind Produkte, wobei in der Verbindung der Formel III s die Zahl 1 oder 2 bedeutet.

Ein ebenfalls bevorzugter Gegenstand der Erfindung sind Produkte,
wobei in der Verbindung der Formel I die Reste

Y unabhängig voneinander OH, $(HOCH_2CH_2)_2N$- oder $-HNR_1$ bedeutet und

$R_1$ Wasserstoff, $C_1$-$C_{10}$-Alkyl, $C_5$-$C_7$-Cycloalkyl,

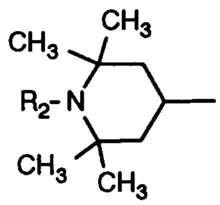

,

$C_3$-$C_6$-Alkenyl, Benzyl oder Phenyl bedeutet, wobei

$R_2$ Wasserstoff, $C_1$-$C_4$-Alkyl, OH, $-CH_2CN$, $C_6$-$C_{12}$-Alkoxy, $C_5$-$C_8$-Cycloalkoxy, Allyl, Benzyl, Acetyl oder $HOCH_2CH_2$- darstellt, und

a die Zahl 1,2,3,4 oder 6 bedeutet, wobei

wenn Y = OH und a = 1 ist,

X $C_1$-$C_{30}$-Alkyl, $C_3$-$C_{18}$-Alkenyl, $-CH_2CH_2T_1(CH_2CH_2O)_bR_4$ oder

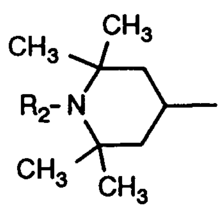

bedeutet, wobei $R_2$ die obige Bedeutung hat, und

$T_1$ Sauerstoff, Schwefel oder $>N-R_5$,

$R_4$ $C_1$-$C_{10}$-Alkyl,

b eine ganze Zahl aus dem Bereich von 0 bis 10 und

$R_5$ Wasserstoff, $C_1$-$C_{10}$-Alkyl oder Phenyl darstellen, oder

wenn Y = OH und a = 2 ist,

X $-CH_2CH_2T_2(CH_2CH_2O)_bCH_2CH_2$-, wobei b die obige Bedeutung hat,

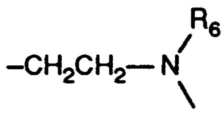 , $-C_cH_{2c}-$ , 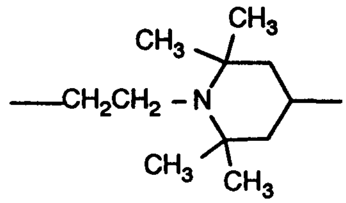 ,

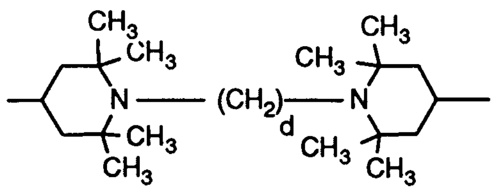 , $-CH_2-CH=CH-CH_2-$ ,

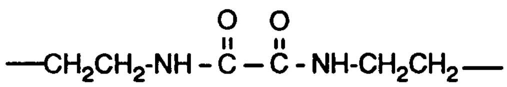 oder

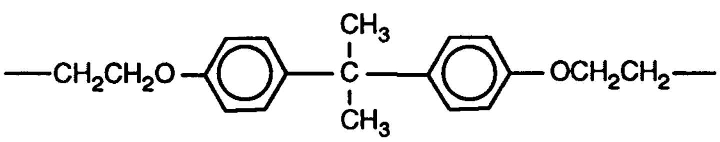

bedeutet, wobei

T

$T_2$ Sauerstoff, Schwefel, $>N-R_5$ oder

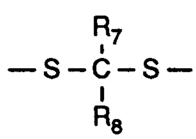

ist und $R_5$ die obige Bedeutung hat,

$R_6$ Wasserstoff, $C_1$-$C_{10}$-Alkyl oder Phenyl,

c eine ganze Zahl aus dem Bereich von 2 bis 10,

d eine ganze Zahl aus dem Bereich von 2 bis 6 und

$R_7$, $R_8$ unabhängig voneinander Wasserstoff, $C_1$-$C_{10}$-Alkyl oder Phenyl sind, oder $R_7$ und $R_8$ mit dem C-Atom, an das sie gebunden sind, einen $C_5$-$C_7$-Cycloalkyl-Ring bilden, oder

wenn Y = $-HNR_1$ und a = 1 ist,

X $C_1$-$C_{10}$-Alkyl, $C_3$-$C_{18}$-Alkenyl, $C_5$-$C_7$-Cycloalkyl, Benzyl, Phenyl,

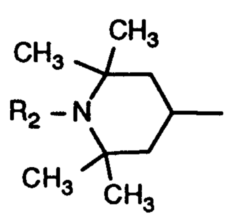 ,

wobei $R_2$ die obige Bedeutung hat, oder X ferner

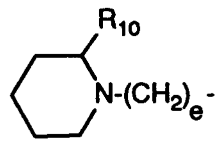

ist, oder X mit $R_1$ gemeinsam eine Gruppe der Formel $-CH_2CH_2CH_2CH_2CH_2-$ oder $-CH_2CH_2OCH_2CH_2-$ bildet, wobei

$R_{10}$ Wasserstoff oder Methyl ist, und
e 2 oder 3 bedeutet, und

in der Verbindung der Formel II die Reste

Z Wasserstoff oder eine Gruppe der Formel

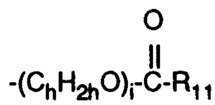

bedeuten, und
k eine ganze Zahl aus dem Bereich von 0 bis 4 darstellt, wobei
h 2 oder 3,
i eine ganze Zahl aus dem Bereich von 0 bis 6 und
$R_{11}$ $C_1$-$C_{20}$-Alkyl, $C_8$-$C_{20}$-Alkenyl, $C_5$-$C_7$-Cycloalkyl, Phenyl oder Benzyl bedeuten, mit der Bedingung, dass die Verbindung der Formel II eine Gruppe

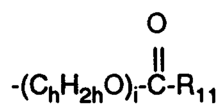

enthält;

in der Verbindung der Formel III

$R_{12}$ $C_1$-$C_6$-Alkyl, $C_5$-$C_7$-Cycloalkyl, Phenyl oder Benzyl bedeutet,
$R_{15}$ Wasserstoff, $C_1$-$C_6$-Alkyl, $C_5$-$C_7$-Cycloalkyl, Phenyl oder Benzyl darstellt,
s 1 oder 2 bedeutet,
Q $-C_mH_{2m}-$,

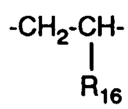 oder 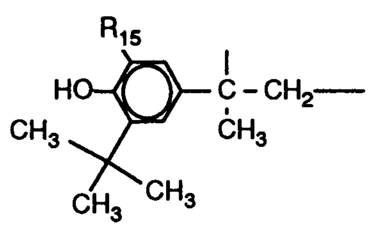

darstellt, wobei $R_{15}$ die obige Bedeutung hat,
m eine ganze Zahl aus dem Bereich von 0 bis 3 bedeutet,
$R_{16}$ $C_1$-$C_4$-Alkyl darstellt und
n eine ganze Zahl aus dem Bereich von 1 bis 6 bedeutet, wobei

wenn n = 1 ist,

$R_{17}$ Wasserstoff, $C_1$-$C_{30}$-Alkyl, $C_5$-$C_7$-Cycloalkyl, $C_2$-$C_{18}$-Alkenyl, einen einwertigen Rest einer Hexose, einen einwertigen Rest eines Hexitols,

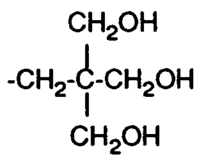 , 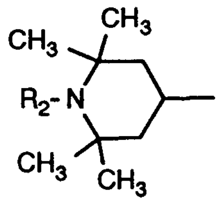 ,

wobei $R_2$ die obige Bedeutung hat, oder ferner $R_{17}$ $-CH_2CH_2-T_3-R_{19}$ oder

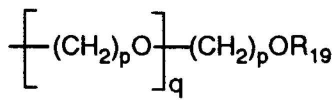

bedeutet, wobei

$T_3$ Sauerstoff, Schwefel oder $>N-R_{22}$ darstellt,

$R_{19}$

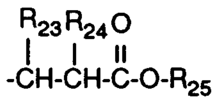 , 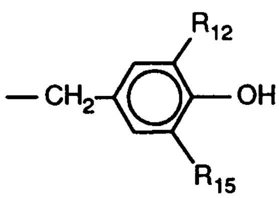 ,

wobei $R_{12}$ und $R_{15}$ die obige Bedeutung haben, oder $R_{19}$ ferner Wasserstoff, $C_1$-$C_{18}$-Alkyl, Phenyl, $C_5$-$C_7$-Cycloalkyl oder

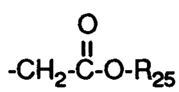

bedeutet, wobei

p eine ganze Zahl aus dem Bereich von 2 bis 4 darstellt,

q eine ganze Zahl aus dem Bereich von 2 bis 20 bedeutet,

$R_{22}$ $C_1$-$C_{10}$-Alkyl, Phenyl oder $C_5$-$C_8$-Cycloalkyl darstellt,

$R_{23}$, $R_{24}$ unabhängig voneinander Wasserstoff oder Methyl bedeuten, mit der Massgabe, dass $R_{23}$ und $R_{24}$ nicht gleichzeitig Methyl sind;

$R_{25}$ Wasserstoff oder $C_1$-$C_{18}$-Alkyl bedeutet, oder

wenn n = 2 ist,

$R_{17}$ einen zweiwertigen Rest einer Hexose, einen zweiwertigen Rest eines Hexitols,

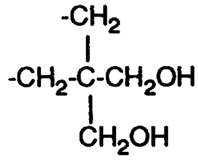 , 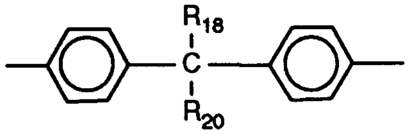 , $-C_rH_{2r}-$,

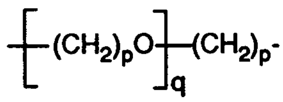 ,

wobei p und q die obige Bedeutung haben,
$-CH_2CH_2-T_4-CH_2CH_2-$, $-CH_2-CH=CH-CH_2-$, $-CH_2-C\equiv C-CH_2-$,

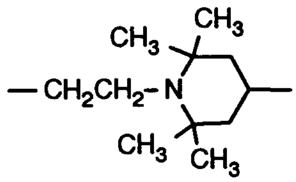 ,

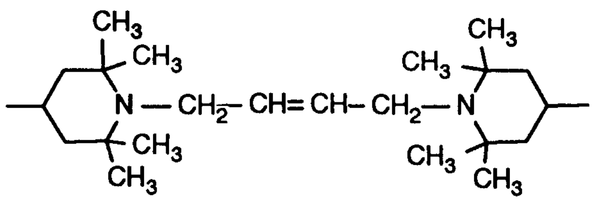 ,

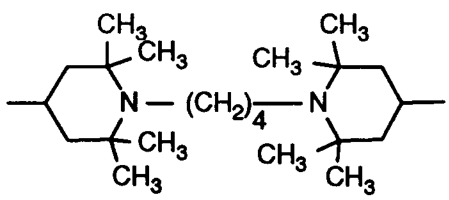 , 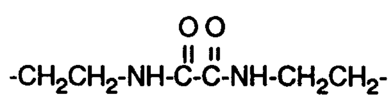 oder

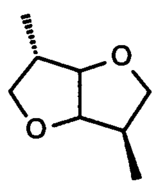

darstellt, wobei

$R_{18}$, $R_{20}$ unabhängig voneinander Wasserstoff oder $C_1$-$C_6$-Alkyl bedeuten oder zusammen den Rest $-CH_2CH_2CH_2CH_2CH_2-$ bilden,

r eine ganze Zahl aus dem Bereich von 2 bis 10 darstellt,

$T_4$ Schwefel, $>N-R_{26}$ oder

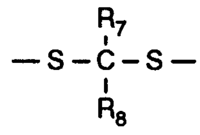

bedeutet, wobei $R_7$ und $R_8$ die obige Bedeutung haben und

$R_{26}$ Wasserstoff, $C_1$-$C_{10}$-Alkyl, Phenyl, $C_5$-$C_8$-Cycloalkyl oder

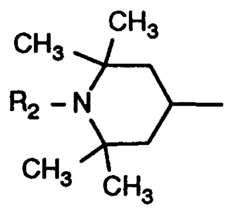

bedeutet, wobei $R_2$ die obige Bedeutung hat.

Ein besonders bevorzugter Gegenstand der Erfindung sind Produkte, wobei in der Verbindung der Formel I die Reste

Y unabhängig voneinander OH, $(HOCH_2CH_2)_2N$- oder $-HNR_1$ darstellt und
$R_1$ Wasserstoff, $C_1$-$C_4$-Alkyl oder

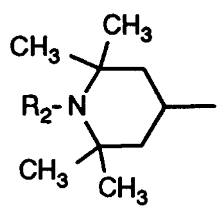

bedeutet, wobei
$R_2$ Wasserstoff, $C_1$-$C_4$-Alkyl, OH, Allyl, Benzyl, Acetyl oder $HOCH_2CH_2$- darstellt, und
a die Zahl 1,2,3,4 oder 6 bedeutet, wobei

wenn Y = OH und a = 1 ist,

X $C_1$-$C_{18}$-Alkyl, $C_3$-$C_{18}$-Alkenyl, $-CH_2CH_2T_1(CH_2CH_2O)_bR_4$ oder

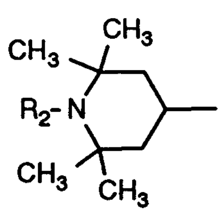

bedeutet, wobei $R_2$ die obige Bedeutung hat, und
$T_1$ Sauerstoff,
$R_4$ $C_1$-$C_4$-Alkyl und
b eine ganze Zahl aus dem Bereich von 0 bis 10 darstellt, oder

wenn Y = OH und a = 2 ist,

X $-CH_2CH_2T_2(CH_2CH_2O)_bCH_2CH_2-$, wobei b die obige Bedeutung hat, oder ferner X $—C_cH_{2c}—$,

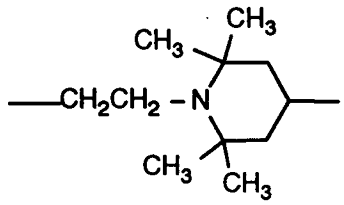

oder $—CH_2\text{-}CH\text{=}CH\text{-}CH_2—$ bedeutet, wobei

$T_2$ Sauerstoff, Schwefel oder >N-$R_5$,

$R_5$ Wasserstoff,

b die Zahl 0 oder 1, und

c eine ganze Zahl aus dem Bereich von 2 bis 8 darstellen, oder

wenn a = 3 ist,

X

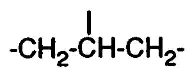

oder $N(CH_2CH_2-)_3$ bedeutet, oder

wenn Y = OH und a = 4 ist,

X

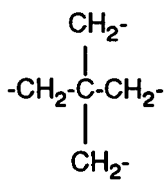 , 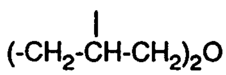 oder 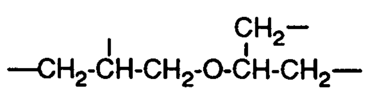

darstellt, oder

wenn Y = OH und a = 6 ist,

X

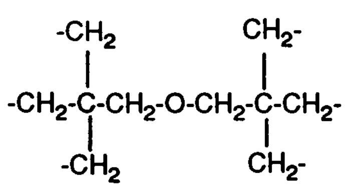 oder 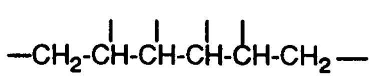

darstellt, oder

wenn Y = $-HNR_1$ und a = 1 ist,

X $C_1$-$C_{10}$-Alkyl, $C_3$-$C_{18}$-Alkenyl, $C_5$-$C_7$-Cycloalkyl oder

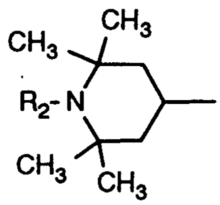

bedeutet, wobei $R_2$ die obige Bedeutung hat, oder

wenn $Y = -HNR_1$ und $a = 2$ ist,

X $-C_fH_{2f}-$ darstellt, wobei
f eine ganze Zahl aus dem Bereich von 2 bis 10 bedeutet, und

in der Verbindung der Formel II die Reste

Z Wasserstoff oder eine Gruppe der Formel

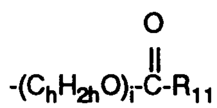

bedeutet, und
k 1, 2 oder 3 darstellt,
h 2 oder 3 bedeutet,
i eine ganze Zahl aus dem Bereich von 0 bis 4 ist und
$R_{11}$ $C_1$-$C_{20}$-Alkyl oder $C_8$-$C_{20}$-Alkenyl bedeutet, mit der Bedingung, dass die Verbindung der Formel II eine Gruppe

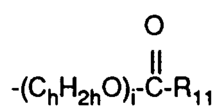

enthält;

in der Verbindung der Formel III

$R_{12}$ $C_1$-$C_6$-Alkyl oder $C_5$-$C_7$-Cycloalkyl bedeutet,
$R_{15}$ Wasserstoff, $C_1$-$C_6$-Alkyl oder $C_5$-$C_7$-Cycloalkyl darstellt,
s 1 oder 2 bedeutet,
Q $-C_mH_{2m}-$ oder

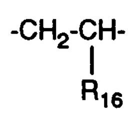

darstellt,
m eine ganze Zahl aus dem Bereich von 0 bis 3 bedeutet,

$R_{16}$ $C_1$-$C_4$-Alkyl darstellt und
n eine ganze Zahl aus dem Bereich von 1 bis 6 bedeutet, wobei

wenn n = 1 ist,

$R_{17}$ Wasserstoff, $C_1$-$C_{18}$-Alkyl, $C_5$-$C_7$-Cycloalkyl, $C_2$-$C_{18}$-Alkenyl, einen einwertigen Rest einer Hexose, einen einwertigen Rest eines Hexitols,

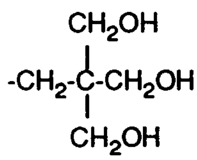 oder 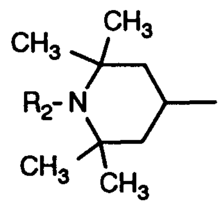 ,

wobei $R_2$ die obige Bedeutung hat, oder ferner $R_{17}$

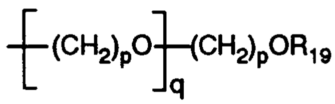

bedeutet, wobei
$R_{19}$ Wasserstoff, $C_1$-$C_{18}$-Alkyl oder $C_5$-$C_7$-Cycloalkyl bedeutet, wobei
p eine ganze Zahl aus dem Bereich von 2 bis 4 darstellt,
q eine ganze Zahl aus dem Bereich von 2 bis 10 bedeutet, oder

wenn n = 2 ist,

$R_{17}$ einen zweiwertigen Rest einer Hexose, einen zweiwertigen Rest eines Hexitols,

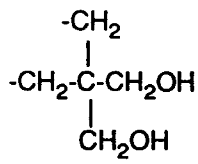 , $-C_rH_{2r}-$, 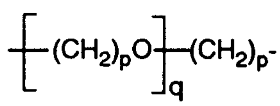 ,

wobei p und q die obige Bedeutung haben, $-CH_2CH_2-T_4-CH_2CH_2-$, oder

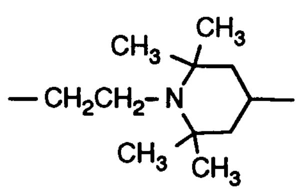

darstellt, wobei
r eine ganze Zahl aus dem Bereich von 2 bis 10 darstellt,
$T_4$ Schwefel oder >N-$R_{26}$ bedeutet und
$R_{26}$ Wasserstoff, $C_1$-$C_{10}$-Alkyl oder $C_5$-$C_8$-Cycloalkyl bedeutet, oder

wenn n = 3 ist,

$R_{17}$ einen dreiwertigen Rest einer Hexose, einen dreiwertigen Rest eines Hexitols,

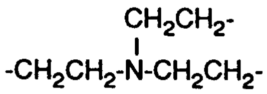
oder
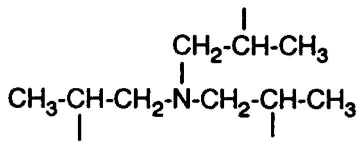

darstellt, oder

wenn n = 4 ist,

$R_{17}$ einen vierwertigen Rest einer Hexose, einen vierwertigen Rest eines Hexitols,

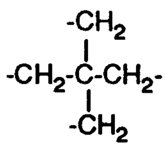
oder
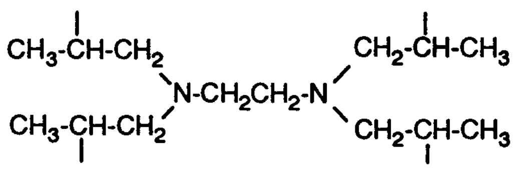

darstellt.

Einen bevorzugten Gegenstand der Erfindung bilden weiterhin Produkte, wobei in der Verbindung der Formel I die Reste

Y unabhängig voneinander Hydroxy oder $-NH_2$ bedeutet und
a eine ganze Zahl aus dem Bereich von 1 bis 4 darstellt, wobei

wenn a = 1 ist,

X =

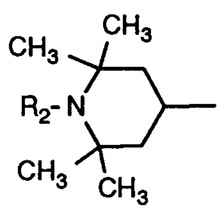

bedeutet, und

$R_2$ Wasserstoff, Methyl oder $HOCH_2CH_2$- darstellt, oder

wenn Y = OH und a = 2 ist,

X $-CH_2CH_2T_2(CH_2CH_2O)_bCH_2CH_2-$, $—C_cH_{2c}—$ oder

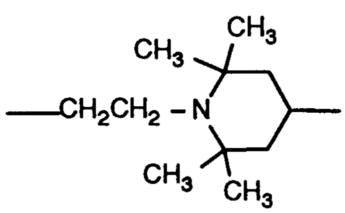

bedeutet, wobei

$T_2$ Sauerstoff, Schwefel oder >N-$R_5$,
$R_5$ Wasserstoff,
b die Zahl 0 oder 1 und
c die Zahl 2, 3 oder 4 darstellen, oder

wenn Y = OH und a = 3 ist,

X

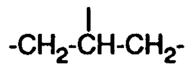

bedeutet, oder

wenn Y = OH und a = 4 ist,

X

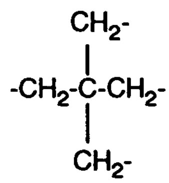

darstellt, und

in der Verbindung der Formel II die Reste

Z Wasserstoff oder eine Gruppe der Formel

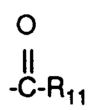

bedeuten,
k die Zahl 1 darstellt, und
$R_{11}$ $C_1$-$C_{20}$-Alkyl oder $C_8$-$C_{20}$-Alkenyl bedeutet, mit der Bedingung, dass die Verbindung der Formel II eine Gruppe

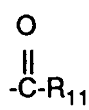

enthält, und

in der Verbindung der Formel III

$R_{12}$ tert-Butyl bedeutet,
$R_{15}$ $C_1$-$C_4$-Alkyl darstellt und in ortho Stellung zur OH-Gruppe gebunden ist,
s die Zahl 1 bedeutet,
Q $-C_mH_{2m}-$ darstellt und para zur OH-Gruppe gebunden ist, wobei
m die Zahl 2 bedeutet,

n 1 darstellt, und

$R_{17}$ $C_1$-$C_4$-Alkyl bedeutet.

Bevorzugte Verbindungen der Formel I sind beispielsweise Pentaerythrit, Thiodiethylenglykol, 1,4-Butandiol, 1,2-Propandiol, Diethylenglykol, Triethylenglykol, Diethanolamin, Glycerin,

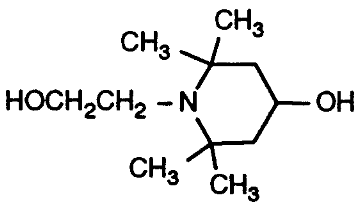 , 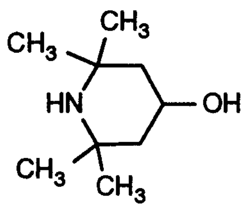 oder 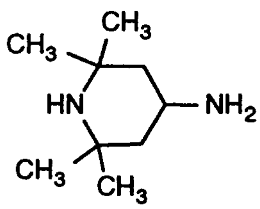 .

Besonders bevorzugt ist Glycerin oder Thiodiethylenglykol.

Bevorzugte Verbindungen der Formel II sind in der Natur vorkommende pflanzliche Oele, Fette und Wachse, tierische Oele und Fette sowie künstlich erzeugte Polyol-Derivate.

Bevorzugte pflanzliche Oele, Fette und Wachse sind beispielsweise Sonnenblumenöl, Kokosfett, Rapsöl, Sojabohnenöl, Maiskeimöl, Distelöl, Olivenöl, Erdnussöl, Baumwollsamenöl, Sesamöl, Safloröl, Rizinusöl, Talgöl, Kürbiskernenöl oder Leinsamenöl.

Bevorzugte tierische Oele und Fette sind beispielsweise Butterfett, Schweinefett, Fischöl, Spermöl, Klauenöl oder Trane.

Bevorzugte künstlich erzeugte Polyol-Derivate sind beispielsweise Radiamuls (Glycerin Tri $C_8$/$C_{10}$) oder Sorbitan-Derivate. Die Sorbitan-Derivate sind z.B. unter den Namen Span®20, Span®40, Span®60, Span®65, Span®80, Span®85, Tween 20®, Tween 40®, Tween 60®, Tween 65®, Tween 80® oder Tween 85® im Handel erhältlich.

Besonders bevorzugt ist Sonnenblumenöl, Kokosfett oder Rapsöl.

Einen bevorzugten Gegenstand der Erfindung bilden ebenfalls Produkte, wobei in der Verbindung der Formel III

$R_{12}$ $C_1$-$C_4$-Alkyl oder Cyclohexyl bedeutet,

$R_{15}$ $C_1$-$C_4$-Alkyl oder Cyclohexyl darstellt und in ortho Stellung zur OH-Gruppe gebunden ist,

s die Zahl 1 bedeutet,

Q $-C_mH_{2m}-$ darstellt und para zur OH-Gruppe gebunden ist, wobei

m eine ganze Zahl aus dem Bereich von 0 bis 3, und

n eine ganze Zahl aus dem Bereich von 1 bis 4 bedeuten, wobei

wenn n = 1 ist,

$R_{17}$ Wasserstoff, $C_1$-$C_{10}$-Alkyl, Cyclohexyl, $C_2$-$C_{18}$-Alkenyl oder

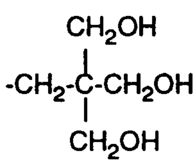

darstellt, oder

wenn n = 2 ist,

$R_{17}$

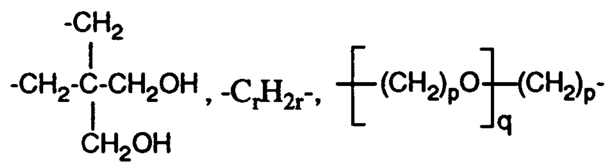

oder $-CH_2CH_2-T_4-CH_2CH_2-$ bedeutet, wobei

| | |
|---|---|
| p | eine ganze Zahl aus dem Bereich von 2 bis 4 darstellt, |
| q | eine ganze Zahl aus dem bereich von 2 bis 10 bedeutet, |
| r | eine ganze Zahl aus dem Bereich von 2 bis 6 darstellt, |
| $T_4$ | Schwefel oder $>N-R_{26}$ bedeutet und |
| $R_{26}$ | Wasserstoff oder $C_1$-$C_4$-Alkyl bedeutet, oder |

wenn n = 3 ist,

$R_{17}$

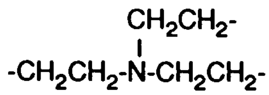

oder

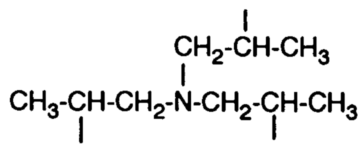

darstellt, oder

wenn n = 4 ist,

$R_{17}$

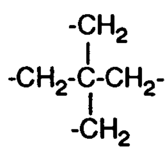

oder

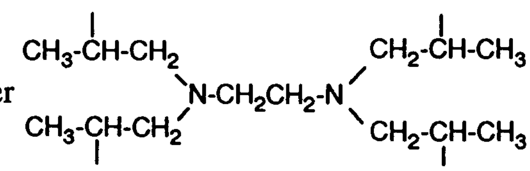

darstellt.

Ein besonders bevorzugter Gegenstand der Erfindung sind ferner Produkte, wobei in der Verbindung der Formel III

| | |
|---|---|
| $R_{12}$ | tert-Butyl bedeutet, |
| $R_{15}$ | $C_1$-$C_4$-Alkyl darstellt und in ortho Stellung zur OH-Gruppe gebunden ist, |
| s | die Zahl 1 bedeutet, |
| Q | $-C_mH_{2m}-$ darstellt und para zur OH-Gruppe gebunden ist, wobei |
| m | die Zahl 2 bedeutet und |
| n | eine ganze Zahl 1,2 oder 4 bedeutet, wobei |

wenn n = 1 ist,

$R_{17}$ $C_1$-$C_4$-Alkyl bedeutet, oder

wenn n = 2 ist,

$R_{17}$

$$-\left[(CH_2)_pO\right]_q-(CH_2)_p-$$

oder $-CH_2CH_2-T_4-CH_2CH_2-$ bedeutet, wobei

p die Zahl 2,

q die Zahl 2 und

$T_4$ Schwefel bedeuten, oder

wenn n = 4 ist,

$R_{17}$

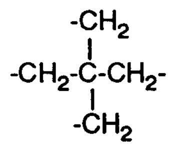

darstellt.

Bevorzugte Verbindungen der Formel III sind beispielsweise auch

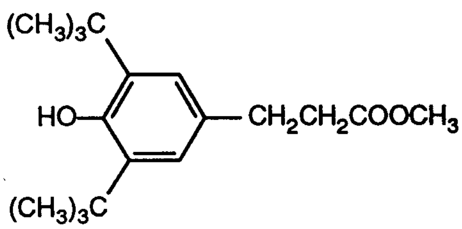

,

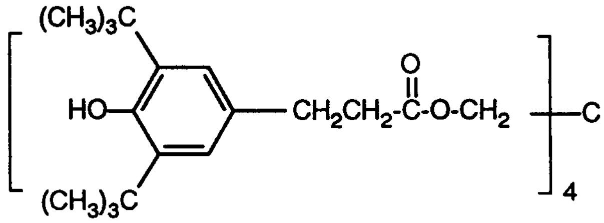

,

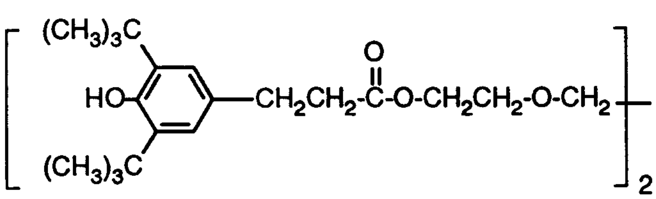

,

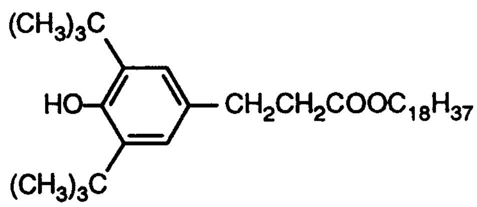

,

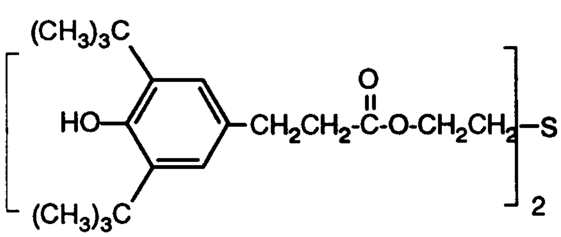

,

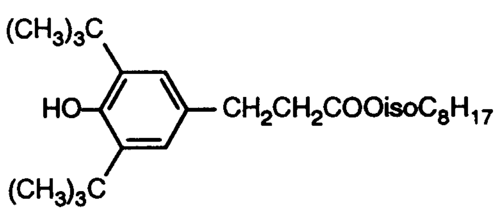

,

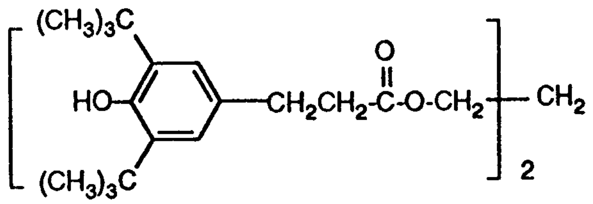 oder 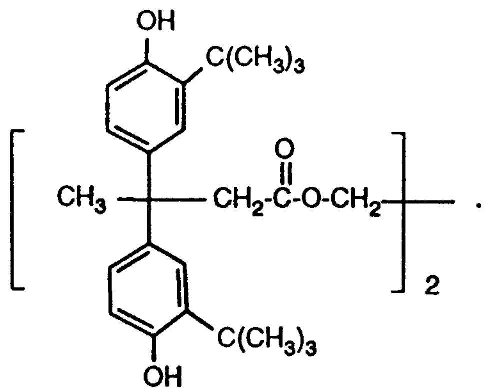

Besonders bevorzugte Verbindungen der Formel III sind 3-(3',5'-Di-tert-butyl-4'-hydroxyphenyl)propionsäuremethylester und 3-(3'-tert-Butyl-4'-hydroxy-5'-methylphenyl)propiorsäuremethylester.

Einen speziell bevorzugten Gegenstand der Erfindung bilden Produkte, erhältlich durch Umsetzung der Komponenten a), b) und c), wobei die Komponente a) eine Verbindung der Formel I, insbesondere Pentaerythrit, Thiodiethylenglykol, 1,4-Butandiol, 1,2-Propandiol, Diethylenglykol, Triethylenglykol, Diethanolamin, Glycerin,

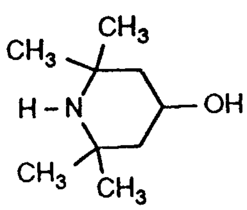 , 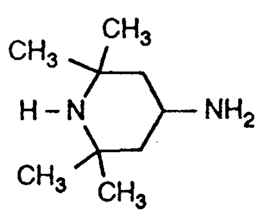 oder 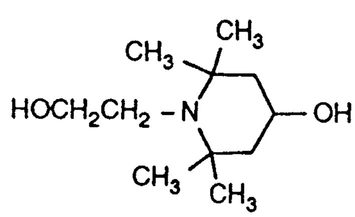

bedeutet oder ein Gemisch davon,
die Komponente b) eine Verbindung der Formel II, insbesondere Sonnenblumenöl, Kokosfett, Rapsöl, Malskeimöl, Distelöl, Olivenöl, Erdnussöl oder Radiamuls darstellt oder ein Gemisch davon und die Komponente c) eine Verbindung der Formel III, insbesondere 3-(3',5'-Di-tert-butyl-4'-hydroxyphenyl)propionsäuremethylester oder 3-(3'-tert-Butyl-4'-hydroxy-5'-methylphenyl)propionsäuremethylester bedeutet.

Die vorliegende Erfindung betrifft ferner Produkte, erhältlich durch Umsetzung der Komponenten a), b) und c) in einem molaren Mengenverhältnis von 0,1:1:0,1 bis 15:1:30. Bevorzugt ist ein molares Mengenverhältnis von 1:1:1 bis 10:1:20. Besonders bevorzugt ist ein molares Mengenverhältnis von 4:1:5 bis 10:1:20. Speziell bevorzugt ist ein molares Mengenverhältnis von 5:1:10.

Die erfindungsgemässen Produkte können z.B. 30 bis 80 Gew.-%, bevorzugt 35 bis 80 Gew.-%, insbesondere 50 bis 80 Gew.-% der Wirkgruppe E-2

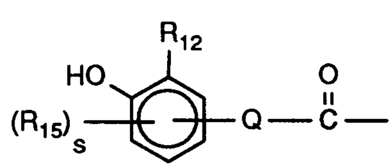 (E-2)

enthalten.

Wie bereits erwähnt, eignen sich die vorliegenden Produkte zum Stabilisieren von organischen Materialien gegen oxidativen, thermischen oder lichtinduzierten Abbau. Insbesondere wird auf ihre hervorragende Wirkung als Antioxidantien bei der Stabilisierung organischer Materialien hingewiesen.

Beispiele für derartige Materialien sind:

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).
b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und altemierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. $C_5$-$C_9$) inklusive hydrierte Modifikationen davon (z.B. Klebrigmacherharze) und Mischungen von Polyalkylenen und Stärke.
5. Polystyrol, Poly-(p-methylstyrol), Poly-($\alpha$-methylstyrol).
6. Copolymere von Styrol oder $\alpha$-Methylstyrol mit Dienen oder Acrylderivaten, wie z.B Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid,

Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastichen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymer-homolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastiches PUR, PC/thermoplastisches PUR, POM/Acrylat, POM/MBS, PPO/HIPS, PPO/PA 6.6 und Copolymere, PA/HDPE, PA/PP, PA/PPO.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Weitere Gegenstände der Erfindung sind daher Zusammensetzungen enthaltend ein gegen oxidativen, thermischen oder lichtinduzierten Abbau empfindliches organisches Material und mindestens ein Produkt, erhältlich durch Umsetzung der Komponenten a), b) und c), sowie die Verwendung dieser Produkte zum Stabilisieren von organischem Material gegen oxidativen, thermischen oder lichtinduzierten Abbau.

Die Erfindung umfasst daher auch ein Verfahren zum Stabilisieren von organischem Material gegen thermischen, oxidativen oder lichtinduzierten Abbau, dadurch gekennzeichnet, dass man diesem Material mindestens ein Produkt, erhältlich durch Umsetzung der Komponenten a), b) und c), zusetzt.

Von besonderem Interesse ist die Verwendung dieser Produkte als Antioxidantien in organischen Materialien.

Bevorzugte organische Materialien sind Polymere, z.B. synthetische Polymere, insbesondere thermoplastische Polymere. Besonders bevorzugte organische Materialien sind Polyolefine und Styrolcopolymere, z.B. die oben unter den Punkten 1 bis 3 und unter den Punkten 6 und 7 angegebenen, insbesondere Polyethylen und Polypropylen sowie ABS und Styrol-Butadien-Copolymere. Bevorzugter Gegenstand der Erfindung sind daher Zusammensetzungen, worin das organische Material ein synthetisches organisches Polymer bzw. ein Gemisch solcher Polymere, insbesondere ein Polyolefin oder ein Styrolcopolymer ist.

Im allgemeinen werden die Produkte dem zu stabilisierenden Material in Mengen von 0,01 bis 10 %, bevorzugt 0,01 bis 5 %, insbesondere 0,01 bis 2 %, bezogen auf das Gesamtgewicht deszu stabilisierenden Materials, zugesetzt. Besonders bevorzugt ist der Einsatz der erfindungsgemässen Produkte in Mengen von 0,01 bis 0,5 %, vor allem 0,05 bis 0,3 %.

Neben dem Produkt können die erfindunsgemässen Zusammensetzungen zusätzlich herkömmliche Additive enthalten, wie beispielsweise die unten angegebenen.

1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butylhydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.

1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.12. Ester der β-(3,5-Di-tert-butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.13. Ester der β-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert-Butyl- 2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl)-phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'-methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300; $[R-CH_2CH_2-COO(CH_2)_3]_2$ mit R = 3'-tert-Butyl-4'-hydroxy-5'-2H-benztriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenyl-salicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensasionsprodukt aus 1-Hydroxyethyl-2,2,6,6-tetramethyl-4-hydroxypiperidin und Bernsteinsäure, Kondensasionsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfld, Pentaerythrit-tetrakis-(β-dodecylmercapto)-propionat.

6. Polyamidstabilisatoren, wie z.B. Kupfersalze in Kombination mit Jodiden und/oder Phosphorverbindungen und

Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Benzofuranone bzw. Indolinone, wie z.B. in US-A-4 325 863 oder US-A-4 338 244 beschrieben.

Die herkömmlichen Additive werden beispielsweise in Konzentrationen von 0,01 bis 10 %, bezogen auf das Gesamtgewicht des zu stabilisierenden Materials, zugesetzt.

Die Einarbeitung der Produkte sowie gegebenenfalls weiterer Additive in das organische Material erfolgt nach bekannten Methoden. Die Einarbeitung in die Materialien kann beispielsweise durch Einmischen oder Aufbringen der Produkte und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbrindung der gelösten oder dispergierten Produkte auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Produkte in Polymere besteht in deren Zugabe vor, während oder unmittelbar nach der Polymerisation der entsprechenden Monomeren bzw. vor der Vernetzung. Die erfindungsgemässen Produkte können dabei als solche, aber auch in verkapselter Form (z.B. in Wachsen, Oelen oder Polymeren) zugesetzt werden. Im Falle der Zugabe vor oder während der Polymerisation können die erfindungsgemässen Produkte auch als Regulatoren für die Kettenlänge der Polymeren (Kettenabbrecher) wirken. Die erfindungsgemässen Produkte können auch in Form eines Masterbatches, der diese beispielsweise in einer Konzentration von 2,5 bis 25 Gew.-% enthält, den zu stabilisierenden Materialien zugesetzt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Weitere Gegenstände der Erfindung sind Zusammensetzungen, enthaltend eine funktionelle Flüssigkeit, bevorzugt aus der Reihe der Schmierstoffe, der Hydraulikflüssigkeiten und der Metallbearbeitungsflüssigkeiten sowie Kraftstoffe zum Antrieb von Motoren des Typs 4-Takt-Otto-, 2-Takt-, Diesel-, Wankel- sowie Orbital und mindestens ein Produkt, erhältlich durch Umsetzung der Komponenten a), b) und c).

Besonders bevorzugt als Schmierstoff sind die Mineralöle, die synthetischen Oele oder Mischungen davon.

Als funktionelle Flüssigkeiten aus der Reihe der Schmierstoffe, der Hydraulikflüssigkeiten und der Metallbearbeitungsflüssigkeiten kommen die an sich bekannten Produkte zum Einsatz.

Die in Frage kommenden Schmierstoffe und Hydraulikflüssigkeiten sind dem Fachmann geläufig und z.B. in Dieter Klamann "Schmierstoffe und verwandte Produkte", Verlag Chemie, Weinheim, 1982, in Schewe-Kobek, "Das Schmiermittel-Taschenbuch", Dr. Alfred Hüthig-Verlag, Heidelberg, 1974, oder in "Ullmanns Encyclopädie der technischen Chemie", Band 13, Seiten 85-94 (Verlag Chemie, Weinheim, 1977) beschrieben.

Beispiele hierfür sind Schmierstoffe und Hydraulikflüssigkeiten auf Mineralöl-Basis oder synthetische Schmierstoffe oder Hydraulykflüssigkeiten, insbesondere solche, die Carbonsäure-Esterderivate darstellen und bei Temperaturen von 200°C und höher verwendet werden.

Beispiele von synthetischen Schmierstoffen umfassen Schmierstoffe auf der Basis eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropan-tripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrittetracaprylat, oder eines komplexen Esters von einwertigen und zweiwertigen Säuren mit mehrwertigen Alkoholen, wie z.B. ein komplexer Ester von Trimethylolpropan mit Capryl- und Sebacinsäure oder von einem Gemisch davon.

Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Die erfindungsgemässen Produkte sind Oele und gut in Schmierstoffen löslich und deshalb als Zusätze zu Schmierstoffen besonders geeignet und es ist auf ihre überraschend gute antioxidative und antikorrosive Wirkung hinzuweisen.

Beispielsweise in Schmierstoffen für Verbrennungsmotoren, wie z.B. in Verbrennungsmotoren nach dem Otto-Prinzip, vermögen die erfindungsgemässen Produkte ihre überraschenden Eigenschaften zu entfalten. So verhindern dabei die erfindungsgemässen Produkte in Schmierölen die Bildung von Ablagerungen (Schlamm) oder reduzieren diese in überraschendem Masse.

Es ist auch möglich, sogenannte Masterbatches herzustellen.

Die erfindungsgemässen Produkte wirken schon in sehr geringen Mengen als Additive in Schmierstoffen. Sie werden den Schmierstoffen zweckmässig in einer Menge von 0,01 bis 5 Gew.-%, vorzugsweise in einer Menge von 0,05 bis 3 Gew.-% und ganz besonders bevorzugt in einer Menge von 0,1 bis 2 Gew.-%, jeweils bezogen auf den Schmierstoff, beigemischt.

Die Schmierstoffe können zusätzlich andere Additive enthalten, die zugegeben werden, um die Grundeigenschaften von Schmierstoffen noch weiter zu verbessern; dazu gehören: Antioxidantien, Metallpassivatoren, Rostinhibitoren, Viskositätsindex-Verbesserer, Stockpunkterniedriger, Dispergiermittel, Detergentien, Hochdruck-Zusätze, Reibungsverbesserer und Antiverschleiss-Additive.

Beispielsweise ist eine Reihe solcher Verbindungen obiger Auflistung "1. Antioxidantien", insbesondere Punkte 1.1 bis 1.16 zu entnehmen. Zusätzlich sind weitere Additive beispielhalft zu nennen:

<u>Beispiele für aminische Antioxidantien:</u>

N,N'-Di-isopropyl-p-phenylendiamin, N,N'-Di-sec-butyl-p-phenylendiamin, N,N'-Bis-(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, N-Allylphenothiazin, N,N,N',N'-Tetraphenyl-1,4-diamino-but-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

<u>Beispiele für weitere Antioxidantien:</u>

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy-3,7,10,14-tetrathiahexadecan.

<u>Beispiele für Metall-Desaktivatoren, z.B. für Kupfer, sind:</u>

a) Benztriazole und deren Derivate, z.B. 4- oder 5-Alkylbenztriazole (z.B. Tolutriazol) und deren Derivate, 4,5,6,7-Tetrahydrobenztriazol, 5,5'-Methylenbis-benztriazol; Mannich-Basen von Benztriazol oder Tolutriazol wie 1-[Di(2-ethylhexyl)aminomethyl)-tolutriazol und 1-[Di(2-ethylhexyl)aminomethyl)-benztriazol; Alkoxyalkylbenztriazole wie 1-(Nonyloxymethyl)-benztriazol, 1-(1-Butoxyethyl)-benztriazol und 1-(1-Cyclohexyloxybutyl)-tolutriazol.

b) 1,2,4-Triazole und deren Derivate, z.B. 3-Alkyl (oder Aryl)- 1,2,4-Triazole, Mannich-Basen von 1,2,4-Triazolen wie 1-[Di(2-ethylhexyl)aminomethyl-1,2,4-triazol; Alkoxyalkyl-1,2,4-triazole wie 1-(1-Butoxyethyl-1,2,4-triazol; acylierte 3-Amino-1,2,4-triazole.

c) Imidazolderivate, z.B. 4,4'-Methylenbis(2-undecyl-5-methylimidazol, Bis[(N-methyl)imidazol-2-yl]carbinol-octylether.

d) Schwefelhaltige heterocyclische Verbindungen, z.B. 2-Mercaptobenzthiazol, 2,5-Dimercapto-1,3,4-thiadiazol und deren Derivate; 3,5-Bis[di(2-ethylhexyl)amino-methyl]-1,3,4-thiadiazolin-2-on.

e) Aminoverbindungen, z.B. Salicyliden-propylendiamin, Salicylaminoguanidin und deren Salze.

<u>Beispiele für Rost-Inhibitoren sind:</u>

a) Organische Säuren, ihre Ester, Metallsalze, Aminsalze und Anhydride, z.B. Alkyl- und Alkenylbernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-(2-Carboxymethyl)-1-dodecyl-3-methylglycerin und dessen Aminsalze.

b) Stickstoffhaltige Verbindungen, z.B.:

I. Primäre, sekundäre oder tertiäre aliphatische oder cycloaliphatische Amine und Aminsalze von organischen und anorganischen Säuren, z.B. öllösliche Alkylammoniumcarboxylate, ferner 1-[N,N-bis-(2-hydroxyethyl)amino]-3-(4-nonylphenoxy)-propan-2-ol.

II. Heterocyclische Verbindungen, z.B.:
Substituierte Imidazoline und Oxazoline, 2-Heptadecenyl-1-(2-hydroxyethyl)-imidazolin.

c) Phosphorhaltige Verbindungen, z.B.:
Aminsalze von Phosphorsäurepartialestern oder Phosphonsäurepartialestern, Zinkdialkyldithiophosphate.

d) Schwefelhaltige Verbindungen, z.B.:
Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.

e) Glycerinderivate, z.B.:
Glycerin-monooleat, 1-(Alkylphenoxy)-3-(2-hydroxyethyl)glycerine, 1-(Alkylphenoxy)-3-(2,3-dihydroxypropyl)glycerine, 2-Carboxyalkyl-1,3-dialkylglycerine.

<u>Beispiele für Viskositätsindex-Verbesserer sind:</u>

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

<u>Beispiele für Stockpunkterniedriger sind:</u>

Polymethacrylat, alkylierte Naphthalinderivate.

<u>Beispiele für Dispergiermittel/Tenside sind:</u>

Polybutenylbernsteinsäureamide oder -imide, Polybutenylphosphonsäurederivate, basische Magnesium-, Calcium-, und Bariumsulfonate und -phenolate.

<u>Beispiele für Verschleissschutz-Additive sind:</u>

Schwefel und/oder Phosphor und/oder Halogen enthaltende Verbindungen, wie geschwefelte Olefine und pfanzliche Oele, Zinkdialkyldithiophosphate, alkylierte Triphenylphosphate, Tritolylphosphat, Tricresylphosphat, chlorierte Paraffine, Alkyl- und Aryldi- und tri-sulfide, Aminsalze von Mono- und Dialkylphosphaten, Aminsalze der Methylphosphonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)-thio]-propionsäure-ethylester, Triphenylthiophosphat (Triphenylphosphorothioat), Tris-(alkylphenyl)phosphorothioate und deren Gemische, (z.B. Tris(isononylphenyl)phosphorothioat), Diphenyl-monononylphenyl-phosphorothioat, Isobutylphenyl-diphenylphosphorothioat, Dodecylaminsalz des 3-Hydroxy-1,3-thiaphosphetan-3-oxids, Trithiophosphorsäure-5,5,5-tris[isooctylacetat (2)], Derivate von 2-Mercaptobenzthiazol wie 1-[N,N-Bis(2-ethylhexyl)aminomethyl-2-mercapto-1H-1,3-benzthiazol, Ethoxycarbonyl-5-octyl-dithiocarbamat.

Die folgenden Beispiele erläutern die Erfindung weiter. Teil- und Prozentangaben beziehen sich darin, soweit nichts anderes angegeben ist, auf das Gewicht.

<u>Beispiel 1</u>: Herstellung der Sonnenblumenöl-Derivate mit Pentaerythrit und 3-(3',5'-Di-tert-butyl-4'-hydroxyphenyl)propionsäuremethylester.

Eine Mischung von 30 g (~34 mMol) Sonnenblumenöl, 4,64 g (34 mMol) Pentaerythrit und 37 mg (0,15 mMol) Dibutylzinnoxid wird in einem Sulfierkolben mit Rückflusskühler und mechanischem Rührer unter Stickstoff während 7 Stunden bei 180-190°C gehalten. Anschliessend werden 9,94 g (34 mMol) 3-(3',5'-Di-tert-butyl-4'-hydroxyphenyl)propionsäuremethylester und nochmals 37 mg (0,15 mMol) Dibutylzinnoxid zugegeben. Das Reaktionsgemisch wird 15 Stunden bei 180-190°C weiter gerührt. Nach dem Abkühlen werden 40,64 g (91 %) Produkt als gelbes Oel mit einem Brecheungsindex $n_D^{20}$ von 1,4882 erhalten.

Beispiel 2: Herstellung der Kokosnussöll-Derivate mit Pentaerythrit und 3-(3',5'-Di-tert-butyl-4'-hydroxyphenyl)propionsäuremethylester.

In analoger Weise werden, wie in Beispiel 1 beschrieben, aus 403 g (~0,616 Mol) Kokosfett, 83,1 g (0,610 Mol) Pentaerythrit, 1,0 g (4 mMol) Dibutylzinnoxid und 178,4 g (0,610 Mol) 3-(3',5'-Di-tert-butyl-4'-hydroxyphenyl)propionsäuremethylester 653 g (98 %) Produkt als braunes Oel mit einem Brechungsindex $n_D^{20}$ von 1,4781 erhalten.

Beispiel 3: Herstellung der Sonnenblumenöl-Derivate mit Thiodiethylenglykol und 3-(3',5'-Di-tert-butyl-4'-hydroxyphenyl)propionsäuremethylester.

In analoger Weise werden, wie in Beispiel 1 beschrieben, aus 50 g (~57 mMol) Sonnenblumenöl, 41,8 g (343 mMol) Thiodiethylenglykol, 448 mg (1,8 mMol) Dibutylzinnoxid und 200 g (684 mMol) 3-(3',5'-Di-tert-butyl-4'-hydroxyphenyl)propionsäuremethylester 279,1 g (96 %) Produkt als gelbes Oel mit einem Brechungsindex $n_D^{20}$ von 1,5170 erhalten.

Beispiel 4: Herstellung der Kokosnussöll-Derivate mit Thiodiethylenglykol und 3-(3',5'-Di-tert-butyl-4'-hydroxyphenyl)propionsäuremethylester.

In analoger Weise, wie in Beispiel 1 beschrieben, werden aus 49,9 g (~76 mMol) Kokosfett, 85,1 g (688 mMol) Thiodiethylenglykol, 797 mg (3,2 mMol) Dibutylzinnoxid und 402,6 g (1,38 Mol) 3-(3',5'-Di-tert-butyl-4'-hydroxyphenyl)propionsäuremethylester 500,7 g (93 %) Produkt als braun-oranges Oel mit einem Brechungsindex $n_D^{20}$ von 1,5210 erhalten.

Beispiel 5: Herstellung der Sonnenblumenöl-Derivate mit 1,4-Butandiol und 3-(3',5'-Di-tert-butyl-4'-hydroxyphenyl)propionsäuremethylester.

In analoger Weise, wie in Beispiel 1 beschrieben, werden aus 30 g (~34 mMol) Sonnenblumenöl, 14 g (155 mMol) 1,4-Butandiol, 199 mg (0,80 mMol) Dibutylzinnoxid und 87,7 g (300 mMol) 3-(3',5'-Di-tert-butyl-4'-hydroxyphenyl)propionsäuremethylester 124 g (94 %) Produkt als rötliches Oel mit einem Brechungsindex $n_D^{20}$ von 1,5070 erhalten.

Beispiel 6: Herstellung der Kokosnussöl-Derivate mit 1,4-Butandiol und 3-(3',5'-Di-tert-butyl-4'-hydroxyphenyl)propionsäuremethylester.

In analoger Weise, wie in Beispiel 1 beschrieben, werden aus 30 g (~46 mMol) Kokosfett, 14 g (155 mMol) 1,4-Butandiol, 199 mg (0,80 mMol) Dibutylzinnoxid und 87,7 g (300 mMol) 3-(3',5'-Di-tert-butyl-4'-hydroxyphenyl)propionsäuremethylester 123 g (93 %) Produkt als rötliches Oel mit einem Brechungsindex $n_D^{20}$ von 1,5025 erhalten.

Beispiel 7: Herstellung der Sonnenblumenöl-Derivate mit 1,2-Propandiol und 3-(3',5'-Di-tert-butyl-4'-hydroxyphenyl)propionsäuremethylester.

In analoger Weise, wie in Beispiel 1 beschrieben, werden aus 30 g (~34 mMol) Sonnenblumenöl, 12,2 g (160 mMol) 1,2-Propandiol, 199 mg (0,80 mMol) Dibutylzinnoxid und 87,7 g (300 mMol) 3-(3',5'-Di-tert-butyl-4'-hydroxyphenyl)propionsäuremethylester 121,7 g (93,7 %) Produkt als gelbes Oel mit einem Brechungsindex $n_D^{20}$ von 1,5047 erhalten.

Beispiel 8: Herstellung der Sonnenblumenöl-Derivate mit Diethylenglykol und 3-(3',5'-Di-tert-butyl-4'-hydroxyphenyl)propionsäuremethylester.

In analoger Weise, wie in Beispiel 1 beschrieben, werden aus 30,1 g (~34 mMol) Sonnenblumenöl, 16,7 g (157 mMol) Diethylenglykol, 199 mg (0,80 mMol) Dibutylzinnoxid und 89,5 g (306 mMol) 3-(3',5'-Di-tert-butyl-4'-hydroxyphenyl)propionsäuremethylester 137,3 g (99 %) Produkt als gelbes Oel mit einem Brechungsindex $n_D^{20}$ von 1,5065 erhalten.

Beispiel 9: Herstellung der Kokosnussöl-Derivate mit Diethylenglykol und 3-(3',5'-Di-tert-butyl-4'-hydroxyphenyl)propionsäuremethylester.

In analoger Weise, wie in Beispiel 1 beschrieben, werden aus 30 g (~46 mMol) Kokosfett, 22,3 g (210 mMol) Diethylenglykol, 249 mg (1,00 mMol) Dibutylzinnoxid und 121,1 g (414 mMol) 3-(3',5'-Di-tert-butyl-4'-hydroxyphenyl)propionsäuremethylester 158,1 g (91 %) Produkt als gelbes Oel mit einem Brechungsindex $n_D^{20}$ von 1,5068 erhal-

ten.

Beispiel 10: Herstellung der Sonnenblumenöl-Derivate mit Triethylenglykol und 3-(3',5'-Di-tert-butyl-4'-hydroxyphenyl)propionsäuremethylester.

In analoger Weise, wie in Beispiel 1 beschrieben, werden aus 30 g (~34 mMol) Sonnenblumenöl, 15 g (100 mMol) Triethylenglykol, 199 mg (0,80 mMol) Dibutylzinnoxid und 90,65 g (310 mMol) 3-(3',5'-Di-tert-butyl-4'-hydroxyphenyl)propionsäuremethylester 129 g (95 %) Produkt als hellgelbes Oel mit einem Brechungsindex $n_D^{20}$ von 1,5050 erhalten.

Beispiel 11: Herstellung der Kokosnussöl-Derivate mit Triethylenglykol und 3-(3',5'-Di-tert-butyl-4'-hydroxyphenyl)propionsäuremethylester.

In analoger Weise, wie in Beispiel 1 beschrieben, werden aus 30 g (~46 mMol) Kokosfett, 15,2 g (100 mMol) Triethylenglykol, 199 mg (0,80 mMol) Dibutylzinnoxid und 90,65 g (310 mMol) 3-(3',5'-Di-tert-butyl-4'-hydroxyphenyl)propionsäuremethylester 129,5 g (95 %) Produkt als leicht gelbes Oel mit einem Brechungsindex $n_D^{20}$ von 1,4992 erhalten.

Beispiel 12: Herstellung der Radiamuls-Derivate mit Diethylenglykol und 3-(3',5'-Di-tert-butyl-4'-hydroxyphenyl)propionsäuremethylester.

In analoger Weise, wie in Beispiel 1 beschrieben, werden aus 30 g (~59 mMol) Radiamuls (Glycerol Tri C8/C10), 16,3 g (154 mMol) Diethylenglykol, 224 mg (0,90 mMol) Dibutylzinnoxid und 95,6 g (327 mMol) 3-(3',5'-Di-tert-butyl-4'-hydroxyphenyl)propionsäuremethylester 132,6 g (99 %) Produkt als leicht gelbes Oel mit einem Brechungsindex $n_D^{20}$ von 1,5022 erhalten.

Beispiel 13: Herstellung der Sonnenblumenöl-Derivate mit Diethanolamin und 3-(3',5'-Di-tert-butyl-4'-hydroxyphenyl)propionsäuremethylester.

In analoger Weise, wie in Beispiel 1 beschrieben, werden aus 60 g (~68 mMol) Sonnenblumenöl, 14,3 g (136 mMol) Diethanolamin, 149 mg (0,60 mMol) Dibutylzinnoxid und 19,9 g (68 mMol) 3-(3',5'-Di-tert-butyl-4'-hydroxyphenyl)propionsäuremethylester 88,4 g (95 %) Produkt als braun-rotes Oel mit einem Brechungsindex $n_D^{20}$ von 1,4940 erhalten.

Beispiel 14: Herstellung der Kokosnussöl-Derivate mit Diethanolamin und 3-(3',5'-Di-tert-butyl-4'-hydroxyphenyl)propionsäuremethylester.

In analoger Weise, wie in Beispiel 1 beschrieben, werden aus 60 g (~92 mMol) Kokosfett, 19,1 g (182 mMol) Diethanolamin, 174 mg (0,70 mMol) Dibutylzinnoxid und 34,2 g (117 mMol) 3-(3',5'-Di-tert-butyl-4'-hydroxyphenyl)propionsäuremethylester 104,9 g (93 %) Produkt als braunes Oel mit einem Brechungsindex $n_D^{20}$ von 1,4905 erhalten.

Beispiel 15: Herstellung der Sonnenblumenöl-Derivate mit Glycerin und 3-(3',5'-Di-tert-butyl-4'-hydroxyphenyl)propionsäuremethylester.

In analoger Weise, wie in Beispiel 1 beschrieben, werden aus 30 g (~34 mMol) Sonnenblumenöl, 14,22 g (154 mMol) Glycerin, 199 mg (0,80 mMol) Dibutylzinnoxid und 87,73 g (300 mMol) 3-(3',5'-Di-tert-butyl-4'-hydroxyphenyl)propionsäuremethylester 126,5 g (96 %) Produkt als hellgelbes, viskoses Oel mit einem Brechungsindex $n_D^{20}$ von 1,5128 erhalten.

Beispiel 16: Herstellung der Kokosnussöl-Derivate mit Glycerin und 3-(3',5'-Di-tert-butyl-4'-hydroxyphenyl)propionsäuremethylester.

In analoger Weise, wie in Beispiel 1 beschrieben, werden aus 30 g (~46 mMol) Kokosfett, 19,4 g (211 mMol) Glycerin, 249 mg (1,0 mMol) Dibutylzinnoxid und 118 g (404 mMol) 3-(3',5'-Di-tert-butyl-4'-hydroxyphenyl)propionsäuremethylester 154 g (92 %) Produkt als hellgelbes, viskoses Oel mit einem Brechungsindex $n_D^{20}$ von 1,5123 erhalten.

Beispiel 17: Herstellung der Sonnenblumenöl-Derivate mit Glycerin und 3-(3'-tert-Butyl-4'-hydroxy-5'-methylphenyl)propionsäuremethylester.

In analoger Weise, wie in Beispiel 1 beschrieben, werden aus 30 g (~34 mMol) Sonnenblumenöl, 14,5 g (157 mMol) Glycerin, 180 mg (0,72 mMol) Dibutylzinnoxid und 75,20 g (300 mMol) 3-(3'-tert-Butyl-4'-hydroxy-5'-methylphenyl)propionsäuremethylester 105,0 g (96 %) Produkt als oranges Oel mit einem Brechungsindex $n_D^{20}$ von 1,5165 erhalten.

Beispiel 18: Herstellung der Kokosnussöl-Derivate mit Diethylenglykol und 3-(3'-tert-Butyl-4'-hydroxy-5'-methylphenyl)propionsäuremethylester.

In analoger Weise, wie in Beispiel 1 beschrieben, werden aus 30 g (~46 mMol) Kokosfett, 22,6 g (213 mMol) Diethylenglykol, 184 mg (0,70 mMol) Dibutylzinnoxid und 94,3 g (390 mMol) 3-(3'-tert-Butyl-4'-hydroxy-5'-methylphenyl)propionsäuremethylester 131,9 g (98 %) Produkt als gelbes Oel mit einem Brechungsindex $n_D^{20}$ von 1,5118 erhalten.

Beispiel 19: Herstellung der Sonnenblumenöl-Derivate mit 4-Hydroxy-2,2,6,6-tetramethylpiperidin und 3-(3',5'-Di-tert-butyl-4'-hydroxyphenyl)propionsäuremethylester.

In analoger Weise, wie in Beispiel 1 beschrieben, werden aus 41,5 g (~47 mMol) Sonnenblumenöl, 7,90 g (50 mMol) 4-Hydroxy-2,2,6,6-tetramethylpiperidin, 50 mg (0,20 mMol) Dibutylzinnoxid und 14,60 g (50 mMol) 3-(3',5'-Di-tert-butyl-4'-hydroxyphenyl)propionsäuremethylester 59,4 g (95 %) Produkt als braunes Oel mit einem Brechungsindex $n_D^{20}$ von 1,4848 erhalten.

Beispiel 20: Herstellung der Sonnenblumenöl-Derivate mit 4-Amino-2,2,6,6-tetramethylpiperidin und 3-(3',5'-Di-tert-butyl-4'-hydroxyphenyl)propionsäuremethylester.

In analoger Weise, wie in Beispiel 1 beschrieben, werden aus 41,5 g (~47 mMol) Sonnenblumenöl, 7,80 g (50 mMol) 4-Amino-2,2,6,6-tetramethylpiperidin, 50 mg (0,20 mMol) Dibutylzinnoxid und 14,60 g (50 mMol) 3-(3',5'-Di-tert-butyl-4'-hydroxyphenyl)propionsäuremethylester 61,8 g (99 %) Produkt als braunes Oel mit einem Brechungsindex $n_D^{20}$ von 1,4887 erhalten.

Beispiel 21: Herstellung der Kokosnussöl-Derivate mit N-(2-Hydroxyethyl)-4-hydroxy-2,2,6,6-tetramethylpiperidin und 3-(3',5'-Di-tert-butyl-4'-hydroxyphenyl)propionsäuremethylester.

In analoger Weise, wie in Beispiel 1 beschrieben, werden aus 31,0 g (~47 mMol) Kokosfett, 10,1 g (50 mMol) N-(2-Hydroxyethyl)-4-hydroxy-2,2,6,6-tetramethylpiperidin, 50 mg (0,20 mMol) Dibutylzinnoxid und 14,60 g (50 mMol) 3-(3',5'-Di-tert-butyl-4'-hydroxyphenyl)propionsäuremethylester 52,4 g (98 %) Produkt als gelbes Oel mit einem Brechungsindex $n_D^{20}$ von 1,4811 erhalten.

Beispiel 22: Herstellung der Rapsöl-Derivate mit Glycerin und 3-(3',5'-Di-tert-butyl-4'-hydroxyphenyl)propionsäuremethylester.

Eine Mischung von 116,3 g (~134 mMol) Rapsöl, 86,1 g (935 mMol) 85 % wässrigem Glycerin und 2,64 g (15,0 mMol) Calciumacetat wird in einem Sulfierkolben mit Rückflusskühler und mechanischem Rührer unter Stickstoff-Atmosphäre während 7 Stunden bei 180-190°C gehalten. Anschliessend werden 357,5 g (1,22 Mol) 3-(3',5'-Di-tert-butyl-4'-hydroxyphenyl)propionsäuremethylester zugegeben. Das Reaktionsgemisch wird 15 Stunden bei 180-190°C weiter gerührt. Nach dem Abkühlen werden 505 g (99 %) Produkt als gelbes Oel mit einem Brechungsindex $n_D^{20}$ von 1,5122 erhalten.

Beispiel 23: Herstellung der Maiskeimöl-Derivate mit Glycerin und 3-(3',5'-Di-tert-butyl-4'-hydroxyphenyl)propionsäuremethylester.

In analoger Weise, wie in Beispiel 22 beschrieben, werden aus 100 g (~113 mMol) Maiskeimöl, 57,5 g (624 mMol) 85 % wässrigem Glycerin, 2,32 g (13,0 mMol) Calciumacetat und 282,4 g (966 mMol) 3-(3',5'-Di-tert-butyl-4'-hydroxyphenyl)propionsäuremethylester 399,0 g (99 %) Produkt als gelbes Oel mit einem Brechungsindex $n_D^{20}$ von 1,5127 erhalten.

Beispiel 24: Herstellung der Distelöl-Derivate mit Glycerin und 3-(3',5'-Di-tert-butyl-4'-hydroxyphenyl)propionsäuremethylester.

In analoger Weise, wie in Beispiel 22 beschrieben, werden aus 100 g (~113 mMol) Distelöl, 57,6 g (625 mMol) 85 % wässrigem Glycerin, 2,11 g (12,0 mMol) Calciumacetat und 286,5 g (980 mMol) 3-(3',5'-Di-tert-butyl-4'-hydroxyphenyl)propionsäuremethylester 403,2 g (99 %) Produkt als gelbes Oel mit einem Brechungsindex $n_D^{20}$ von 1,5140 erhalten.

Beispiel 25: Herstellung der Olivenöl-Derivate mit Glycerin und 3-(3',5'-Di-tert-butyl-4'-hydroxyphenyl)propionsäuremethylester.

In analoger Weise, wie in Beispiel 22 beschrieben, werden aus 100 g (~114 mMol) Olivenöl, 58,0 g (630 mMol) 85 % wässrigem Glycerin, 2,11 g (12,0 mMol) Calciumacetat und 290,4 g (993 mMol) 3-(3',5'-Di-tert-butyl-4'-hydroxyphenyl)propionsäuremethylester 408,6 g (99 %) Produkt als gelbes Oel mit einem Brechungsindex $n_D^{20}$ von 1,5110 erhalten.

Beispiel 26: Herstellung der Erdnussöl-Derivate mit Glycerin und 3-(3',5'-Di-tert-butyl-4'-hydroxyphenyl)propionsäuremethylester.

In analoger Weise, wie in Beispiel 22 beschrieben, werden aus 100 g (~114 mMol) Erdnussöl, 58,0 g (630 mMol) 85 % wässrigem Glycerin, 2,11 g (12,0 mMol) Calciumacetat und 291,4 g (997 mMol) 3-(3',5'-Di-tert-butyl-4'-hydroxyphenyl)propionsäuremethylester 413,5 g (99 %) Produkt als gelbes Oel mit einem Brechungsindex $n_D^{20}$ von 1,5100 erhalten.

Beispiel 27: "Deposit and Oxidation Panel Test" (DOPT)

Beim "Deposit and Oxidation Panel Test" (DOPT) handelt es sich um eine Variante einer Prüfmethode für Motorenöle, insbesondere für Dieselmotorenöle, welche durch G. Abellaneda et al, IIIè Symposium CEC, 1989, 61, New Cavendish Street, London WIM8AR, England beschrieben wurde. Dabei wird die Eignung der Oele mit Stabilisator zur Verhinderung von Ablagerungen auf den Kolben geprüft.

Die Testdauer beträgt 20 Stunden, die Panel-Temperatur 260°C und der Oelfluss 1 ml/Minute. Die feuchte Luft-Atmosphäre wird mit 260 ppm $NO_2$ und 26 ppm $SO_2$ angereichert. Nach dem Test wird die Metallplatte (Panel), auf die das Oel tropft, gewogen und visuell bewertet. Je kleiner die Zahlen sind, desto besser. Als Schmieroel wird ein handelsübliches CD-Oel, welches mit dem Grundoel STANCO 150 verdünnt wird, verwendet. Diesem vorbereiteten Oel werden nun die in Tabelle 1 angegebenen Stabilisatoren in einer Menge von 0,6 Gew-% bezogen auf das Oel, zugemischt und einer "DOPT-Prüfung" unterzogen.

Tabelle 1

| "Deposit and Oxidation Panel Test" (DOPT) | | | |
|---|---|---|---|
| Produkt gemäss Beispiel | Konz. in Gew.-% | Ablagerungen auf Panel | |
| | | Gewicht (mg) | visuell |
| 8 | 0,6 | 14 | 4 |
| 9 | 0,6 | 10 | 4 |
| 10 | 0,6 | 11 | 6 |
| 15 | 0,6 | 14 | 6 |
| ohne Zusatz | ohne Zusatz | 72 | 14 |

**Patentansprüche**

1. Produkt, erhältlich durch Umsetzung der Komponenten a), b) und c), wobei die Komponente a) eine Verbindung der Formel I oder ein Gemisch von Verbindungen der Formel I, die Komponente b) eine Verbindung der Formel II

oder ein Gemisch von Verbindungen der Formel II und die Komponente c) eine Verbindung der Formel III oder ein Gemisch von Verbindungen der Formel III sind,

$X(Y)_a$ , 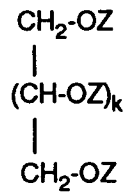 , 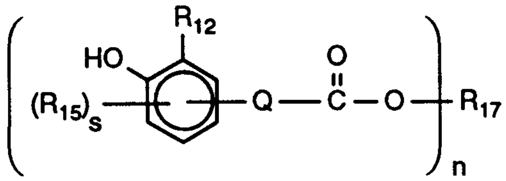

(I) (II) (III)

wobei in der Verbindung der Formel I die Reste

Y unabhängig voneinander OH, $(HOCH_2CH_2)_2N$- oder $-HNR_1$ darstellt und

$R_1$ Wasserstoff, $C_1$-$C_{18}$-Alkyl, $C_5$-$C_{12}$-Cycloalkyl,

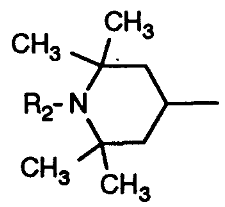 ,

$C_3$-$C_6$-Alkenyl, $C_7$-$C_9$-Phenylalkyl, Phenyl oder durch 1 bis 3 Reste $A_1$ substituiertes Phenyl bedeutet, wobei die Reste $A_1$ unabhängig voneinander $C_1$-$C_{12}$-Alkyl, Halogen, Hydroxy, Methoxy oder Ethoxy bedeuten, wobei

$R_2$ Wasserstoff, $C_1$-$C_8$-Alkyl, $O^{\bullet}$, OH, NO, $-CH_2CN$, $C_1$-$C_{18}$-Alkoxy, $C_5$-$C_{12}$-Cycloalkoxy, $C_3$-$C_6$-Alkenyl, $C_7$-$C_9$-Phenylalkyl oder $C_7$-$C_9$-Phenylalkyl, welches am Phenylring mit $C_1$-$C_4$-Alkyl mono-, di- oder tri-substituiert ist, oder $R_2$ ferner $C_1$-$C_8$-Acyl oder $HOCH_2CH_2$- darstellt, und

a die Zahl 1,2,3,4 oder 6 bedeutet, wobei

wenn Y = OH und a = 1 ist,

X $C_1$-$C_{45}$-Alkyl, $C_3$-$C_{18}$-Alkenyl, $-CH_2CH_2T_1(CH_2CH_2O)_bR_4$ oder

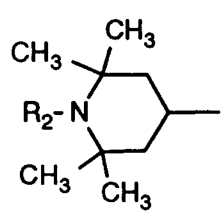

bedeutet, wobei $R_2$ die obige Bedeutung hat, und

$T_1$ Sauerstoff, Schwefel oder $>N-R_5$,

$R_4$ $C_1$-$C_{20}$-Alkyl,

b eine ganz Zahl aus dem Bereich von 0 bis 10 und

$R_5$ Wasserstoff, $C_1$-$C_{18}$-Alkyl oder Phenyl darstellt, oder

wenn Y = OH und a = 2 ist,

X $-CH_2CH_2T_2(CH_2CH_2O)_bCH_2CH_2$-, wobei b die obige Bedeutung hat,

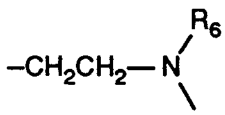 , $-C_cH_{2c}-$ , 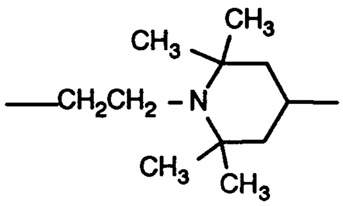 ,

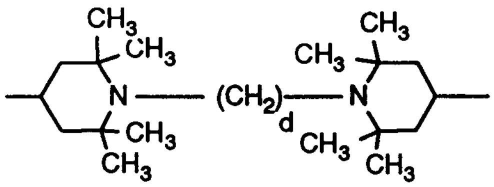 , $-CH_2\text{-}CH{=}CH\text{-}CH_2-$ ,

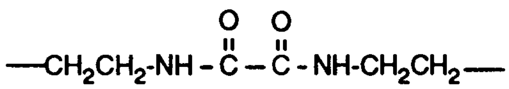 oder

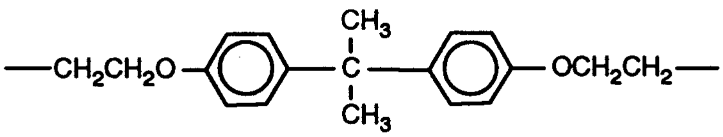

bedeutet, wobei

$T_2$ Sauerstoff, Schwefel, $>N\text{-}R_5$ oder

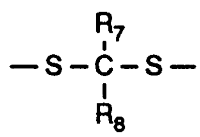

ist und $R_5$ die obige Bedeutung hat,

$R_6$ Wasserstoff, $C_1$-$C_{18}$-Alkyl oder Phenyl,

c eine ganze Zahl aus dem Bereich von 2 bis 10,

d eine ganze Zahl aus dem Bereich von 2 bis 6 und

$R_7$, $R_8$ unabhängig voneinander Wasserstoff, $C_1$-$C_{18}$-Alkyl oder Phenyl sind, oder $R_7$ und $R_8$ mit dem C-Atom, an das sie gebunden sind, einen $C_5$-$C_{12}$-Cycloalkyl-Ring bilden, oder

wenn a = 3 ist,

X $C_3$-$C_{10}$-Alkantriyl oder $N(CH_2CH_2\text{-})_3$ bedeutet, oder

wenn Y = OH und a = 4 ist,

X $C_4$-$C_{10}$-Alkantetrayl,

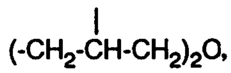,

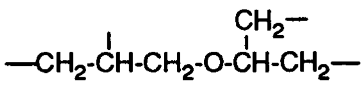, 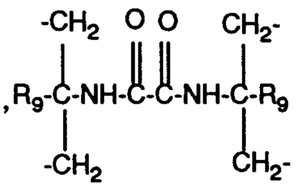, 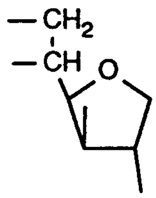 oder

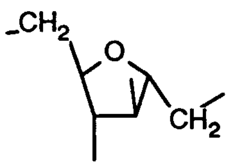

darstellt, wobei

$R_9$ $C_1$-$C_4$-Alkyl bedeutet, oder

wenn Y = OH und a = 6 ist,

X

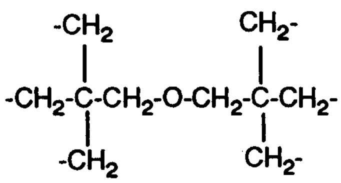, 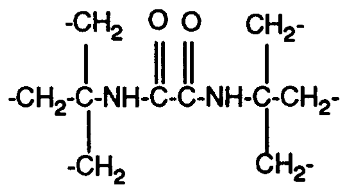

oder $C_6$-$C_{10}$-Alkanhexayl darstellt, oder

wenn Y = $-HNR_1$ und a = 1 ist,

X $C_1$-$C_{18}$-Alkyl, $C_3$-$C_{18}$-Alkenyl, $C_5$-$C_{12}$-Cycloalkyl, $C_7$-$C_9$-Phenylalkyl, Phenyl,

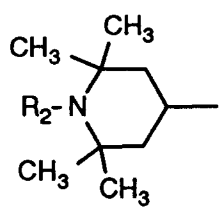,

wobei $R_2$ die obige Bedeutung hat, oder X ferner

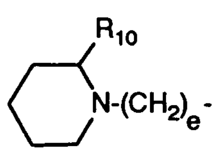

ist, oder X mit $R_1$ gemeinsam eine Gruppe der Formel $-CH_2CH_2CH_2CH_2CH_2-$ oder $-CH_2CH_2OCH_2CH_2-$ bildet, wobei

$R_{10}$ Wasserstoff oder Methyl, und

e 2 oder 3 bedeutet, oder

wenn Y = $-HNR_1$ und a = 2 ist,

X $-C_fH_{2f}-$,

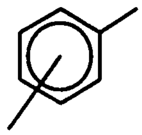 oder $-(CH_2CH_2\overset{H}{\overset{|}{N}})_g-CH_2CH_2-$

darstellt, wobei
f eine ganze Zahl aus dem Bereich von 2 bis 10 und
g eine ganze Zahl aus dem Bereich von 1 bis 6 bedeutet, und

in der Verbindung der Formel II die Reste

Z Wasserstoff oder eine Gruppe der Formel

$$-(C_hH_{2h}O)_i-\overset{O}{\overset{\|}{C}}-R_{11}$$

bedeutet, und
k eine ganze Zahl aus dem Bereich von 0 bis 6 darstellt, wobei
h 2 oder 3,
i eine ganze Zahl aus dem Bereich von 0 bis 12 und
$R_{11}$ $C_1$-$C_{30}$-Alkyl, $C_8$-$C_{30}$-Alkenyl, $C_5$-$C_{12}$-Cycloalkyl, Phenyl oder $C_7$-$C_9$-Phenylalkyl bedeutet, mit der Bedingung, dass die Verbindung der Formel II eine Gruppe

$$-(C_hH_{2h}O)_i-\overset{O}{\overset{\|}{C}}-R_{11}$$

enthält;

in der Verbindung der Formel III

$R_{12}$ $C_1$-$C_{18}$-Alkyl, $C_5$-$C_{12}$-Cycloalkyl, Phenyl oder $C_7$-$C_9$-Phenylalkyl bedeutet,
$R_{15}$ Wasserstoff, $C_1$-$C_{18}$-Alkyl, $C_5$-$C_{12}$-Cycloalkyl, Phenyl oder $C_7$-$C_9$-Phenylalkyl darstellt,
s 0, 1 oder 2 bedeutet,
Q $-C_mH_{2m}-$,

$-CH_2-\underset{R_{16}}{\underset{|}{CH}}-$ oder 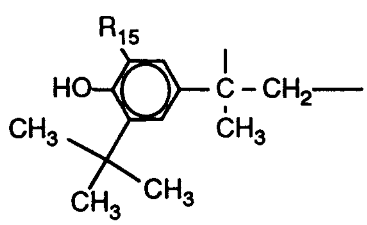

darstellt, wobei $R_{15}$ die obige Bedeutung hat,
m eine ganze Zahl aus dem Bereich von 0 bis 3 bedeutet,
$R_{16}$ $C_1$-$C_8$-Alkyl darstellt und
n eine ganze Zahl aus dem Bereich von 1 bis 6 bedeutet, wobei

wenn n = 1 ist,

$R_{17}$ Wasserstoff, $C_1$-$C_{45}$-Alkyl, $C_5$-$C_{12}$-Cycloalkyl, $C_2$-$C_{18}$-Alkenyl, einen einwertigen Rest einer Hexose, einen einwertigen Rest eines Hexitols,

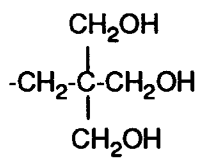 , 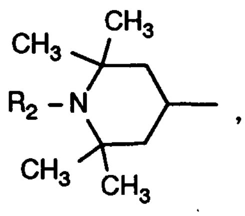 ,

wobei $R_2$ die obige Bedeutung hat, oder ferner $R_{17}$ $-CH_2CH_2-T_3-R_{19}$ oder

$$-\left[(CH_2)_pO\right]_q-(CH_2)_pOR_{19}$$

bedeutet, wobei

$T_3$ Sauerstoff, Schwefel oder >N-$R_{22}$ darstellt,

$R_{19}$

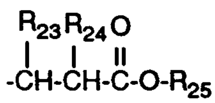 oder 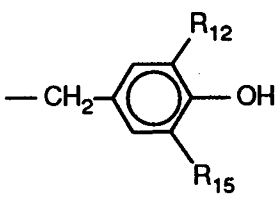

ist, wobei $R_{12}$ und $R_{15}$ die obige Bedeutung haben, oder $R_{19}$ ferner Wasserstoff, $C_1$-$C_{24}$-Alkyl, Phenyl, $C_5$-$C_{12}$-Cycloalkyl oder

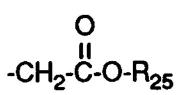

bedeutet, wobei

p eine ganze Zahl aus dem Bereich von 2 bis 4 darstellt,

q eine ganze Zahl aus dem Bereich von 2 bis 20 bedeutet,

$R_{22}$ $C_1$-$C_{18}$-Alkyl, Phenyl oder durch 1 bis 3 Reste $A_1$ substituiertes Phenyl bedeutet, wobei die Reste $A_1$ unabhängig voneinander $C_1$-$C_{12}$-Alkyl, Halogen, Hydroxy, Methoxy oder Ethoxy bedeuten, oder $R_{22}$ ferner $C_5$-$C_8$-Cycloalkyl darstellt,

$R_{23}$, $R_{24}$ unabhängig voneinander Wasserstoff oder Methyl bedeuten mit der Massgabe, dass $R_{23}$ und $R_{24}$ nicht gleichzeitig Methyl sind;

$R_{25}$ Wasserstoff oder $C_1$-$C_{24}$-Alkyl bedeutet, oder

wenn n = 2 ist,

$R_{17}$ einen zweiwertigen Rest einer Hexose, einen zweiwertigen Rest eines Hexitols,

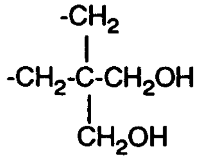, 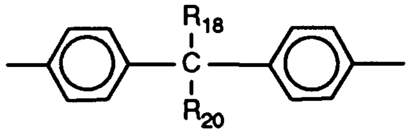, $-C_rH_{2r}-$,

$-[(CH_2)_pO]_q-(CH_2)_p-$,

wobei p und q die obige Bedeutung haben,
$-CH_2CH_2-T_4-CH_2CH_2-$, $-CH_2-CH=CH-CH_2-$, $-CH_2-C \equiv C-CH_2-$,

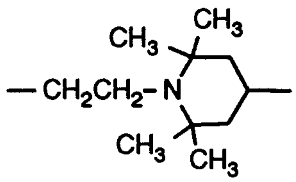,

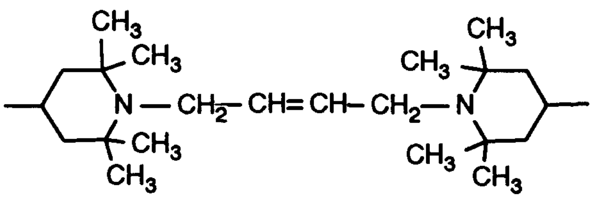,

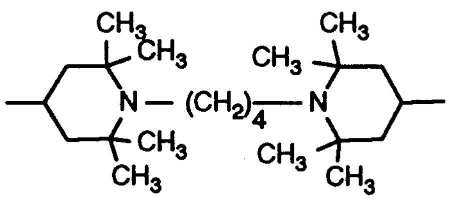, 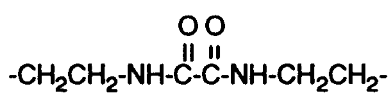 oder

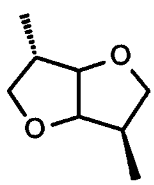

darstellt, wobei

| | |
|---|---|
| $R_{18}$, $R_{20}$ | unabhängig voneinander Wasserstoff oder $C_1$-$C_{12}$-Alkyl bedeuten oder zusammen den Rest $-CH_2CH_2CH_2CH_2CH_2-$ bilden, |
| r | eine ganze Zahl aus dem Bereich von 2 bis 10 darstellt, |
| $T_4$ | Schwefel, >N-$R_{26}$ oder |

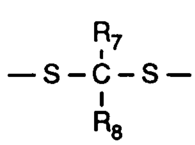

bedeutet, wobei $R_7$ und $R_8$ die obige Bedeutung haben, und

| | |
|---|---|
| $R_{26}$ | Wasserstoff, $C_1$-$C_{18}$-Alkyl, Phenyl oder durch 1 bis 3 Reste $A_1$ substituiertes Phenyl, wobei die Reste $A_1$ die obige für die Formel I beschriebene Bedeutung haben, oder $R_{26}$ ferner $C_5$-$C_8$-Cycloalkyl oder |

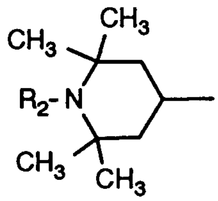

bedeutet, wobei $R_2$ die obige Bedeutung hat, oder

wenn n = 3 ist,

$R_{17}$ einen dreiwertigen Rest einer Hexose, einen dreiwertigen Rest eines Hexitols,

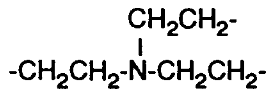 , 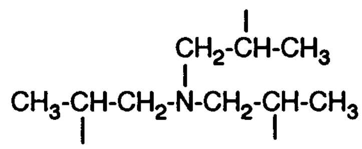 oder 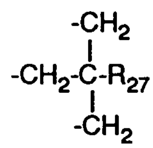

darstellt, wobei

$R_{27}$ Wasserstoff, $-CH_2OH$, $C_1$-$C_4$-Alkyl, $C_1$-$C_{18}$-Alkylamido oder

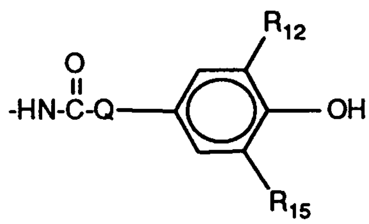

bedeutet, wobei Q, $R_{12}$ und $R_{15}$ die obige Bedeutung haben, oder

wenn n = 4 ist,

$R_{17}$ einen vierwertigen Rest einer Hexose, einen vierwertigen Rest eines Hexitols, $C_4$-$C_{10}$-Alkantetrayl,

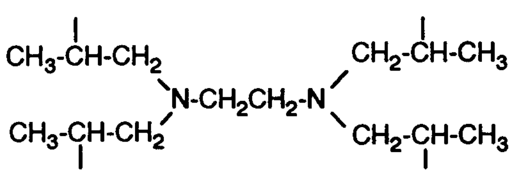 , 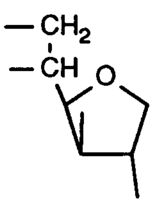 oder

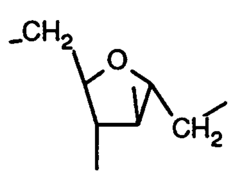

darstellt, oder

wenn n = 5 ist,

$R_{17}$ einen fünfwertigen Rest einer Hexose oder einen fünfwertigen Rest eines Hexitols darstellt, oder

wenn n = 6 ist,

$R_{17}$ einen sechswertigen Rest eines Hexitols oder

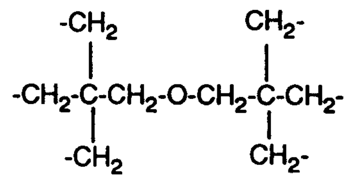

bedeutet.

2. Produkt gemäss Anspruch 1, wobei in der Verbindung der Formel III s die Zahl 1 oder 2 bedeutet.

3. Produkt gemäss Anspruch 1,
wobei in der Verbindung der Formel I die Reste

Y unabhängig voneinander OH, $(HOCH_2CH_2)_2N$- oder $-HNR_1$ bedeutet und
$R_1$ Wasserstoff, $C_1$-$C_{10}$-Alkyl, $C_5$-$C_7$-Cycloalkyl,

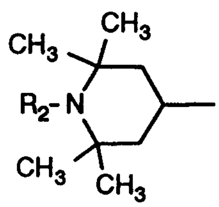

,

$C_3$-$C_6$-Alkenyl, Benzyl oder Phenyl bedeutet, wobei
$R_2$ Wasserstoff, $C_1$-$C_4$-Alkyl, OH, $-CH_2CN$, $C_6$-$C_{12}$-Alkoxy, $C_5$-$C_8$-Cycloalkoxy, Allyl, Benzyl, Acetyl oder $HOCH_2CH_2$- darstellt, und
a die Zahl 1,2,3,4 oder 6 bedeutet, wobei

wenn Y = OH und a = 1 ist,

X $C_1$-$C_{30}$-Alkyl, $C_3$-$C_{18}$-Alkenyl, $-CH_2CH_2T_1(CH_2CH_2O)_bR_4$ oder

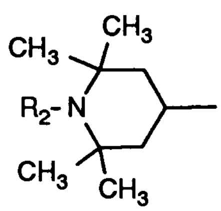

bedeutet, wobei $R_2$ die obige Bedeutung hat, und
$T_1$ Sauerstoff, Schwefel oder $>N$-$R_5$,
$R_4$ $C_1$-$C_{10}$-Alkyl,
b eine ganze Zahl aus dem Bereich von 0 bis 10 und
$R_5$ Wasserstoff, $C_1$-$C_{10}$-Alkyl oder Phenyl darstellen, oder

wenn Y = OH und a = 2 ist,

X $-CH_2CH_2T_2(CH_2CH_2O)_bCH_2CH_2-$, wobei b die obige Bedeutung hat,

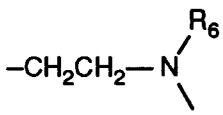 , $-C_cH_{2c}-$ , 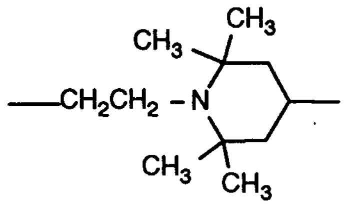 ,

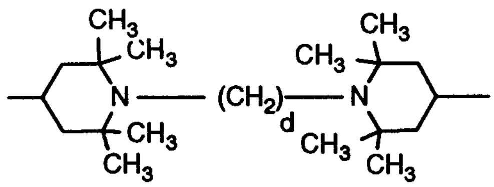 , $-CH_2\text{-}CH{=}CH\text{-}CH_2-$ ,

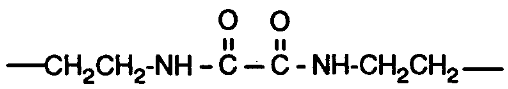 oder

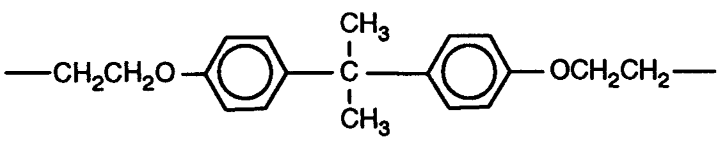

bedeutet, wobei

$T_2$ Sauerstoff, Schwefel, >N-$R_5$ oder

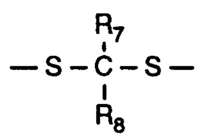

ist und $R_5$ die obige Bedeutung hat,

$R_6$ Wasserstoff, $C_1$-$C_{10}$-Alkyl oder Phenyl,

c eine ganze Zahl aus dem Bereich von 2 bis 10,

d eine ganze Zahl aus dem Bereich von 2 bis 6 und

$R_7$, $R_8$ unabhängig voneinander Wasserstoff, $C_1$-$C_{10}$-Alkyl oder Phenyl sind, oder $R_7$ und $R_8$ mit dem C-Atom, an das sie gebunden sind, einen $C_5$-$C_7$-Cycloalkyl-Ring bilden, oder

wenn Y = $-HNR_1$ und a = 1 ist,

X $C_1$-$C_{10}$-Alkyl, $C_3$-$C_{18}$-Alkenyl, $C_5$-$C_7$-Cycloalkyl, Benzyl, Phenyl,

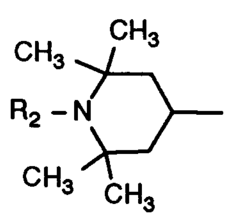 ,

wobei $R_2$ die obige Bedeutung hat, oder X ferner

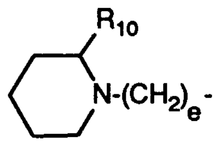

ist, oder X mit $R_1$ gemeinsam eine Gruppe der Formel $-CH_2CH_2CH_2CH_2CH_2-$ oder $-CH_2CH_2OCH_2CH_2-$ bildet, wobei

$R_{10}$ Wasserstoff oder Methyl ist, und
e 2 oder 3 bedeutet, und

in der Verbindung der Formel II die Reste

Z Wasserstoff oder eine Gruppe der Formel

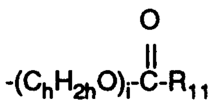

bedeuten, und
k eine ganze Zahl aus dem Bereich von 0 bis 4 darstellt, wobei
h 2 oder 3,
i eine ganze Zahl aus dem Bereich von 0 bis 6 und
$R_{11}$ $C_1$-$C_{20}$-Alkyl, $C_8$-$C_{20}$-Alkenyl, $C_5$-$C_7$-Cycloalkyl, Phenyl oder Benzyl bedeuten, mit der Bedingung, dass die Verbindung der Formel II eine Gruppe

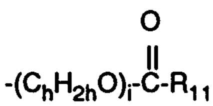

enthält;

in der Verbindung der Formel III

$R_{12}$ $C_1$-$C_6$-Alkyl, $C_5$-$C_7$-Cycloalkyl, Phenyl oder Benzyl bedeutet,
$R_{15}$ Wasserstoff, $C_1$-$C_6$-Alkyl, $C_5$-$C_7$-Cycloalkyl, Phenyl oder Benzyl darstellt,
s 1 oder 2 bedeutet,
Q $-C_mH_{2m}-$,

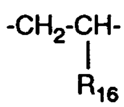 oder 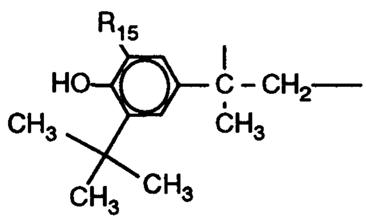

darstellt, wobei $R_{15}$ die obige Bedeutung hat,
m eine ganze Zahl aus dem Bereich von 0 bis 3 bedeutet,
$R_{16}$ $C_1$-$C_4$-Alkyl darstellt und
n eine ganze Zahl aus dem Bereich von 1 bis 6 bedeutet, wobei

wenn n = 1 ist,

$R_{17}$ Wasserstoff, $C_1$-$C_{30}$-Alkyl, $C_5$-$C_7$-Cycloalkyl, $C_2$-$C_{18}$-Alkenyl, einen einwertigen Rest einer Hexose, einen einwertigen Rest eines Hexitols,

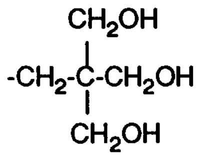 , 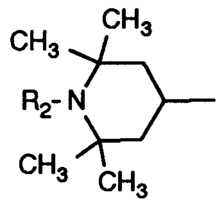 ,

wobei $R_2$ die obige Bedeutung hat, oder ferner $R_{17}$ $-CH_2CH_2-T_3-R_{19}$ oder

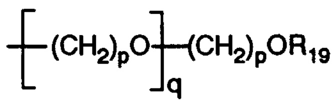

bedeutet, wobei

$T_3$ Sauerstoff, Schwefel oder >N-$R_{22}$ darstellt,

$R_{19}$

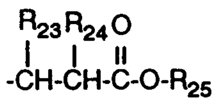 , 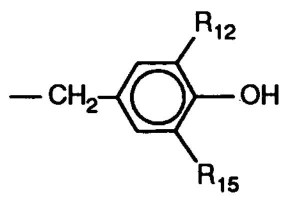 ,

wobei $R_{12}$ und $R_{15}$ die obige Bedeutung haben, oder $R_{19}$ ferner Wasserstoff, $C_1$-$C_{18}$-Alkyl, Phenyl, $C_5$-$C_7$-Cycloalkyl oder

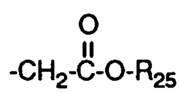

bedeutet, wobei

p eine ganze Zahl aus dem Bereich von 2 bis 4 darstellt,

q eine ganze Zahl aus dem Bereich von 2 bis 20 bedeutet,

$R_{22}$ $C_1$-$C_{10}$-Alkyl, Phenyl oder $C_5$-$C_8$-Cycloalkyl darstellt,

$R_{23}$, $R_{24}$ unabhängig voneinander Wasserstoff oder Methyl bedeuten, mit der Massgabe, dass $R_{23}$ und $R_{24}$ nicht gleichzeitig Methyl sind;

$R_{25}$ Wasserstoff oder $C_1$-$C_{18}$-Alkyl bedeutet, oder

wenn n = 2 ist,

$R_{17}$ einen zweiwertigen Rest einer Hexose, einen zweiwertigen Rest eines Hexitols,

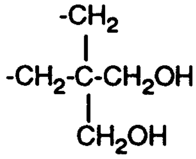, 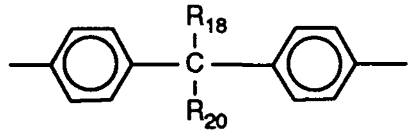, $-C_rH_{2r}-$,

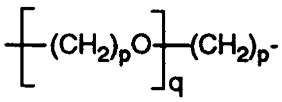,

wobei p und q die obige Bedeutung haben, $-CH_2CH_2-T_4-CH_2CH_2-$, $-CH_2-CH=CH-CH_2-$, $-CH_2-C \equiv C-CH_2-$,

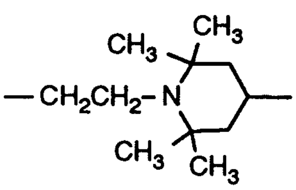, 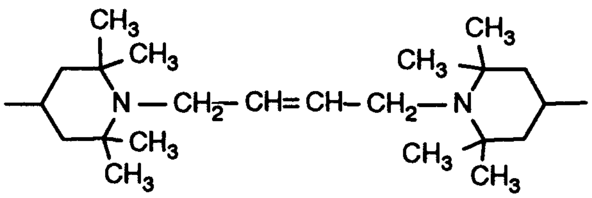,

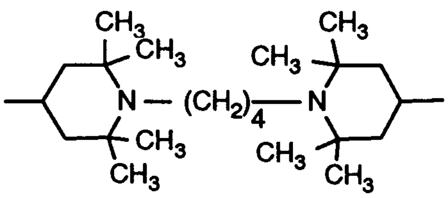, 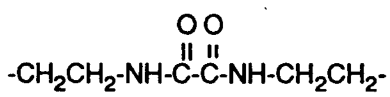 oder

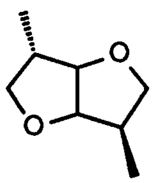

darstellt, wobei

$R_{18}$, $R_{20}$ unabhängig voneinander Wasserstoff oder $C_1$-$C_6$-Alkyl bedeuten oder zusammen den Rest $-CH_2CH_2CH_2CH_2CH_2-$ bilden,

r eine ganze Zahl aus dem Bereich von 2 bis 10 darstellt,

$T_4$ Schwefel, $>N-R_{26}$ oder

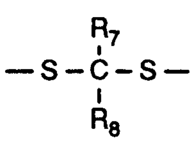

bedeutet, wobei $R_7$ und $R_8$ die obige Bedeutung haben und

$R_{26}$ Wasserstoff, $C_1$-$C_{10}$-Alkyl, Phenyl, $C_5$-$C_8$-Cycloalkyl oder

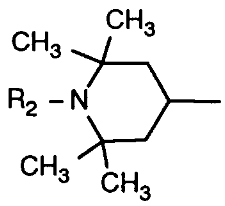

bedeutet, wobei $R_2$ die obige Bedeutung hat.

**4.** Produkt gemäss Anspruch 1, wobei in der Verbindung der Formel I die Reste

Y unabhängig voneinander OH, $(HOCH_2CH_2)_2N$- oder $-HNR_1$ darstellt und

$R_1$ Wasserstoff, $C_1$-$C_4$-Alkyl oder

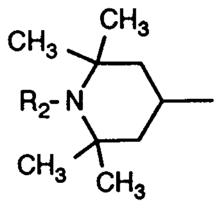

bedeutet, wobei

$R_2$ Wasserstoff, $C_1$-$C_4$-Alkyl, OH, Allyl, Benzyl, Acetyl oder $HOCH_2CH_2$- darstellt, und

a die Zahl 1,2,3,4 oder 6 bedeutet, wobei

wenn Y = OH und a = 1 ist,

X $C_1$-$C_{18}$-Alkyl, $C_3$-$C_{18}$-Alkenyl, $-CH_2CH_2T_1(CH_2CH_2O)_bR_4$ oder

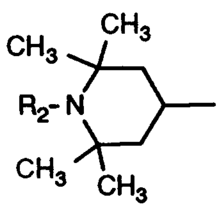

bedeutet, wobei $R_2$ die obige Bedeutung hat, und
$T_1$ Sauerstoff,
$R_4$ $C_1$-$C_4$-Alkyl und
b eine ganze Zahl aus dem Bereich von 0 bis 10 darstellt, oder

wenn Y = OH und a = 2 ist,

X $-CH_2CH_2T_2(CH_2CH_2O)_bCH_2CH_2$-, wobei b die obige Bedeutung hat, oder ferner X $-C_cH_{2c}-$,

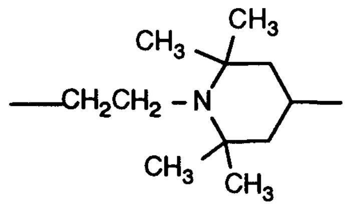

oder $—CH_2\text{-}CH{=}CH\text{-}CH_2—$ bedeutet, wobei

| | |
|---|---|
| $T_2$ | Sauerstoff, Schwefel oder >N-$R_5$, |
| $R_5$ | Wasserstoff, |
| b | die Zahl 0 oder 1, und |
| c | eine ganze Zahl aus dem Bereich von 2 bis 8 darstellen, oder |

wenn a = 3 ist,

X

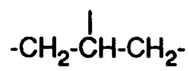

oder $N(CH_2CH_2\text{-})_3$ bedeutet, oder

wenn Y = OH und a = 4 ist,

X

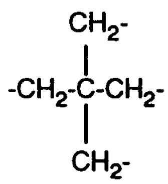
,
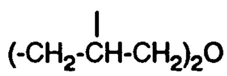
oder
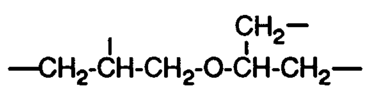

darstellt, oder

wenn Y = OH und a = 6 ist,

X

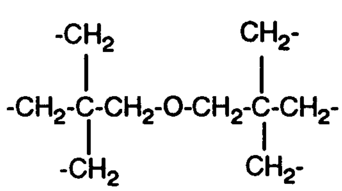
oder
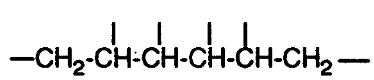

darstellt, oder

wenn Y = $-HNR_1$ und a = 1 ist,

X $C_1$-$C_{10}$-Alkyl, $C_3$-$C_{18}$-Alkenyl, $C_5$-$C_7$-Cycloalkyl oder

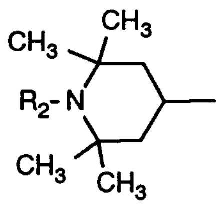

bedeutet, wobei $R_2$ die obige Bedeutung hat, oder

wenn Y = $-HNR_1$ und a = 2 ist,

X $-C_fH_{2f}-$ darstellt, wobei
f eine ganze Zahl aus dem Bereich von 2 bis 10 bedeutet, und

in der Verbindung der Formel II die Reste

Z Wasserstoff oder eine Gruppe der Formel

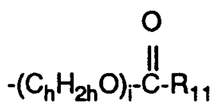

bedeutet, und
k 1, 2 oder 3 darstellt,
h 2 oder 3 bedeutet,
i eine ganze Zahl aus dem Bereich von 0 bis 4 ist und
$R_{11}$ $C_1$-$C_{20}$-Alkyl oder $C_8$-$C_{20}$-Alkenyl bedeutet, mit der Bedingung, dass die Verbindung der Formel II eine Gruppe

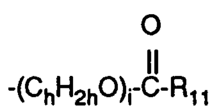

enthält;

in der Verbindung der Formel III

$R_{12}$ $C_1$-$C_6$-Alkyl oder $C_5$-$C_7$-Cycloalkyl bedeutet,
$R_{15}$ Wasserstoff, $C_1$-$C_6$-Alkyl oder $C_5$-$C_7$-Cycloalkyl darstellt,
s 1 oder 2 bedeutet,
Q $-C_mH_{2m}-$ oder

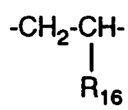

darstellt,
m eine ganze Zahl aus dem Bereich von 0 bis 3 bedeutet,
$R_{16}$ $C_1$-$C_4$-Alkyl darstellt und
n eine ganze Zahl aus dem Bereich von 1 bis 6 bedeutet, wobei

wenn n = 1 ist,

$R_{17}$ Wasserstoff, $C_1$-$C_{18}$-Alkyl, $C_5$-$C_7$-Cycloalkyl, $C_2$-$C_{18}$-Alkenyl, einen einwertigen Rest einer Hexose, einen einwertigen Rest eines Hexitols,

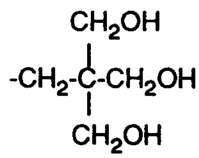 oder 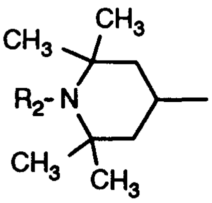 ,

wobei $R_2$ die obige Bedeutung hat, oder ferner $R_{17}$

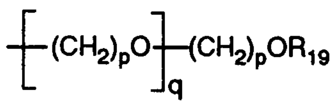

bedeutet, wobei

$R_{19}$ Wasserstoff, $C_1$-$C_{18}$-Alkyl oder $C_5$-$C_7$-Cycloalkyl bedeutet, wobei

p eine ganze Zahl aus dem Bereich von 2 bis 4 darstellt,

q eine ganze Zahl aus dem Bereich von 2 bis 10 bedeutet, oder

wenn n = 2 ist,

$R_{17}$ einen zweiwertigen Rest einer Hexose, einen zweiwertigen Rest eines Hexitols,

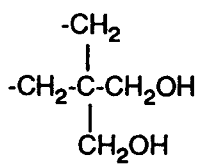 , $-C_rH_{2r}-$, 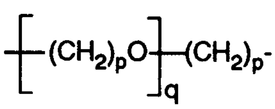

wobei p und q die obige Bedeutung haben, $-CH_2CH_2-T_4-CH_2CH_2-$ oder

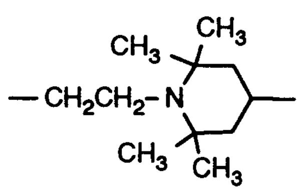

darstellt, wobei

r eine ganze Zahl aus dem Bereich von 2 bis 10 darstellt,

$T_4$ Schwefel oder >N-$R_{26}$ bedeutet und

$R_{26}$ Wasserstoff, $C_1$-$C_{10}$-Alkyl oder $C_5$-$C_8$-Cycloalkyl bedeutet, oder

wenn n = 3 ist,

$R_{17}$ einen dreiwertigen Rest einer Hexose, einen dreiwertigen Rest eines Hexitols,

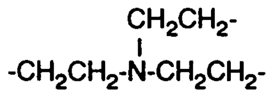 oder 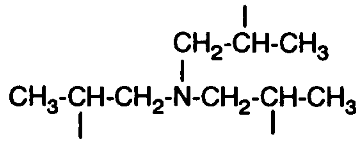

darstellt, oder

wenn n = 4 ist,

$R_{17}$ einen vierwertigen Rest einer Hexose, einen vierwertigen Rest eines Hexitols,

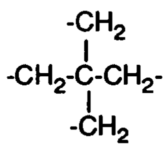 oder 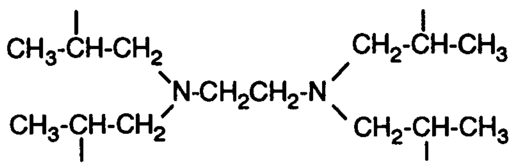

darstellt.

**5.** Produkt gemäss Anspruch 1, wobei in der Verbindung der Formel I die Reste

Y unabhängig voneinander Hydroxy oder $-NH_2$ bedeutet und
a eine ganze Zahl aus dem Bereich von 1 bis 4 darstellt, wobei

wenn a = 1 ist,

X =

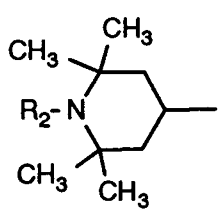

bedeutet, und

$R_2$ Wasserstoff, Methyl oder $HOCH_2CH_2$- darstellt, oder

wenn Y = OH und a = 2 ist,

X $-CH_2CH_2T_2(CH_2CH_2O)_bCH_2CH_2-$, $—C_cH_{2c}—$ oder

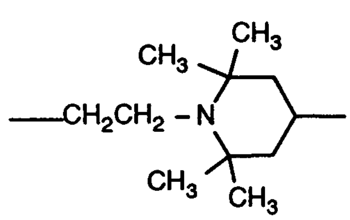

bedeutet, wobei

| | |
|---|---|
| $T_2$ | Sauerstoff, Schwefel oder >N-$R_5$, |
| $R_5$ | Wasserstoff, |
| b | die Zahl 0 oder 1 und |
| c | die Zahl 2, 3 oder 4 darstellen, oder |

wenn Y = OH und a = 3 ist,

X

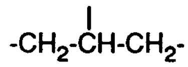

bedeutet, oder

wenn Y = OH und a = 4 ist,

X

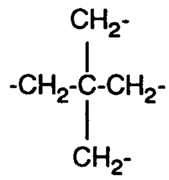

darstellt, und

in der Verbindung der Formel II die Reste

Z Wasserstoff oder eine Gruppe der Formel

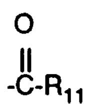

bedeuten,

| | |
|---|---|
| k | die Zahl 1 darstellt, und |
| $R_{11}$ | $C_1$-$C_{20}$-Alkyl oder $C_8$-$C_{20}$-Alkenyl bedeutet, mit der Bedingung, dass die Verbindung der Formel II eine Gruppe |

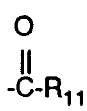

enthält, und

in der Verbindung der Formel III

| | |
|---|---|
| $R_{12}$ | tert-Butyl bedeutet, |
| $R_{15}$ | $C_1$-$C_4$-Alkyl darstellt und in ortho Stellung zur OH-Gruppe gebunden ist, |
| s | die Zahl 1 bedeutet, |
| Q | $-C_mH_{2m}-$ darstellt und para zur OH-Gruppe gebunden ist, wobei |
| m | die Zahl 2 bedeutet, |

n 1 darstellt, und

$R_{17}$ $C_1$-$C_4$-Alkyl bedeutet.

6. Produkt gemäss Anspruch 1, wobei in der Verbindung der Formel III

$R_{12}$ $C_1$-$C_4$-Alkyl oder Cyclohexyl bedeutet,

$R_{15}$ $C_1$-$C_4$-Alkyl oder Cyclohexyl darstellt und in ortho Stellung zur OH-Gruppe gebunden ist,

s die Zahl 1 bedeutet,

Q $-C_mH_{2m}-$ darstellt und para zur OH-Gruppe gebunden ist, wobei

m eine ganze Zahl aus dem Bereich von 0 bis 3, und

n eine ganze Zahl aus dem Bereich von 1 bis 4 bedeuten, wobei

wenn n = 1 ist,

$R_{17}$ Wasserstoff, $C_1$-$C_{10}$-Alkyl, Cyclohexyl, $C_2$-$C_{18}$-Alkenyl oder

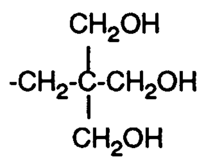

darstellt, oder

wenn n = 2 ist,

$R_{17}$

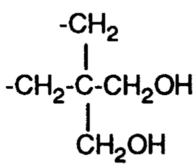, $-C_rH_{2r}-$, $-[(CH_2)_pO]_q-(CH_2)_p-$

oder $-CH_2CH_2-T_4-CH_2CH_2-$bedeutet, wobei

p eine ganze Zahl aus dem Bereich von 2 bis 4 darstellt,

q eine ganze Zahl aus dem bereich von 2 bis 10 bedeutet,

r eine ganze Zahl aus dem Bereich von 2 bis 6 darstellt,

$T_4$ Schwefel oder $>N-R_{26}$ bedeutet und

$R_{26}$ Wasserstoff oder $C_1$-$C_4$-Alkyl bedeutet, oder

wenn n = 3 ist,

$R_{17}$

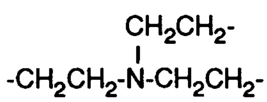 oder 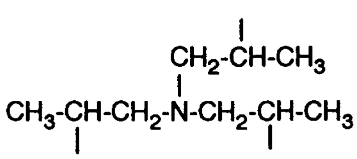

darstellt, oder

wenn n = 4 ist,

$R_{17}$

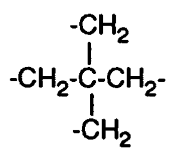 oder 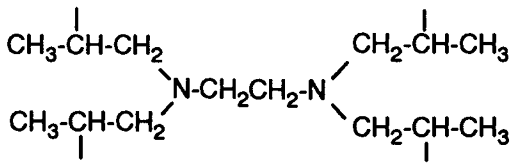

darstellt.

7. Produkt gemäss Anspruch 1,
wobei in der Verbindung der Formel III

| | |
|---|---|
| $R_{12}$ | tert-Butyl bedeutet, |
| $R_{15}$ | $C_1$-$C_4$-Alkyl darstellt und in ortho Stellung zur OH-Gruppe gebunden ist, |
| s | die Zahl 1 bedeutet, |
| Q | $-C_mH_{2m}-$ darstellt und para zur OH-Gruppe gebunden ist, wobei |
| m | die Zahl 2 bedeutet und |
| n | eine ganze Zahl 1,2 oder 4 bedeutet, wobei |

wenn n = 1 ist,

$R_{17}$ $C_1$-$C_4$-Alkyl bedeutet, oder

wenn n = 2 ist,

$R_{17}$

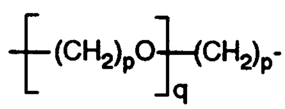

oder $-CH_2CH_2-T_4-CH_2CH_2-$ bedeutet, wobei

| | |
|---|---|
| p | die Zahl 2, |
| q | die Zahl 2 und |
| $T_4$ | Schwefel bedeuten, oder |

wenn n = 4 ist,

$R_{17}$

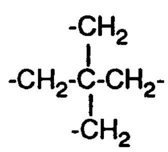

darstellt.

8. Produkt gemäss Anspruch 1, wobei die Verbindung der Formel I Pentaerythrit, Thiodiethylenglykol, 1,4-Butandiol, 1,2-Propandiol, Diethylenglykol,
Triethylenglykol, Diethanolamin, Glycerin,

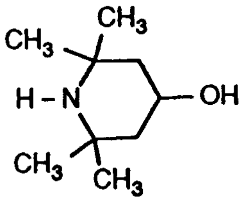 , 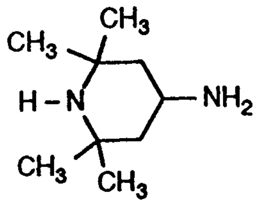 oder 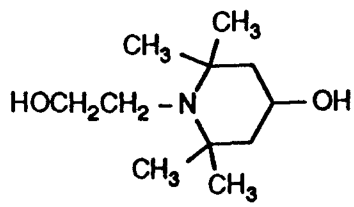

bedeutet, die Verbindung der Formel II Sonnenblumenöl, Kokosfett, Rapsöl, Maiskeimöl, Distelöl, Otivenöl, Erdnussöl oder Radiamuls darstellt, und die Verbindung der Formel III 3-(3',5'-Di-tert-butyl-4'-hydroxyphenyl)propionsäuremethylester oder 3-(3'-tert-Butyl-4'-hydroxy-5'-methylphenyl)propionsäuremethylester bedeutet.

**9.** Produkt gemäss Anspruch 1, worin das molare Mengenverhätnis der Komponenten a), b) und c) 0,1:1:0,1 bis 15:1:30 beträgt.

**10.** Produkt gemäss Anspruch 1, worin der Gewichtsanteil der Wirkgruppe E-2

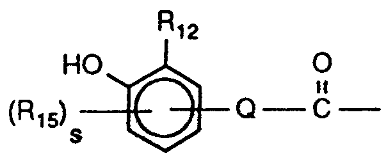 (E-2)

30 bis 80 Gew.-% beträgt.

**11.** Produkt gemäss Anspruch 1, wobei zunächst die Komponenten a) und b) miteinander umgesetzt werden und anschliessend das so erhaltene Zwischenprodukt mit der Komponente c) zur Reaktion gebracht wird.

**12.** Zusammensetzung, enthaltend

α) ein dem oxidativen, thermischen oder lichtinduzierten Abbau unterworfenes organisches Material und
β) mindestens ein Produkt gemäss Anspruch 1.

**13.** Zusammensetzung nach Anspruch 12, worin die Komponente α) ein Schmierstoff, eine Hydraulikflüssigkeit, eine Metallbearbeitungsflüssigkeit oder ein synthetisches Polymer ist.

**14.** Zusammensetzung nach Anspruch 12, worin die Komponente α) ein Schmierstoff aus der Reihe der Mineralöle, der synthetischen Oele oder einer Mischung davon ist.

**15.** Zusammensetzung nach Anspruch 12, worin die Komponente α) ein synthetisches Polymer ist.

**16.** Zusammensetzung nach Anspruch 12, worin die Komponente α) ein Polyolefin oder Styrolcopolymer ist.

**17.** Verwendung von Produkten gemäss Anspruch 1 zum Stabilisieren von Polymeren und Oelen gegen oxidativen, thermischen oder lichtinduzierten Abbau.

**18.** Verfahren zum Stabilisieren von Polymeren oder Oelen gegen oxidativen, thermischen oder lichtinduzierten Abbau, dadurch gekennzeichnet, dass man diesen ein Produkt gemäss Anspruch 1 einverleibt.

**19.** Verfahren zur Herstellung der Produkte gemäss Anspruch 1, dadurch gekennzeichnet, dass die im Anspruch 1 definierten Komponenten a), b) und c) in einem molaren Mengenverhältnis von 0,1:1:0,1 bis 15:1:30 umgesetzt werden.

## Claims

1. A product obtainable by reacting components a), b) and c), where component a) is a compound of the formula I or a mixture of compounds of the formula I, component b) is a compound of the formula II or a mixture of compounds of the formula II and component c) is a compound of the formula III or a mixture of compounds of the formula III,

$X(Y)_a$

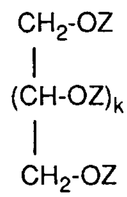

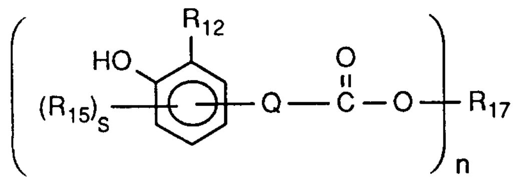

(I) (II) (III)

in which, in the compound of the formula I, the radicals
Y independently of one another are OH, $(HOCH_2CH_2)_2N$- or $-HNR_1$ and
$R_1$ is hydrogen, $C_1$-$C_{18}$alkyl, $C_5$-$C_{12}$cycloalkyl,

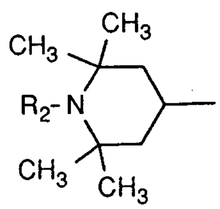,

$C_3$-$C_6$alkenyl,
$C_7$-$C_9$phenylalkyl, phenyl, or phenyl which is substituted by 1 to 3 radicals $A_1$, the radicals $A_1$ independently of one another being $C_1$-$C_{12}$alkyl, halogen, hydroxyl, methoxy or ethoxy, in which
$R_2$ is hydrogen, $C_1$-$C_8$alkyl, $O^\bullet$, OH, NO, $-CH_2CN$, $C_1$-$C_{18}$alkoxy, $C_5$-$C_{12}$cycloalkoxy, $C_3$-$C_6$alkenyl, $C_7$-$C_9$phenylalkyl or $C_7$-$C_9$phenylalkyl which is mono-, di- or trisubstituted on the phenyl ring by $C_1$-$C_4$alkyl, or $R_2$ is furthermore $C_1$-$C_8$acyl or $HOCH_2CH_2$-, and a is the number 1, 2, 3, 4 or 6, where,
if Y is OH and a is 1,
X is $C_1$-$C_{45}$alkyl, $C_3$-$C_{18}$alkenyl, $-CH_2CH_2T_1(CH_2CH_2O)_bR_4$ or

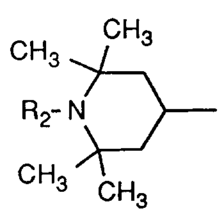,

in which $R_2$ is as defined above, and
$T_1$ is oxygen, sulfur or $>N-R_5$,
$R_4$ is $C_1$-$C_{20}$alkyl,
b is an integer ranging from 0 to 10 and
$R_5$ is hydrogen, $C_1$-$C_{18}$alkyl or phenyl, or,
if Y is OH and a is 2,
X is $-CH_2CH_2T_2(CH_2CH_2O)_bCH_2CH_2$-, in which b is as defined above,

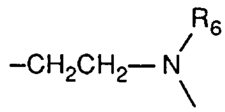 , $—C_cH_{2c}—$ , 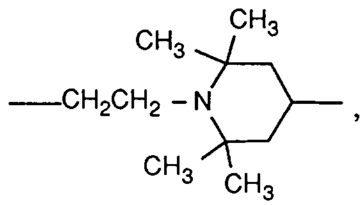 ,

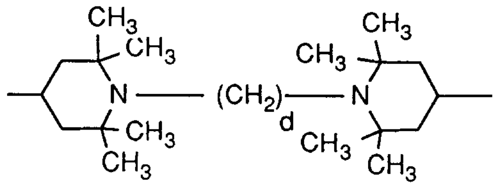 , $—CH_2\text{-}CH\text{=}CH\text{-}CH_2—$ ,

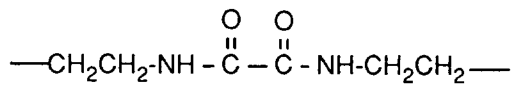 or

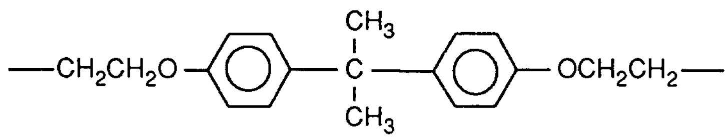 ,

in which
$T_2$ is oxygen, sulfur, $>N\text{-}R_5$ or

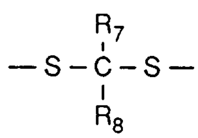

and $R_5$ is as defined above,
$R_6$ is hydrogen, $C_1$-$C_{18}$alkyl or phenyl,
c is an integer ranging from 2 to 10,
d is an integer ranging from 2 to 6 and
$R_7$ and $R_8$ independently of one another are hydrogen, $C_1$-$C_{18}$alkyl or phenyl, or $R_7$ and $R_8$ together with the C atom to which they are bonded form a $C_5$-$C_{12}$cycloalkyl ring, or,
if a is 3,
X is $C_3$-$C_{10}$alkanetriyl or $N(CH_2CH_2\text{-})_3$, or,
if Y is OH and a is 4,
X is $C_4$-$C_{10}$alkanetetrayl,

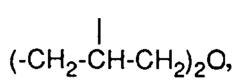

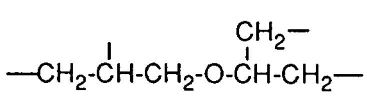 , 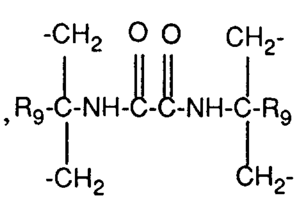 , 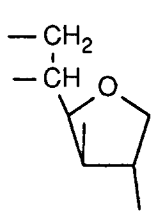 or 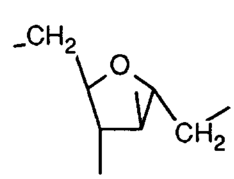 ,

in which
$R_9$ is $C_1$-$C_4$alkyl, or,

if Y is OH and a is 6,
X is

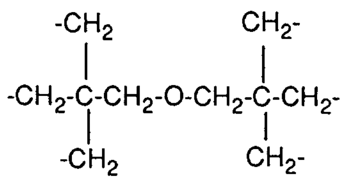
,
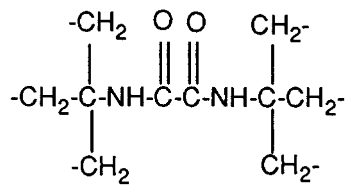

or $C_6$-$C_{10}$alkanehexayl, or,
if Y is -$HNR_1$ and a is 1,
X is $C_1$-$C_{18}$alkyl, $C_3$-$C_{18}$alkenyl, $C_5$-$C_{12}$cycloalkyl, $C_7$-$C_9$phenylalkyl, phenyl,

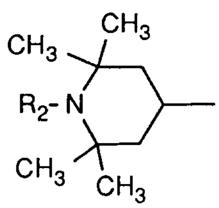
,

in which $R_2$ is as defined above or X is furthermore

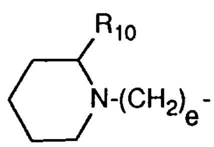
,

or X together with $R_1$ is a group of the formula $-CH_2CH_2CH_2CH_2CH_2-$ or $-CH_2CH_2OCH_2CH_2-$, in which
$R_{10}$ is hydrogen or methyl and
e is 2 or 3, or,
if Y is -$HNR_1$ and a is 2,
X is $-C_fH_{2f}-$,

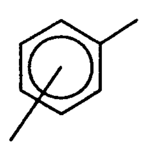
or
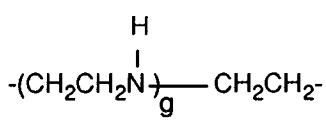
,

in which
f is an integer ranging from 2 to 10 and
g is an integer ranging from 1 to 6, and,
in the compound of the formula II, the radicals
Z are hydrogen or a group of the formula

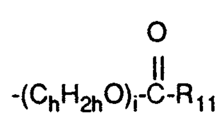

and
k is an integer ranging from 0 to 6, in which
h is 2 or 3,
i is an integer ranging from 0 to 12 and
$R_{11}$ is $C_1$-$C_{30}$alkyl, $C_8$-$C_{30}$alkenyl, $C_5$-$C_{12}$cycloalkyl, phenyl or $C_7$-$C_9$phenylalkyl, with the proviso that the compound of the formula II has a group

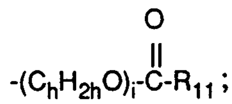 ;

in the compound of the formula III,
$R_{12}$ is $C_1$-$C_{18}$alkyl, $C_5$-$C_{12}$cycloalkyl, phenyl or $C_7$-$C_9$phenylalkyl,
$R_{15}$ is hydrogen, $C_1$-$C_{18}$alkyl, $C_5$-$C_{12}$cycloalkyl, phenyl or $C_7$-$C_9$phenylalkyl,
s is 0, 1 or 2,
Q is $-C_mH_{2m}-$,

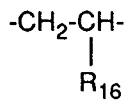 or 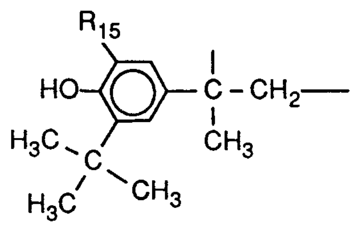 ,

in which $R_{15}$ is as defined above,
m is an integer ranging from 0 to 3,
$R_{16}$ is $C_1$-$C_8$alkyl and
n is an integer ranging from 1 to 6, where,
if n is 1,
$R_{17}$ is hydrogen, $C_1$-$C_{45}$alkyl, $C_5$-$C_{12}$cycloalkyl, $C_2$-$C_{18}$alkenyl, a monovalent radical of a hexose, a monovalent radical of a hexitol,

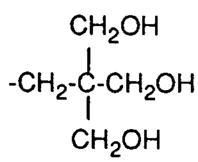 , 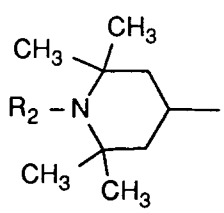 ,

in which
$R_2$ is as defined above, or furthermore $R_{17}$ is $-CH_2CH_2-T_3-R_{19}$ or

$$-\left[(CH_2)_pO\right]_q-(CH_2)_pOR_{19},$$

in which
$T_3$ is oxygen, sulfur or >N-$R_{22}$ ,
$R_{19}$ is

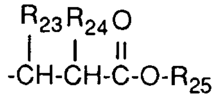 or 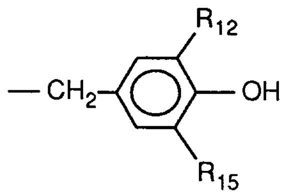 ,

in which $R_{12}$ and $R_{15}$ are as defined above, or $R_{19}$ is furthermore hydrogen, $C_1$-$C_{24}$alkyl, phenyl, $C_5$-$C_{12}$cycloalkyl or

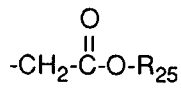 ,

in which
p is an integer ranging from 2 to 4,
q is an integer ranging from 2 to 20,
$R_{22}$ is $C_1$-$C_{18}$alkyl, phenyl or phenyl which is substituted by 1 to 3 radicals $A_1$, in which the radicals $A_1$ independently of one another are $C_1$-$C_{12}$alkyl, halogen, hydroxyl, methoxy or ethoxy, or $R_{22}$ is furthermore $C_5$-$C_8$cycloalkyl,
$R_{23}$ and $R_{24}$ independently of one another are hydrogen or methyl, with the proviso that
$R_{23}$ and $R_{24}$ are not simultaneously methyl;
$R_{25}$ is hydrogen or $C_1$-$C_{24}$alkyl, or,
if n is 2,
$R_{17}$ is a divalent radical of a hexose, a divalent radical of a hexitol,

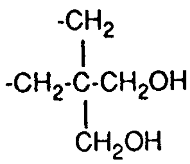 , 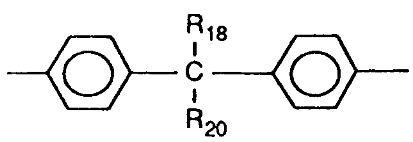 ,

$-C_rH_{2r}-$, $-[(CH_2)_pO]_q-(CH_2)_p-$ ,

in which p and q are as defined above,
$-CH_2CH_2-T_4-CH_2CH_2-$, $-CH_2-CH=CH-CH_2-$, $-CH_2-C\equiv C-CH_2-$,

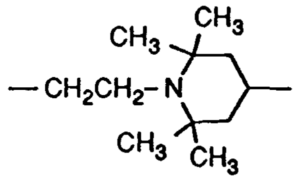,

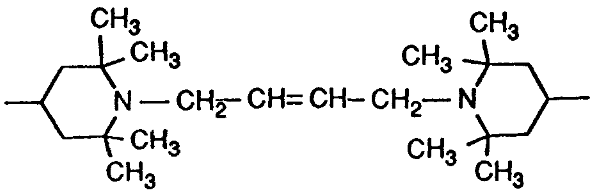, 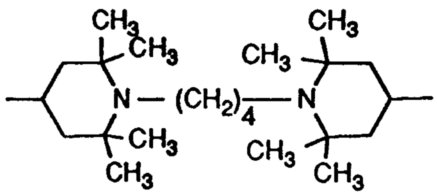,

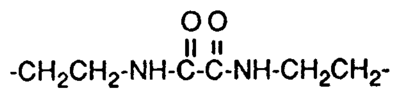 or 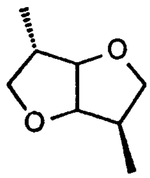,

in which

$R_{18}$ and $R_{20}$ independently of one another are hydrogen or $C_1$-$C_{12}$alkyl or together are the radical -$CH_2CH_2CH_2CH_2CH_2$-,

r is an integer ranging from 2 to 10,

$T_4$ is sulfur, >N-$R_{26}$ or

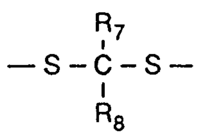,

in which $R_7$ and $R_8$ are as defined above, and

$R_{26}$ is hydrogen, $C_1$-$C_{18}$alkyl, phenyl or phenyl which is substituted by 1 to 3 radicals $A_1$, in which the radicals $A_1$ are as defined above in formula I, or $R_{26}$ is furthermore $C_5$-$C_8$cycloalkyl or

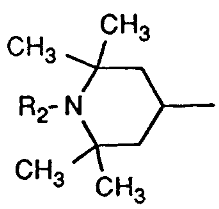,

in which $R_2$ is as defined above, or,

if n is 3,

$R_{17}$ is a trivalent radical of a hexose, a trivalent radical of a hexitol,

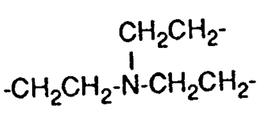, 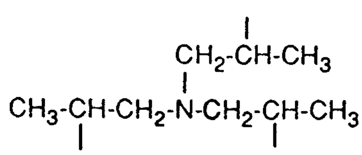 or 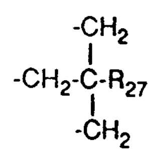,

in which

$R_{27}$ is hydrogen, $-CH_2OH$, $C_1$-$C_4$alkyl, $C_1$-$C_{18}$alkylamido or

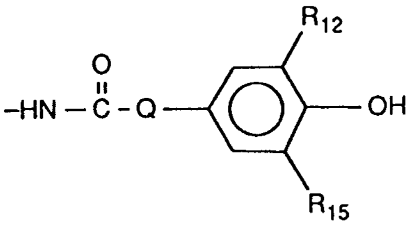 ,

in which Q, $R_{12}$ and $R_{15}$ are as defined above, or,
if n is 4,
$R_{17}$ is a tetravalent radical of a hexose, a tetravalent radical of a hexitol, $C_4$-$C_{10}$alkanetetrayl,

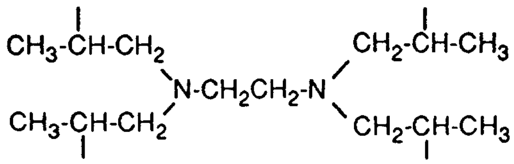 , 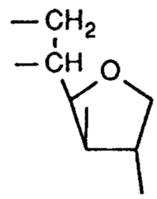 or 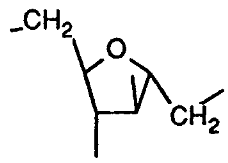 ,

or, if n is 5,
$R_{17}$ is a pentavalent radical of a hexose or a pentavalent radical of a hexitol, or,
if n is 6,
$R_{17}$ is a hexavalent radical of a hexitol or

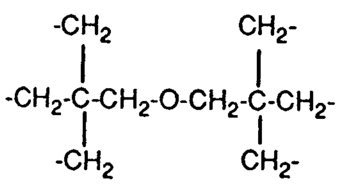 .

2. A product according to claim 1, in which, in the compound of the formula III, s is the number 1 or 2.

3. A product according to claim 1,
in which, in the compound of the formula I, the radicals
Y independently of one another are OH, $(HOCH_2CH_2)_2N$- or $-HNR_1$ and
$R_1$ is hydrogen, $C_1$-$C_{10}$alkyl, $C_5$-$C_7$cycloalkyl,

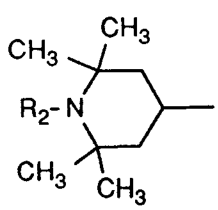 ,

$C_3$-$C_6$alkenyl, benzyl or phenyl, in which
$R_2$ is hydrogen, $C_1$-$C_4$alkyl, OH, $-CH_2CN$, $C_6$-$C_{12}$alkoxy, $C_5$-$C_8$cycloalkoxy, allyl, benzyl, acetyl or $HOCH_2CH_2$- and
a is the number 1, 2, 3, 4 or 6, where,
if Y is OH and a is 1,
X is $C_1$-$C_{30}$alkyl, $C_3$-$C_{18}$alkenyl, $-CH_2CH_2T_1(CH_2CH_2O)_bR_4$ or

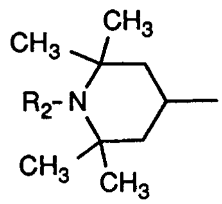 ,

in which $R_2$ is as defined above, and
$T_1$ is oxygen, sulfur or $>N-R_5$,
$R_4$ is $C_1$-$C_{10}$alkyl,
b is an integer ranging from 0 to 10 and
$R_5$ is hydrogen, $C_1$-$C_{10}$alkyl or phenyl, or,
if Y is OH and a is 2,
X is $-CH_2CH_2T_2(CH_2CH_2O)_bCH_2CH_2-$, in which b is as defined above,

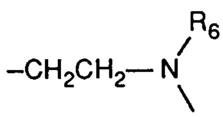 , $-C_cH_{2c}-$ , 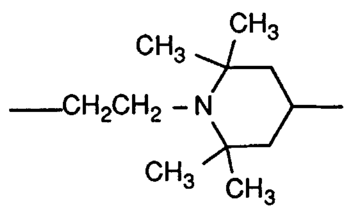 ,

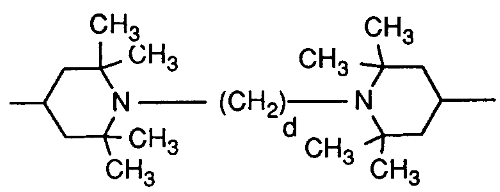 , $-CH_2-CH=CH-CH_2-$ ,

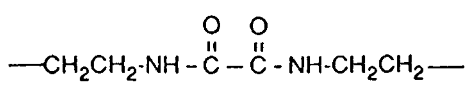 or 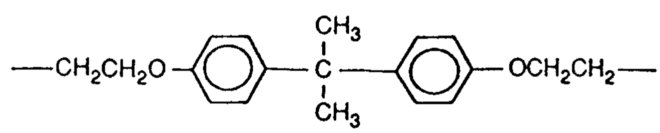 ,

in which
$T_2$ is oxygen, sulfur, $>N-R_5$ or

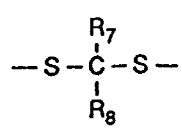

and $R_5$ is as defined above,
$R_6$ is hydrogen, $C_1$-$C_{10}$alkyl or phenyl,
c is an integer ranging from 2 to 10,
d is an integer ranging from 2 to 6 and
$R_7$ and $R_8$ independently of one another are hydrogen, $C_1$-$C_{10}$alkyl or phenyl, or $R_7$ and $R_8$ together with the C atom to which they are bonded form a $C_5$-$C_7$cycloalkyl ring, or,

if Y is $-HNR_1$ and a is 1,
X is $C_1$-$C_{10}$alkyl, $C_3$-$C_{18}$alkenyl, $C_5$-$C_7$cycloalkyl, benzyl, phenyl,

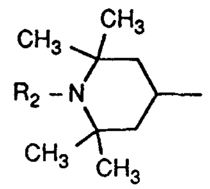 ,

in which $R_2$ is as defined above, or X is furthermore

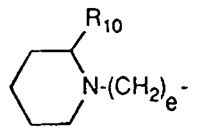 ,

or X together with $R_1$ is a group of the formula $-CH_2CH_2CH_2CH_2CH_2-$ or $-CH_2CH_2OCH_2CH_2-$, in which $R_{10}$ is hydrogen or methyl and
e is 2 or 3, and,
in the compound of the formula II, the radicals
Z are hydrogen or a group of the formula

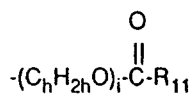

and
k is an integer ranging from 0 to 4, in which
h is 2 or 3,
i is an integer ranging from 0 to 6 and
$R_{11}$ is $C_1$-$C_{20}$alkyl, $C_8$-$C_{20}$alkenyl, $C_5$-$C_7$cycloalkyl, phenyl or benzyl, with the proviso that the compound of the formula II comprises a group

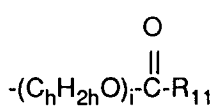 ;

in the compound of the formula III,
$R_{12}$ is $C_1$-$C_6$alkyl, $C_5$-$C_7$cycloalkyl, phenyl or benzyl
$R_{15}$ is hydrogen, $C_1$-$C_6$alkyl, $C_5$-$C_7$cycloalkyl, phenyl or benzyl,
s is 1 or 2,
Q is $-C_mH_{2m}-$,

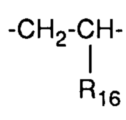 or 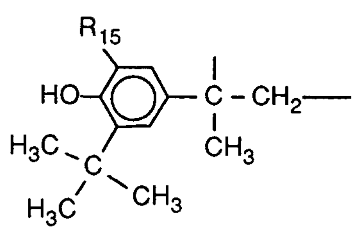 ,

in which $R_{15}$ is as defined above,
m is an integer ranging from 0 to 3,
$R_{16}$ is $C_1$-$C_4$alkyl and
n is an integer ranging from 1 to 6, where,
if n is 1,
$R_{17}$ is hydrogen, $C_1$-$C_{30}$alkyl, $C_5$-$C_7$cycloalkyl, $C_2$-$C_{18}$alkenyl, a monovalent radical of a hexose, a monovalent radical of a hexitol,

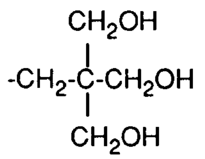 , 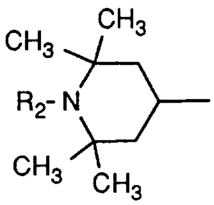 ,

in which $R_2$ is as defined above, or furthermore $R_{17}$ is $-CH_2CH_2-T_3-R_{19}$ or

$$-\left[(CH_2)_pO\right]_q-(CH_2)_pOR_{19},$$

in which
$T_3$ is oxygen, sulfur or >N-$R_{22}$ ,
$R_{19}$ is

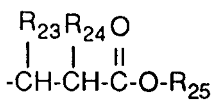 , 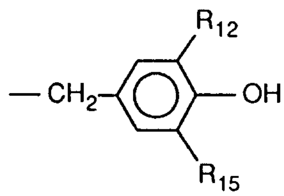 ,

in which $R_{12}$ and $R_{15}$ are as defined above, or $R_{19}$ is furthermore hydrogen, $C_1$-$C_{18}$alkyl, phenyl, $C_5$-$C_7$cycloalkyl or

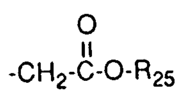 ,

in which
p is an integer ranging from 2 to 4,
q is an integer ranging from 2 to 20,
$R_{22}$ is $C_1$-$C_{10}$alkyl, phenyl or $C_5$-$C_8$cycloalkyl,
$R_{23}$ and $R_{24}$ independently of one another are hydrogen or methyl with the proviso that $R_{23}$ and $R_{24}$ are not simultaneously methyl;
$R_{25}$ is hydrogen or $C_1$-$C_{18}$alkyl, or,
if n is 2,
$R_{17}$ is a divalent radical of a hexose, a divalent radical of a hexitol,

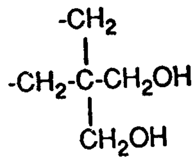, 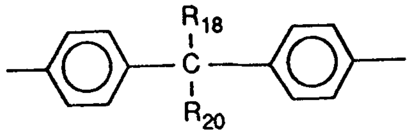,

$-C_rH_{2r}-$, $-\!\left[(CH_2)_pO\right]_q\!-(CH_2)_p-$,

in which p and q are as defined above, $-CH_2CH_2-T_4-CH_2CH_2-$, $-CH_2-CH=CH-CH_2-$, $-CH_2-C\equiv C-CH_2-$,

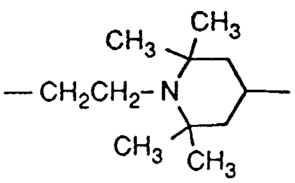,

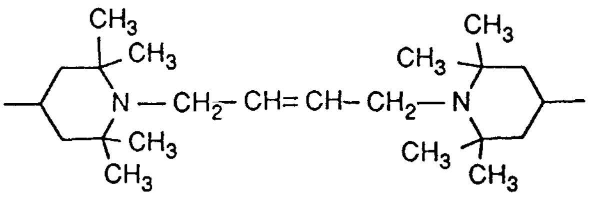, 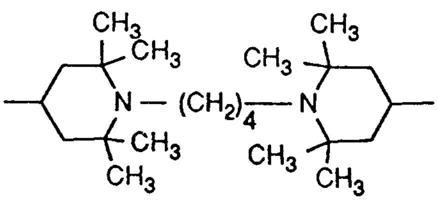,

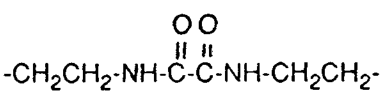 or 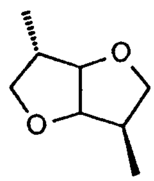,

in which

$R_{18}$ and $R_{20}$ independently of one another are hydrogen or $C_1$-$C_6$alkyl or together are the radical $-CH_2CH_2CH_2CH_2CH_2-$,

r is an integer ranging from 2 to 10,

$T_4$ is sulfur, >N-$R_{26}$ or

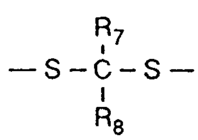,

in which $R_7$ and $R_8$ are as defined above and

$R_{26}$ is hydrogen, $C_1$-$C_{10}$alkyl, phenyl, $C_5$-$C_8$cycloalkyl or

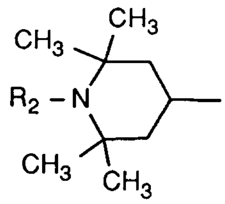 ,

in which $R_2$ is as defined above.

4. A product according to claim 1,
in which, in the compound of the formula I, the radicals
Y independently of one another are OH, $(HOCH_2CH_2)_2N$- or $-HNR_1$ and
$R_1$ is hydrogen, $C_1$-$C_4$alkyl or

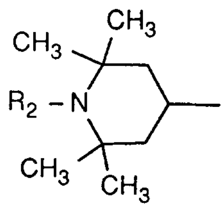 ,

in which
$R_2$ is hydrogen, $C_1$-$C_4$alkyl, OH, allyl, benzyl, acetyl or $HOCH_2CH_2$- and a is the number 1, 2, 3, 4 or 6, where,
if Y is OH and a is 1,
X is $C_1$-$C_{18}$alkyl, $C_3$-$C_{18}$alkenyl, $-CH_2CH_2T_1(CH_2CH_2O)_bR_4$ or

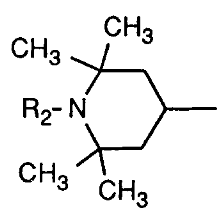 ,

in which $R_2$ is as defined above, and
$T_1$ is oxygen,
$R_4$ is $C_1$-$C_4$alkyl and
b is an integer ranging from 0 to 10, or,
if Y is OH and a is 2,
X is $-CH_2CH_2T_2(CH_2CH_2O)_bCH_2CH_2$-, in which b is as defined above, or furthermore X is $-C_cH_{2c}-$,

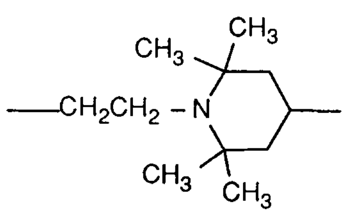

or $-CH_2$-$CH$=$CH$-$CH_2-$, in which
$T_2$ is oxygen, sulfur or $>N$-$R_5$,
$R_5$ is hydrogen,

b is the number 0 or 1 and
c is an integer ranging from 2 to 8, or,
if a is 3,
X is

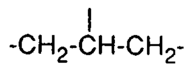

or $N(CH_2CH_2-)_3$, or,
if Y is OH and a is 4,
X is

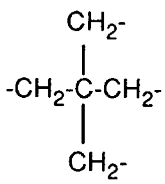 , 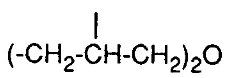 or 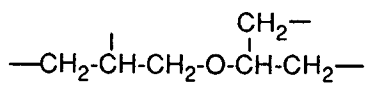 ,

or,
if Y is OH and a is 6,
X is

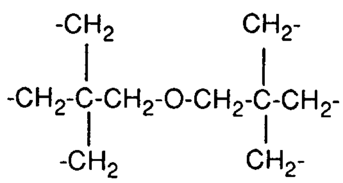 or 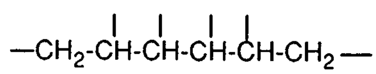 ,

or,
if Y is $-HNR_1$ and a is 1,
X is $C_1$-$C_{10}$alkyl, $C_3$-$C_{18}$alkenyl, $C_5$-$C_7$cycloalkyl or

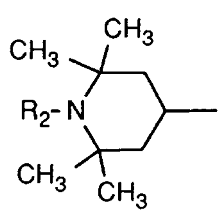 ,

where $R_2$ is as defined above, or,
if Y is $-HNR_1$ and a is 2,
X is $-C_fH_{2f}-$ in which
f is an integer ranging from 2 to 10 and,
in the compound of the formula II, the radicals
Z are hydrogen or a group of the formula

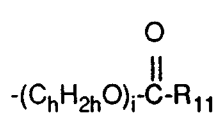

and

k is 1, 2 or 3,

h is 2 or 3,

i is an integer ranging from 0 to 4 and

$R_{11}$ is $C_1$-$C_{20}$alkyl or $C_8$-$C_{20}$alkenyl, with the proviso that the compound of the formula II comprises a group

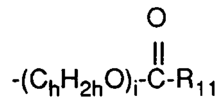

;

in the compound of the formula III,

$R_{12}$ is $C_1$-$C_6$alkyl or $C_5$-$C_7$cycloalkyl,

$R_{15}$ is hydrogen, $C_1$-$C_6$alkyl or $C_5$-$C_7$cycloalkyl,

s is 1 or 2,

Q is $-C_mH_{2m}-$ or

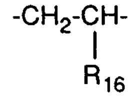

,

m is an integer ranging from 0 to 3,

$R_{16}$ is $C_1$-$C_4$alkyl and

n is an integer ranging from 1 to 6, where,

if n is 1,

$R_{17}$ is hydrogen, $C_1$-$C_{18}$alkyl, $C_5$-$C_7$cycloalkyl, $C_2$-$C_{18}$alkenyl, a monovalent radical of a hexose, a monovalent radical of a hexitol,

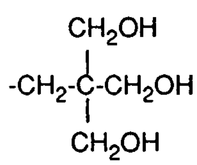 or 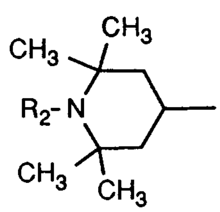 ,

in which $R_2$ is as defined above, or furthermore $R_{17}$ is

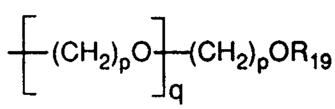

in which

$R_{19}$ is hydrogen, $C_1$-$C_{18}$alkyl or $C_5$-$C_7$cycloalkyl, in which

p is an integer ranging from 2 to 4,

q is an integer ranging from 2 to 10, or,

if n is 2,

$R_{17}$ is a divalent radical of a hexose, a divalent radical of a hexitol,

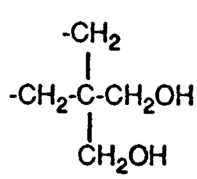

,

$-C_rH_{2r}-$, $-\left[(CH_2)_pO\right]_q-(CH_2)_p-$ ,

in which p and q are as defined above,
$-CH_2CH_2-T_4-CH_2CH_2-$, or

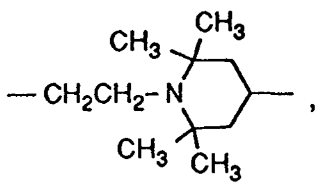 ,

in which
r is an integer ranging from 2 to 10,
$T_4$ is sulfur or $>N-R_{26}$ and
$R_{26}$ is hydrogen, $C_1$-$C_{10}$alkyl or $C_5$-$C_8$cycloalkyl, or,
if n is 3,
$R_{17}$ is a trivalent radical of a hexose, a trivalent radical of a hexitol,

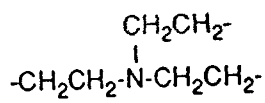 or 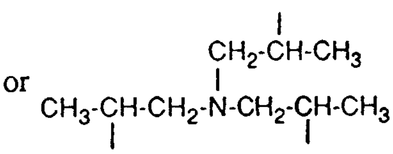 ,

or,
if n is 4,
$R_{17}$ is a tetravalent radical of a hexose, a tetravalent radical of a hexitol,

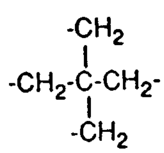 or 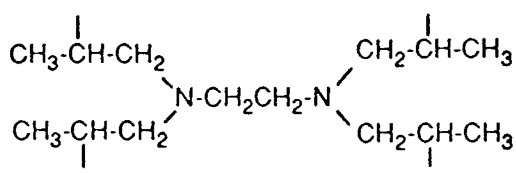 .

**5.** A product according to claim 1,
in which, in the compound of the formula I, the radicals
Y independently of one another are hydroxyl or $-NH_2$ and
a is an integer ranging from 1 to 4, where
if a is 1,
X is

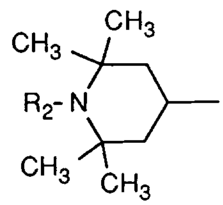

and

$R_2$ is hydrogen, methyl or $HOCH_2CH_2$-, or,

if Y is OH and a is 2,

X is $-CH_2CH_2T_2(CH_2CH_2O)_bCH_2CH_2-$, $—C_cH_{2c}—$ or

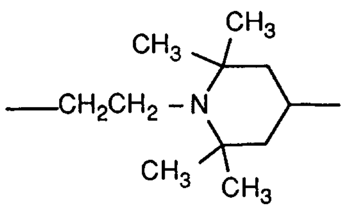,

in which

$T_2$ is oxygen, sulfur or >N-$R_5$,

$R_5$ is hydrogen,

b is the number 0 or 1 and

c is the number 2, 3 or 4, or,

if Y is OH and a is 3,

X is

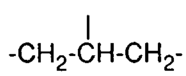 ,

or,

if Y is OH and a is 4,

X is

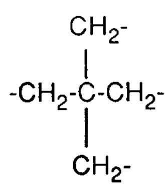

and,

in the compound of the formula II, the radicals

Z are hydrogen or a group of the formula

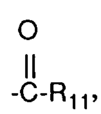

k is the number 1 and

$R_{11}$ is $C_1$-$C_{20}$alkyl or $C_8$-$C_{20}$alkenyl, with the proviso that the compound of the formula II comprises a group

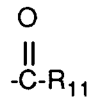,

and,
in the compound of the formula III,
$R_{12}$ is tert-butyl,
$R_{15}$ is $C_1$-$C_4$alkyl and is bonded in the ortho-position relative to the OH group,
s is the number 1,
Q is $-C_mH_{2m}-$ and is bonded in the para-position relative to the OH group, where,
m is the number 2,
n is 1 and
$R_{17}$ is $C_1$-$C_4$alkyl.

**6.** A product according to claim 1,
in which, in the compound of the formula III,
$R_{12}$ is $C_1$-$C_4$alkyl or cyclohexyl,
$R_{15}$ is $C_1$-$C_4$alkyl or cyclohexyl and is bonded in the ortho-position relative to the OH group,
s is the number 1,
Q is $-C_mH_{2m}-$ and is bonded in the para-position relative to the OH group, where
m is an integer ranging from 0 to 3 and
n is an integer ranging from 1 to 4, where,
if n is 1,
$R_{17}$ is hydrogen, $C_1$-$C_{10}$alkyl, cyclohexyl, $C_2$-$C_{18}$alkenyl or

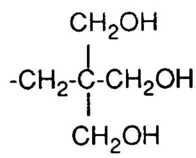 ,

or,
if n is 2,
$R_{17}$ is

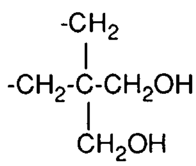 , $-C_rH_{2r}-$, 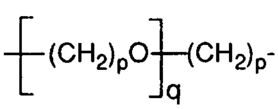

or $-CH_2CH_2-T_4-CH_2CH_2-$ in which
p is an integer ranging from 2 to 4,
q is an integer ranging from 2 to 10,
r is an integer ranging from 2 to 6,
$T_4$ is sulfur or $>N-R_{26}$ and
$R_{26}$ is hydrogen or $C_1$-$C_4$alkyl, or,
if n is 3,
$R_{17}$ is

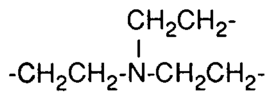 or 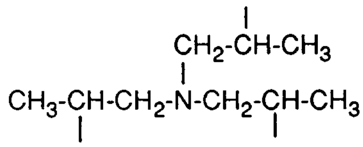 ,

or,
if n is 4,
$R_{17}$ is

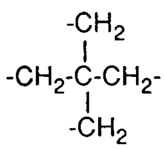 or 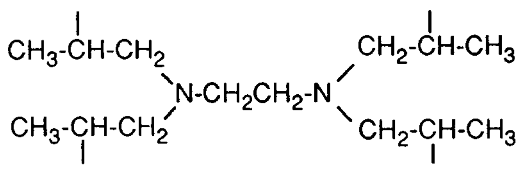 .

**7.** A product according to claim 1,
in which, in the compound of the formula III,
$R_{12}$ is tert-butyl,
$R_{15}$ is $C_1$-$C_4$alkyl and is bonded in the ortho-position relative to the OH group,
s is the number 1,
Q is $-C_mH_{2m}-$ and is bonded in the para-position relative to the OH group, in which
n is the number 2 and
n is an integer 1, 2 or 4, where,
if n is 1,
$R_{17}$ is $C_1$-$C_4$alkyl, or,
if n is 2,
$R_{17}$ is

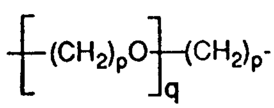

or $-CH_2CH_2-T_4-CH_2CH_2-$, in which
p is the number 2,
q is the number 2 and
$T_4$ is sulfur, or,
if n is 4,
$R_{17}$ is

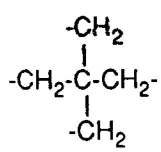 .

8. A product according to claim 1, in which the compound of the formula I is pentaerythritol, thiodiethylene glycol, 1,4-butanediol, 1,2-propanediol, diethylene glycol,
triethylene glycol, diethanolamine, glycerol,

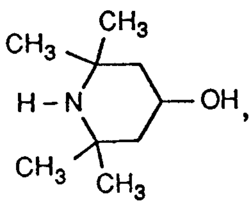, 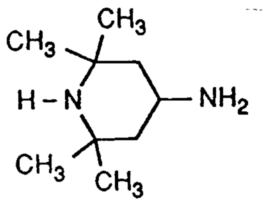 or 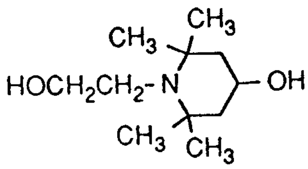,

the compound of the formula II is sunflower oil, coconut fat,
rapeseed oil, maize germ oil, safflower oil, olive oil, groundnut oil or Radiamuls, and the compound of the formula III is methyl 3-(3',5'-di-tert-butyl-4'-hydroxyphenyl)propionate or methyl 3-(3'-tert-butyl-4'-hydroxy-5'-methylphenyl)propionate.

9. A product according to claim 1, in which the molar quantitative ratio of components a), b) and c) is 0.1:1:0.1 to 15:1:30.

10. A product according to claim 1, in which the amount by weight of active group E-2

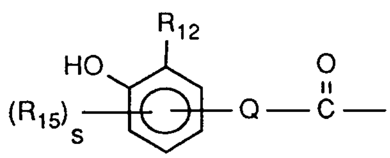 (E-2)

is 30 to 80 % by weight.

11. A product according to claim 1, in which first components a) and b) are reacted with each other and the resulting intermediate is subsequently reacted with component c).

12. A composition comprising

α) an organic material subjected to oxidative, thermal or light-induced degradation and
β) at least one product according to claim 1.

13. A composition according to claim 12, in which component α) is a lubricant, a hydraulic fluid, a metal-working fluid or a synthetic polymer.

14. A composition according to claim 12, in which component α) is a lubricant from the series of the mineral oils, the synthetic oils or a mixture of these.

15. A composition according to claim 12, in which component α) is a synthetic polymer.

16. A composition according to claim 12, in which component α) is a polyolefin or a styrene copolymer.

17. The use of a product according to claim 1 for stabilising polymers and oils against oxidative, thermal or light-induced degradation..

18. A process for stabilising polymers or oils against oxidative, thermal or light-induced degradation, which comprises incorporating a product according to claim 1 into these polymers or oils.

19. A process for the preparation of a product according to claim 1, which comprises reacting the components a), b) and c) which have been defined in claim 1 in a molar quantitative ratio of 0.1:1:0.1 to 15:1:30.

## Revendications

1. Produit que l'on peut obtenir par réaction des composants a), b) et c), le composant a) étant un composé de formule I ou un mélange de composés de formule I, le composant b) un composé de formule II ou un mélange de composés de formule II et le composant c) un composé de formule III ou un mélange de composés de formule III

$X(Y)_a$ ,  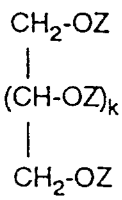 ,  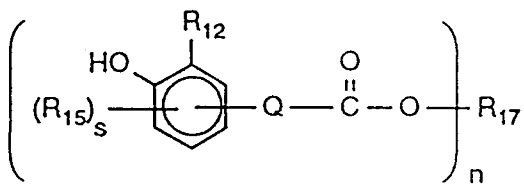

(I) (II) (III)

où dans le composé de formule I les radicaux

Y indépendamment les uns des autres, représentent OH, $(HOCH_2CH_2)_2N$- ou $-HNR_1$ et
$R_1$ représente l'hydrogène, alkyle en $C_1$-$C_{18}$, cycloalkyle en $C_5$-$C_{12}$

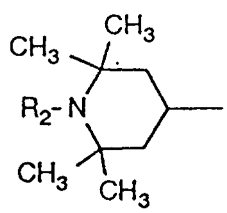 ,

alcényle en $C_3$-$C_6$, phénylalkyle en $C_7$-$C_9$, phényle ou phényle substitué par 1 à 3 radicaux $A_1$, les radicaux $A_1$, indépendamment les uns des autres, représentant alkyle en $C_1$-$C_{12}$, halogéne, hydroxy, méthoxy ou éthoxy,

$R_2$ représente l'hydrogène, alkyle en $C_1$-$C_8$, $O^{\bullet}$, OH, NO, $-CH_2CN$, alcoxy en $C_1$-$C_{18}$, cycloalcoxy en $C_5$-$C_{12}$, alcényle en $C_3$-$C_6$, phénylalkyle en $C_7$-$C_9$ ou phénylalkyle en $C_7$-$C_9$ mono-, di- ou trisubstitué par alkyle en $C_1$-$C_4$ sur le cycle de phényle, ou $R_2$ peut être de plus acyle en $C_1$-$C_8$ ou $HOCH_2CH_2$-, et

a vaut 1, 2, 3, 4 ou 6, où

lorsque Y = OH et a = 1,

X représente alkyle en $C_1$-$C_{45}$, alcényle en $C_3$-$C_{18}$, $-CH_2CH_2T_1(CH_2CH_2O)_bR_4$ ou

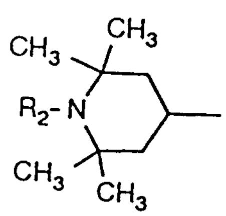

$R_2$ a les significations ci-dessus, et

$T_1$ étant l'oxygène, le soufre ou >N-$R_5$,
$R_4$ étant alkyle en $C_1$-$C_{20}$,
b étant un nombre entier de 0 à 10 et
$R_5$ étant l'hydrogène, alkyle en $C_1$-$C_{18}$ ou phényle, ou

lorsque Y = OH et a = 2,

X $-CH_2CH_2T_2(CH_2CH_2O)_bCH_2CH_2$-, b ayant la signification donnée ci-dessus

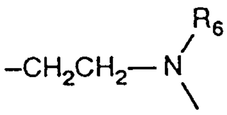 , $-C_cH_{2c}-$ , 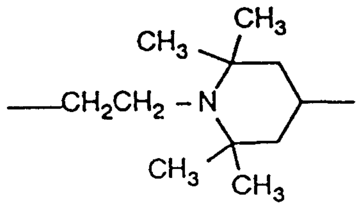 ,

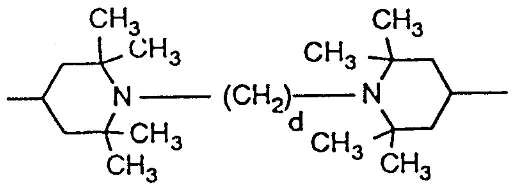 , $-CH_2\text{-}CH{=}CH\text{-}CH_2-$ ,

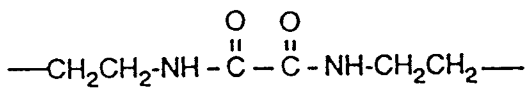 ou

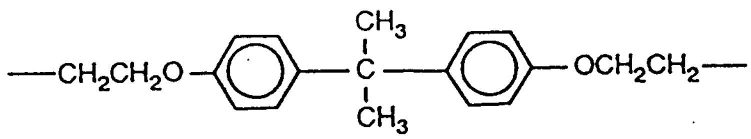

$T_2$ étant l'oxygène, le soufre ou >N-$R_5$ ou

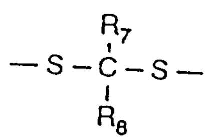

et $R_5$ ayant la signification donnée ci-dessus,

$R_6$ étant l'hydrogène, alkyle en $C_1$-$C_{18}$ ou phényle,

c étant un nombre entier de 2 à 10,

d étant un nombre entier de 2 à 6, et

$R_7$, $R_8$, indépendamment les uns des autres, représentent l'hydrogène, alkyle en $C_1$-$C_{18}$ ou phényle, ou $R_7$ et $R_8$ avec l'atome de carbone auquel ils sont liés forment un cycle cycloalkyle en $C_5$-$C_{12}$, ou

lorsque a = 3,

X représente (alcane en $C_3$-$C_{10}$)triyle ou $N(CH_2CH_2\text{-})_3$, ou

lorsque Y = OH et a = 4,

X représente (alcane en $C_4$-$C_{10}$)tétrayle ou

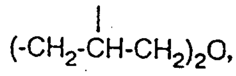

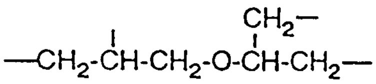, 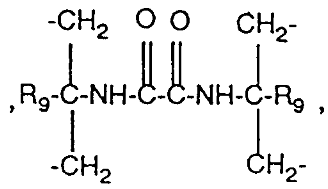, 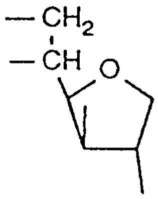 ou

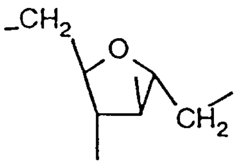

$R_9$ représente alkyle en $C_1$-$C_4$, ou

lorsque Y = OH et a = 6,

X représente

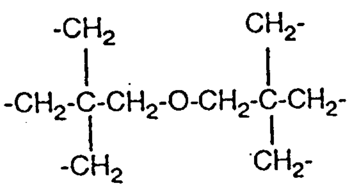, 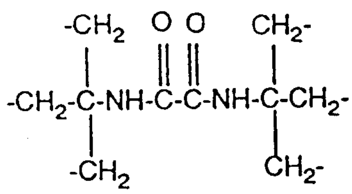

ou (alcane en $C_6$-$C_{10}$)hexayle, ou

lorsque Y = $-HNR_1$ et a = 1,

X représente alkyle en $C_1$-$C_{18}$, alcényle en $C_3$-$C_{18}$, cycloalkyle en $C_5$-$C_{12}$, phénylalkyle en $C_7$-$C_9$, phényle,

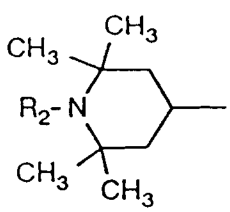

$R_2$ a la signification donnée ci-dessus, ou X est de plus

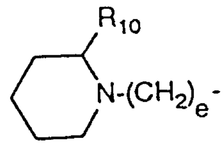

ou X avec $R_1$ forment ensemble un groupe de formule $-CH_2CH_2CH_2CH_2CH_2-$ ou $-CH_2CH_2OCH_2CH_2-$,

$R_{10}$ étant l'hydrogène ou méthyle, et

e vaut 2 ou 3, ou

lorsque $Y = -HNR_1$ et a = 2,

X représente $-C_fH_{2f}-$,

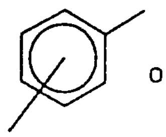 ou $-(CH_2CH_2\overset{H}{\overset{|}{N}})_g-CH_2CH_2-$

f étant un nombre entier de 2 à 10 et

g étant un nombre entier de 1 à 6, et

dans le composé de formule II les radicaux

Z représente l'hydrogène ou un groupe de formule

$$-(C_hH_{2h}O)_i-\overset{O}{\overset{\|}{C}}-R_{11}$$

et

k représente un nombre entier de 0 à 6, où

h vaut 2 ou 3,

i est un nombre entier de 0 à 12 et

$R_{11}$ représente alkyle en $C_1$-$C_{30}$, alcényle en $C_8$-$C_{30}$, cycloalkyle en $C_5$-$C_{12}$, phényle ou phénylalkyle en $C_7$-$C_9$, à la condition que le composé de formule II contienne un groupe

$$-(C_hH_{2h}O)_i-\overset{O}{\overset{\|}{C}}-R_{11} \quad ;$$

dans le composé de formule III

$R_{12}$ représente alkyle en $C_1$-$C_{18}$, cycloalkyle en $C_5$-$C_{12}$, phényle ou phénylalkyle en $C_7$-$C_9$,

$R_{15}$ représente l'hydrogène, alkyle en $C_1$-$C_{18}$, cycloalkyle en $C_5$-$C_{12}$, phényle ou phénylalkyle en $C_7$-$C_9$,

s vaut 0, 1 ou 2,

Q $-C_mH_{2m}-$,

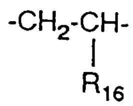 ou 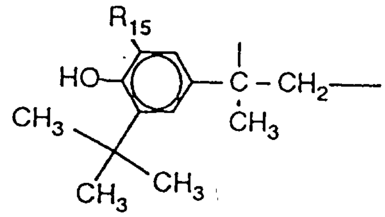

$R_{15}$ ayant la signification donnée ci-dessus,

m étant un nombre entier de 0 à 3,

$R_{16}$ étant alkyle en $C_1$-$C_8$, et

n étant un nombre entier de 1 à 6,

lorsque n = 1,

$R_{17}$ étant l'hydrogène, alkyle en $C_1$-$C_{45}$, cycloalkyle en $C_5$-$C_{12}$, alcényle en $C_2$-$C_{18}$, un radical monovalent d'un hexose, un radical monovalent d'un hexitol,

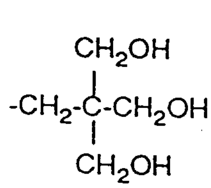 , 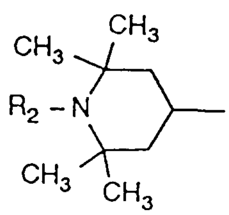

$R_2$ ayant la signification donnée ci-dessus ou de plus $R_{17}$ représente $-CH_2CH_2-T_3-R_{19}$ ou

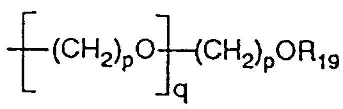

$T_3$ étant l'oxygène, le soufre ou >N-$R_{22}$

$R_{19}$ étant

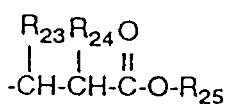 ou 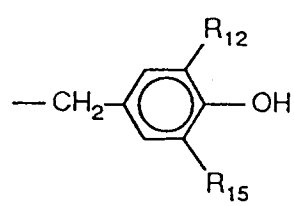

$R_{12}$ et $R_{15}$ ayantla signification donnée ci-dessus, ou $R_{19}$ étant de plus l'hydrogène, alkyle en $C_1$-$C_{24}$, phényle, cycloalkyle en $C_5$-$C_{12}$ ou

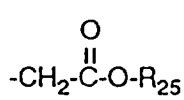

p étant un nombre entier de 2 à 4,

q étant un nombre entier de 2 à 20,

$R_{22}$ représentant alkyle en $C_1$-$C_{18}$, phényle ou phényle substitué par 1 à 3 radicaux $A_1$, les radicaux $A_1$, indépendamment les uns des autres, étant alkyle en $C_1$-$C_{12}$, halogène, hydroxy, méthoxy ou éthoxy, ou $R_{22}$ représente de plus cycloalkyle en $C_5$-$C_8$,

$R_{23}$, $R_{24}$, indépendamment l'un de l'autre, représentent l'hydrogène ou méthyle à la condition que $R_{23}$ et $R_{24}$ ne soient pas simultanément méthyle ;

$R_{25}$ représente l'hydrogène ou alkyle en $C_1$-$C_{24}$ ou

lorsque n = 2,

$R_{17}$ représente un radical bivalent d'un hexose, un radical bivalent d'un hexitol

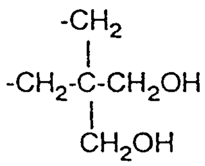, 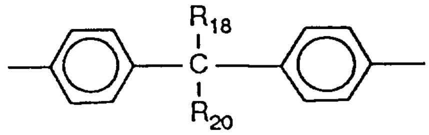, $-C_rH_{2r}-$,

$-[(CH_2)_pO]_q-(CH_2)_p-$ ,

p et q ayant la signification ci-dessus $-CH_2CH_2-T_4-CH_2CH_2-$, $-CH_2-CH=CH-CH_2-$, $-CH_2-C\equiv C-CH_2-$ ,

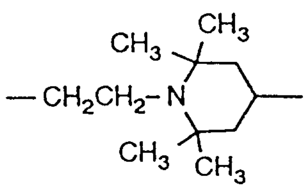 ,

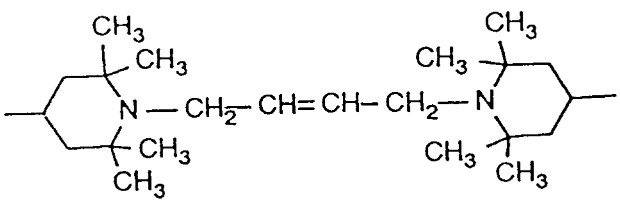 ,

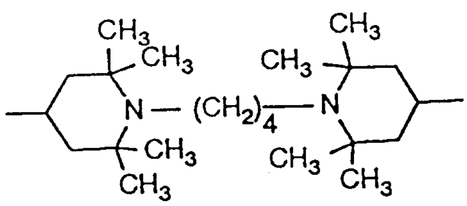 , 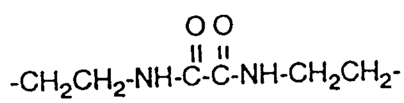 ou

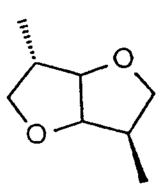

$R_{18}$, $R_{20}$, indépendamment l'un de l'autre, étant l'hydrogène ou alkyle en $C_1$-$C_{12}$ ou formant ensemble le radical $-CH_2CH_2CH_2CH_2CH_2-$,

r un nombre entier de 2 à 10,

$T_4$ étant le soufre, $>N-R_{26}$ ou

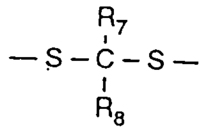

$R_7$ et $R_8$ ayant la signification donnée ci-dessus, et

$R_{26}$ étant l'hydrogène, alkyle en $C_1$-$C_{18}$, phényle ou phényle substitué par 1 à 3 radicaux $A_1$, les radicaux $A_1$ ayant la signification donnée ci-dessus pour la formule I, ou $R_{26}$ représente de plus cycloalkyle en $C_5$-$C_8$ ou

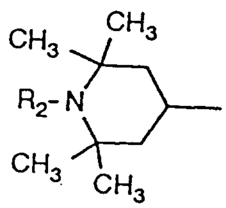

$R_2$ ayant la signification donnée ci-dessus, ou

lorsque n = 3,

$R_{17}$ est un radical trivalent d'un hexose, un radical trivalent d'un hexitol,

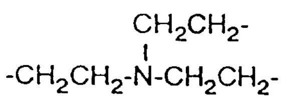 , 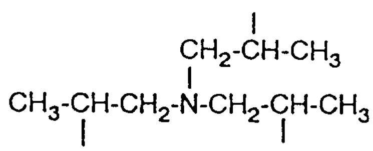 ou 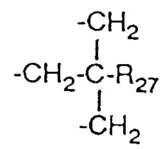

$R_{27}$ étant l'hydrogène, $-CH_2OH$, alkyle en $C_1$-$C_4$, (alkyle en $C_1$-$C_{18}$)amido ou

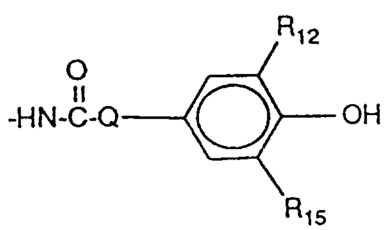

Q, $R_{12}$ et $R_{15}$ ayant la signification donnée ci-dessus, ou

lorsque n = 4,

$R_{17}$ étant un radical tétravalent d'un hexose, un radical tétravalent d'un hexitol, (alcane en $C_4$-$C_{10}$)tétrayle

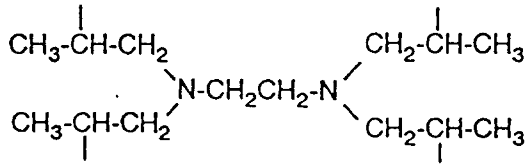,

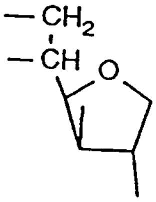 ou 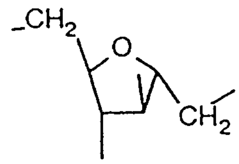

lorsque n = 5,

$R_{17}$ représente un radical pentavalent d'un hexose ou un radical pentavalent d'un hexitol, ou

lorsque n = 6,

$R_{17}$ étant un radical hexavalent d'un hexitol ou

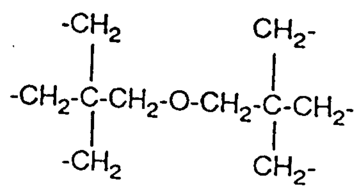

2. Produit selon la revendication 1 où dans le composé de formule III s vaut 1 ou 2.

3. Produits selon la revendication 1, où dans le composé de formule I les radicaux

Y indépendamment les uns des autres, représentent OH, $(HOCH_2CH_2)_2N$- ou $-HNR_1$ et
$R_1$ représente l'hydrogène, alkyle en $C_1$-$C_{10}$, cycloalkyle en $C_5$-$C_7$

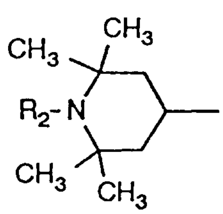,

alcényle en $C_3$-$C_6$, benzyle ou phényle,
$R_2$ représentant l'hydrogène, alkyle en $C_1$-$C_4$, OH, $-CH_2CN$, alcoxy en $C_6$-$C_{12}$, cycloalcoxy en $C_5$-$C_8$, allyle, benzyle, acétyle ou $HOCH_2CH_2$-, et
a valant 1, 2, 3, 4 ou 6,

lorsque Y = OH et a = 1,

X représente alkyle en $C_1$-$C_{30}$, alcényle en $C_3$-$C_{18}$, $-CH_2CH_2T_1(CH_2CH_2O)_bR_4$ ou

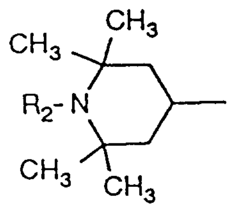

$R_2$ ayant la signification donnée ci-dessus, et

- $T_1$ représente l'oxygène, le soufre ou >N-$R_5$,
- $R_4$ représente alkyle en $C_1$-$C_{10}$,
- b étant un nombre entier de 0 à 10 et
- $R_5$ représente l'hydrogène, alkyle en $C_1$-$C_{10}$ ou phényle, ou

lorsque Y = OH et a = 2,

X représente $-CH_2CH_2T_2(CH_2CH_2O)_bCH_2CH_2-$, b ayant la signification donnée ci-dessus

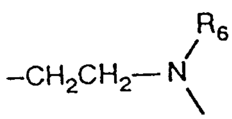 , $-C_cH_{2c}-$ , 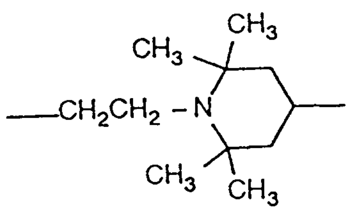 ,

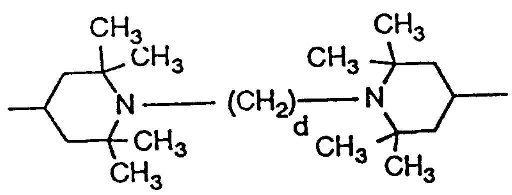 , $-CH_2-CH=CH-CH_2-$ ,

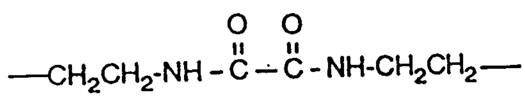 ou

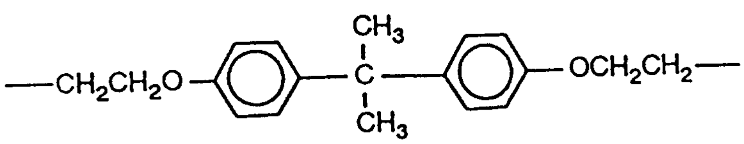

$T_2$ étant l'oxygène, le soufre, >N-$R_5$ ou

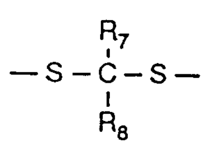

et $R_5$ ayant la signification donnée ci-dessus,

- $R_6$ étant l'hydrogène, alkyle en $C_1$-$C_{10}$ ou phényle,
- c étant un nombre entier de 2 à 10,
- d étant un nombre entier de 2 à 6, et
- $R_7$, $R_8$, indépendamment l'un de l'autre, représente l'hydrogène, alkyle en $C_1$-$C_{10}$ ou phényle, ou $R_7$ et $R_8$ avec l'atome de carbone auquel ils sont liés forment un cycle cycloalkyle en $C_5$-$C_7$, ou

lorsque Y = $-HNR_1$ et a = 1,

X représente alkyle en $C_1$-$C_{10}$, alcényle en $C_3$-$C_{18}$, cycloalkyle en $C_5$-$C_7$, benzyle, phényle

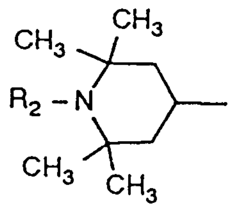
,

$R_2$ ayant la signification donnée ci-dessus, ou X étant de plus

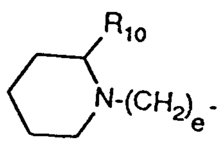

ou X avec $R_1$ forment ensemble un groupe de formule $-CH_2CH_2CH_2CH_2CH_2-$ ou $-CH_2CH_2OCH_2CH_2-$,

$R_{10}$ étant l'hydrogène ou méthyle, et

e vaut 2 ou 3, et

dans le composé de formule II les radicaux

Z représente l'hydrogène ou un groupe de formule

$$-(C_hH_{2h}O)_i-\overset{O}{\overset{\|}{C}}-R_{11}$$

et

k représente un nombre entier de 0 à 4,

h valant 2 ou 3,

i étant un nombre entier de 0 à 6, et

$R_{11}$ étant alkyle en $C_1$-$C_{20}$, alcényle en $C_8$-$C_{20}$, cycloalkyle en $C_5$-$C_7$, phényle ou benzyle, à la condition que le composé de formule II contienne un groupe

$$-(C_hH_{2h}O)_i-\overset{O}{\overset{\|}{C}}-R_{11}$$

dans le composé de formule III

$R_{12}$ représente alkyle en $C_1$-$C_6$, cycloalkyle en $C_5$-$C_7$, phényle ou benzyle,

$R_{15}$ représente l'hydrogène, alkyle en $C_1$-$C_6$, cycloalkyle en $C_5$-$C_7$, phényle ou benzyle,

s valant 1 ou 2,

Q représente $-C_mH_{2m}-$,

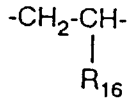 ou 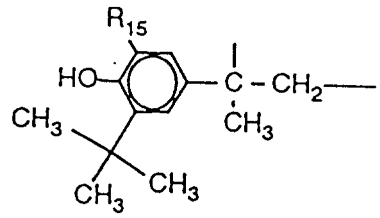

$R_{15}$ ayant la signification donnée ci-dessus,

| | |
|---|---|
| m | étant un nombre entier de 0 à 3, |
| $R_{16}$ | représente alkyle en $C_1$-$C_4$, et |
| n | est un nombre entier de 1 à 6, |

lorsque n = 1,

$R_{17}$ représente l'hydrogène, alkyle en $C_1$-$C_{30}$, cycloalkyle en $C_5$-$C_7$, alcényle en $C_2$-$C_{18}$, un radical monovalent d'un hexose, un radical monovalent d'un hexitol,

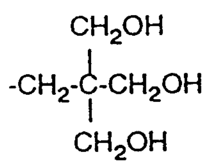 , 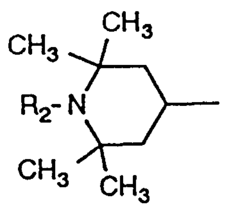 ,

$R_2$ ayant la signification donnée ci-dessus, ou, en outre, $R_{17}$ représente $-CH_2CH_2-T_3-R_{19}$ ou

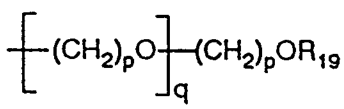

| | |
|---|---|
| $T_3$ | étant l'oxygène, le soufre ou >N-$R_{22}$ |
| $R_{19}$ | représente |

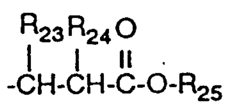 , 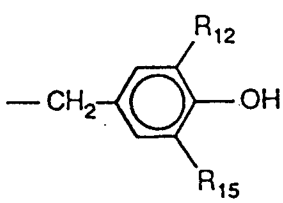

$R_{12}$ et $R_{15}$ ayant la signification donnée ci-dessus, ou $R_{19}$ étant de plus l'hydrogène, alkyle en $C_1$-$C_{18}$, phényle, cycloalkyle en $C_5$-$C_7$ ou

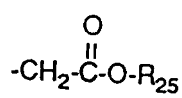

| | |
|---|---|
| p | étant un nombre entier de 2 à 4, |
| q | étant un nombre entier de 2 à 20, |
| $R_{22}$ | représentant alkyle en $C_1$-$C_{10}$, phényle ou cycloalkyle en $C_5$-$C_8$, |
| $R_{23}$, $R_{24}$, | indépendamment l'un de l'autre, représentent l'hydrogène ou méthyle à la condition que $R_{23}$ et $R_{24}$ |

ne soient pas simultanément méthyle ;

$R_{25}$ représente l'hydrogène ou alkyle en $C_1$-$C_{18}$ ou

lorsque n = 2,

$R_{17}$ représente un radical bivalent d'un hexose, un radical bivalent d'un hexitol

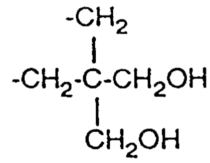, 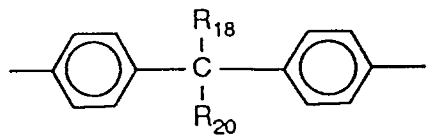, $-C_rH_{2r}-$,

$-\left[(CH_2)_pO\right]_q-(CH_2)_p-$ ,

p et q ayant la signification ci-dessus $-CH_2CH_2-T_4-CH_2CH_2-$, $-CH_2-CH{=}CH-CH_2-$, $-CH_2-C{\equiv}C-CH_2-$ ,

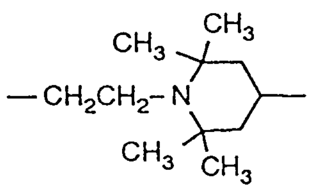, 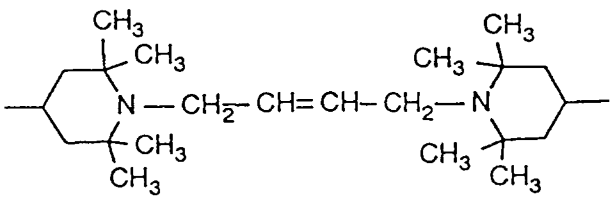,

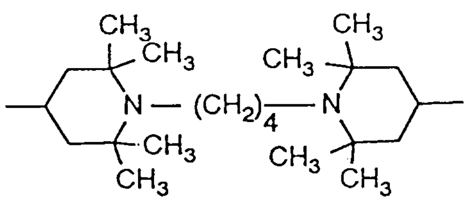, 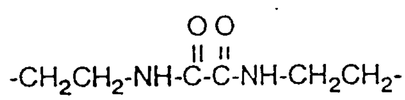 ou

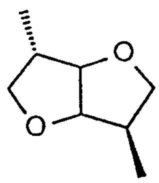

$R_{18}$, $R_{20}$, indépendamment l'un de l'autre, représentent l'hydrogène ou alkyle en $C_1$-$C_6$ ou ensemble forment le radical $-CH_2CH_2CH_2CH_2CH_2-$,

r est un nombre entier de 2 à 10,

$T_4$ représente le soufre, >N-$R_{26}$ ou

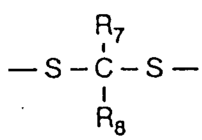

$R_7$ et $R_8$ ayant la signification donnée ci-dessus et

$R_{26}$ représente l'hydrogène, alkyle en $C_1$-$C_{10}$, phényle cycloalkyle en $C_5$-$C_8$ ou

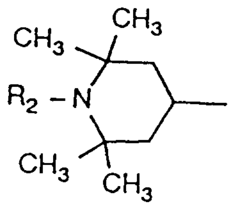

$R_2$ ayant la signification donnée ci-dessus.

**4.** Produits selon la revendication 1, où dans le composé de formule I les radicaux

Y indépendamment les uns des autres, représentent OH, $(HOCH_2CH_2)_2N$- ou $-HNR_1$ et

$R_1$ représente l'hydrogène, alkyle en $C_1$-$C_4$ ou

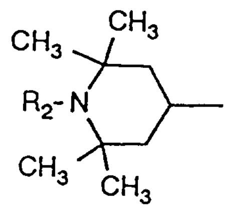

$R_2$ étant l'hydrogène, alkyle en $C_1$-$C_4$, OH, allyle, benzyle, acétyle ou $HOCH_2CH_2$-, et

a valant 1, 2, 3, 4 ou 6,

lorsque Y = OH et a = 1,

X représente alkyle en $C_1$-$C_{18}$, alcényle en $C_3$-$C_{18}$, $-CH_2CH_2T_1(CH_2CH_2O)_bR_4$ ou

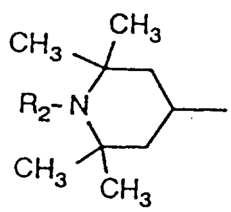

$R_2$ ayant la signification donnée ci-dessus, et

$T_1$ représente l'oxygène,

$R_4$ représente alkyle en $C_1$-$C_4$, et

b est un nombre entier de 0 à 10, ou

lorsque Y = OH et a = 2,

X représente $-CH_2CH_2T_2(CH_2CH_2O)_bCH_2CH_2$-, b ayant la signification donnée ci-dessus, ou de plus $—C_cH_{2c}—$,

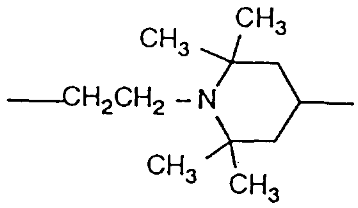

ou $—CH_2\text{-}CH\text{=}CH\text{-}CH_2—$

$T_2$ représente l'oxygène, le soufre ou $>N\text{-}R_5$,

$R_5$ représente l'hydrogène,

b vaut 0 ou 1, et

c est un nombre entier de 2 à 8, ou

lorsque a = 3,

X représente

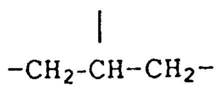

ou $N(CH_2CH_2\text{-})_3$

lorsque Y = OH et a = 4,

X représente

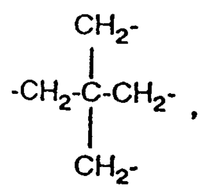

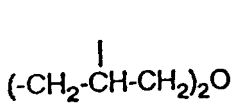

ou

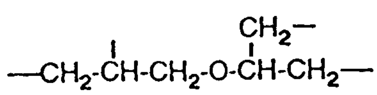

ou

lorsque Y = OH et a = 6,

X représente

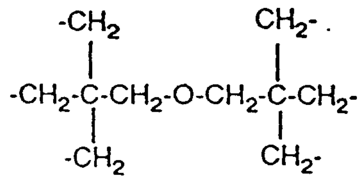

ou

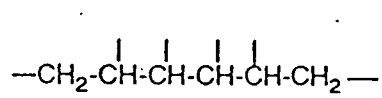

lorsque Y = $-HNR_1$ et a = 1,

X représente alkyle en $C_1$-$C_{10}$, alcényle en $C_3$-$C_{18}$, cycloalkyle en $C_5$-$C_7$ ou

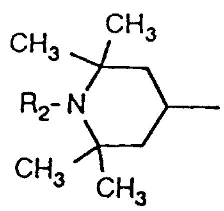

$R_2$ ayant la signification donnée ci-dessus, ou

lorsque Y = $-HNR_1$ et a = 2,

X représente $-C_fH_{2f}-$,
f étant un nombre de 2 à 10, et

dans les composés de formule II les radicaux

Z représente l'hydrogène ou un groupe de formule

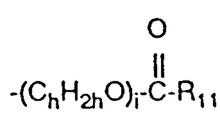

et

k vaut 1, 2 ou 3,
h vaut 2 ou 3,
i est un nombre entier de 0 à 4, et
$R_{11}$ représente alkyle en $C_1$-$C_{20}$ ou alcényle en $C_8$-$C_{20}$, à la condition que le composé de formule II contienne un groupe

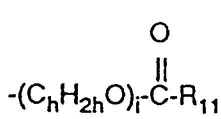

dans le composé de formule III

| | |
|---|---|
| $R_{12}$ | représente alkyle en $C_1$-$C_6$ ou cycloalkyle en $C_5$-$C_7$, |
| $R_{15}$ | représente l'hydrogène, alkyle en $C_1$-$C_6$ ou cycloalkyle en $C_5$-$C_7$, |
| s | vaut 1 ou 2, |
| Q | représente $-C_mH_{2m}$ ou |

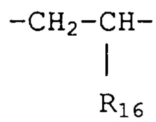

| | |
|---|---|
| m | est un nombre entier de 0 à 3, |
| $R_{16}$ | représente alkyle en $C_1$-$C_4$, et |
| n | est un nombre entier de 1 à 6, |

lorsque n = 1,

| | |
|---|---|
| $R_{17}$ | représente l'hydrogène, alkyle en $C_1$-$C_{18}$, cycloalkyle en $C_5$-$C_7$, alcényle en $C_2$-$C_{18}$, un radical monovalent d'un hexose, un radical monovalent d'un hexitol, |

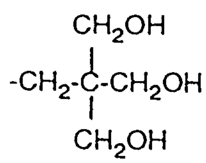

ou

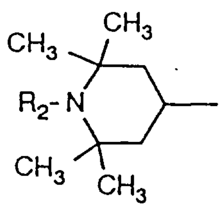

,

$R_2$ ayant la signification donnée ci-dessus, ou en outre $R_{17}$ représente

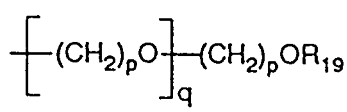

| | |
|---|---|
| $R_{19}$ | représente l'hydrogène, alkyle en $C_1$-$C_{18}$ ou cycloalkyle en $C_5$-$C_7$, |
| p | est un nombre entier de 2 à 4, |
| q | est un nombre entier de 2 à 10, ou |

lorsque n = 2,

| | |
|---|---|
| $R_{17}$ | représente un radical bivalent d'un hexose, un radical bivalent d'un hexitol |

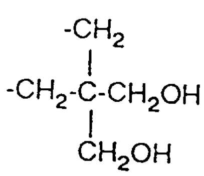

, $-C_rH_{2r}-$,

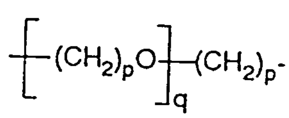

,

p et q ayant les significations données ci-dessus, $-CH_2CH_2-T_4-CH_2CH_2-$, ou

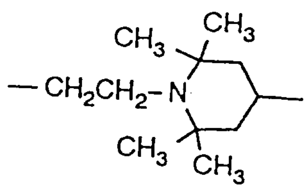

r étant un nombre entier de 2 à 10,

$T_4$ représente le soufre ou >N-$R_{26}$ et

$R_{26}$ représente l'hydrogène, alkyle en $C_1$-$C_{10}$ ou cycloalkyle en $C_5$-$C_8$ ou

lorsque n = 3,

$R_{17}$ représente un radical trivalent d'un hexose, un radical trivalent d'un hexitol

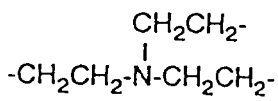 ou 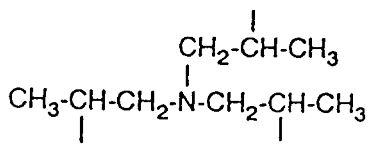

lorsque n = 4,

$R_{17}$ représente un radical tétravalent d'un hexose, un radical tétravalent d'un hexitol

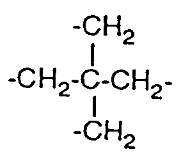 ou 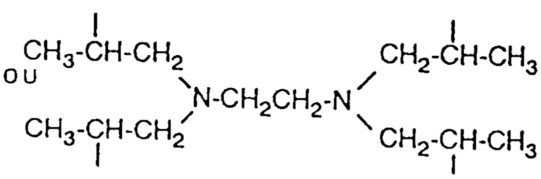

**5.** Produits selon la revendication 1, où dans le composé de formule I les radicaux

Y indépendamment les uns des autres, représentent hydroxyle ou -$NH_2$ et
a un nombre entier de 1 à 4,

lorsque a = 1,

X représente

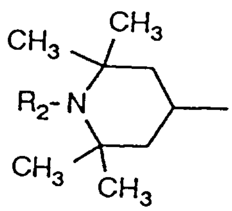

| | |
|---|---|
| $R_2$ | représente l'hydrogène, méthyle ou $HOCH_2CH_2$-, ou |

lorsque Y = OH et a = 2,

| | |
|---|---|
| X | représente $-CH_2CH_2T_2(CH_2CH_2O)_bCH_2CH_2$-, $—C_cH_{2c}—$ ou |

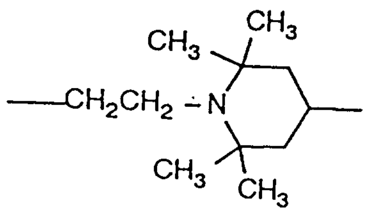

| | |
|---|---|
| $T_2$ | étant l'oxygène, le soufre ou $>N-R_5$, |
| $R_5$ | représente l'hydrogène, |
| b | vaut 0 ou 1, et |
| c | vaut 2, 3 ou 4, ou |

lorsque Y = OH et a = 3,

X représente

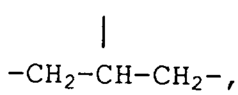

ou

lorsque Y = OH et a = 4,

X représente

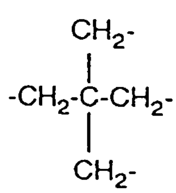

,

et

dans le composé de formule II les radicaux

Z représente l'hydrogène ou un groupe de formule

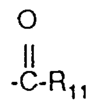

et

k vaut 1, et

$R_{11}$ représente alkyle en $C_1$-$C_{20}$ ou alcényle en $C_8$-$C_{20}$, à la condition que le composé de formule II contienne un groupe

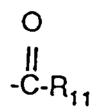

dans le composé de formule III

$R_{12}$ représente tert-butyle,

$R_{15}$ représente alkyle en $C_1$-$C_4$ et est lié en position ortho par rapport au groupe OH,

s vaut 1,

Q représente $-C_mH_{2m}-$ et est lié en position para par rapport au groupe OH,

m valant 2,

n étant 1, et

$R_{17}$ étant alkyle en $C_1$-$C_4$.

**6.** Produits selon la revendication 1, où dans le composé de formule III

$R_{12}$ représente alkyle en $C_1$-$C_4$ ou cyclohexyle,

$R_{15}$ représente alkyle en $C_1$-$C_4$ ou cyclohexyle et est lié en position ortho par rapport au groupe OH,

s vaut 1,

Q représente $-C_mH_{2m}-$ et est lié en position para au groupe OH,

m étant un nombre entier de 0 à 3, et

n étant un nombre entier de 1 à 4,

lorsque n = 1,

$R_{17}$ représente l'hydrogène, alkyle en $C_1$-$C_{10}$, cyclohexyle, alcényle en $C_2$-$C_{18}$ ou

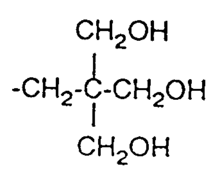

lorsque n = 2,

$R_{17}$ représente

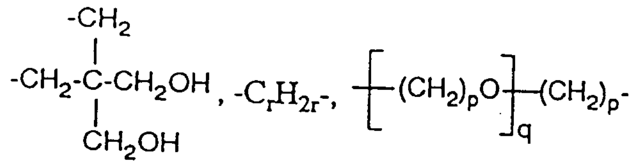

| | |
|---|---|
| | ou $-CH_2CH_2-T_4-CH_2CH_2-$, |
| p | étant un nombre entier de 2 à 4, |
| q | étant un nombre entier de 2 à 10, |
| r | étant un nombre entier de 2 à 6, |
| $T_4$ | représente le soufre ou >N-$R_{26}$ et |
| $R_{26}$ | représente l'hydrogène ou alkyle en $C_1$-$C_4$ ou |

lorsque n = 3,

| | |
|---|---|
| $R_{17}$ | représente |

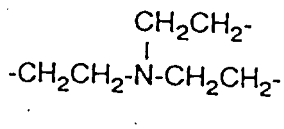 ou 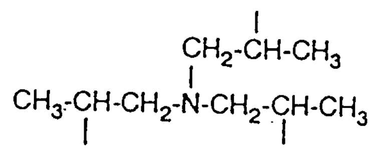

lorsque n = 4,

| | |
|---|---|
| $R_{17}$ | représente |

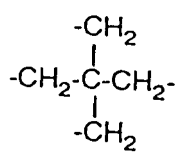 ou 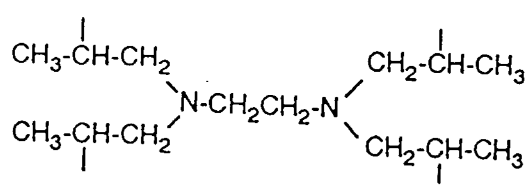

**7.** Produits selon la revendication 1, où dans le composé de formule III

| | |
|---|---|
| $R_{12}$ | représente tert-butyle, |
| $R_{15}$ | représente alkyle en $C_1$-$C_4$ et est lié en position ortho par rapport au groupe OH, |
| s | vaut 1, |
| Q | représente $-C_mH_{2m}-$ et est lié en position para par rapport au groupe OH, |
| m | valant 2, et |
| n | étant un nombre entier de 1, 2 ou 4, |

lorsque n = 1,

| | |
|---|---|
| $R_{17}$ | représente alkyle en $C_1$-$C_4$, ou |

lorsque n = 2,

$R_{17}$ représente

$$-\left[(CH_2)_pO\right]_q-(CH_2)_p-$$

ou $-CH_2CH_2-T_4-CH_2CH_2-$

p valant 2,

q valant 2, et

$T_4$ étant le soufre ou

lorsque n = 4,

$R_{17}$ représente

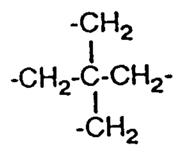

**8.** Produits selon la revendication 1, le composé de formule I étant le pentaérythritol, le thiodiéthylèneneglycol, le 1,4-butanediol, le 1,2-propanediol, le diéthylèneglycol, le triéthylèneglycol, la diéthanolamine, la glycérine

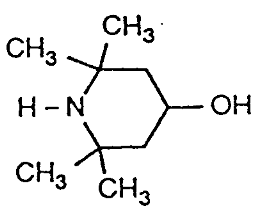 , 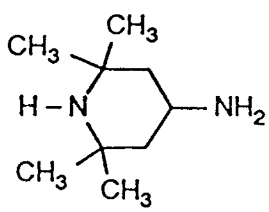 ou 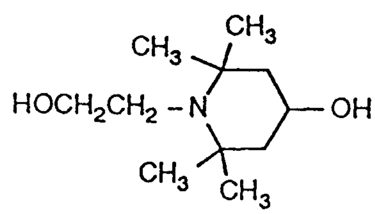

le composé de formule II sont l'huile de tournesol, l'huile de coco, l'huile de colza, l'huile de maïs, l'huile de chardon, l'huile d'olive, l'huile d'arachide ou Radiamuls, et le composé de formule III le 3-(3',5'-di-tert-butyl-4'-hydroxyphényl)propionate de méthyle ou le 3-(3'-tert-butyl-4'-hydroxy-5'-méthylphényl)propionate de méthyle.

**9.** Produit selon la revendication 1, dans lequel le rapport quantitatif en moles des composants a), b) et c) est de 0,1:1:0,1 à 15:1:30.

**10.** Produit selon la revendication 1, dans lequel le taux en poids du groupe efficace E-2

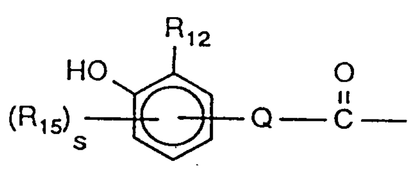 (E-2)

est de 30 à 80 % en poids.

**11.** Produit selon la revendication 1, où l'on fait réagir d'abord le composant a) et le composant b) et ensuite on fait réagir le produit intermédiaire ainsi obtenu avec le composant c).

**12.** Composition contenant

α) une matière organique soumise à la dégradation par oxydation, par la chaleur ou par la lumière, et
β) au moins un produit selon la revendication 1.

**13.** Composition selon la revendication 12 où la composant α est un lubrifiant, un fluide hydraulique, un fluide d'usinage de métaux ou un polymère synthétique.

**14.** Composition selon la revendication 12 dans laquelle le composant α est un lubrifiant pris dans la gamme des huiles minérales, des huiles synthétiques ou un mélange.

**15.** Composition selon la revendication 12 dans laquelle le composant α est un polymère synthétique.

**16.** Composition selon la revendication 12 dans laquelle le composant α est une polyoléfine ou un copolymère de styrène.

**17.** Utilisation des produits selon la revendication 1 pour la stabilisation de polymères et d'huile contre la dégradation induite par oxydation, par la chaleur ou par la lumière.

**18.** Procédé pour la stabilisation de polymères ou d'huile contre la dégradation induite par oxydation, par la chaleur ou par la lumière, caractérisé en ce que leur incorpore un produit selon la revendication 1.

**19.** Procédé pour la préparation des produits selon la revendication 1, caractérisé en ce que l'on fait réagir les composants a), b) et c) définis dans la revendication 1 dans un rapport quantitatif en moles de 0,1:1:0,1 à 15:1:30.